# EUROPEAN PATENT APPLICATION

(11) **EP 2 163 245 A1**
(43) Date of publication of application: **17.03.2010**
(21) Application number: 08164072.4
(22) Date of filing: 10.09.2008
(51) Int. Cl.: A61K 31/16, A61K 31/437, A61K 31/443, A61K 31/4433, A61K 31/538, A61P 17/06

(54) **Renin inhibitors for the treatment of psoriasis**

(71) Applicant: Novartis AG, 4056 Basel (CH)
(72) Inventor: The designation of the inventor has not yet been filed
(74) Representative: Pommerenke, Alexander

(57) **Abstract**

The application relates to renin inhibitors, in particular substituted piperidines and delta-amino-gamma-hydroxy-omega-(hetero)aryl alkanoic acid amide derivatives, useful as a treatment of psoriasis.

## Description

The present invention relates to renin inhibitors, in particular substituted piperidines and delta-amino-gamma-hydroxy-omega-(hetero)aryl alkanoic acid amide derivatives, useful as medicines, in particular as a treatment of psoriasis.

Renin inhibitors are known in the art. Renin inhibitors are mainly described as useful in the treatment or prevention of hypertension, heart failure, glaucoma, myocardial infarction, kidney failure and restenoses. Piperidine derivatives for use as renin inhibitors are known, for example from WO 97/09311, WO 2006/005741, WO 2006/103275, WO 2006/103277, WO 2007/082907 and WO 2008/040464. Delta-amino-gamma-hydroxy-omega-(hetero)aryl alkanoic acid amide derivatives for use as renin inhibitors are known, for example from EP 678503, WO 2005/090305, WO 2006/095020 and WO 2007/031558.

Psoriasis is a common skin disease in which the cutaneous lesions are characterised by benign epidermal hyperplasia, abnormal differentiation, inflammation and changes in the dermal vasculature. It is very likely that a combination of immunological predisposition (auto-immunity, superantigen stimulation) and keratinocyte abnormalities is key to the disease mechanism. Pharmacological treatments for psoriasis are generally based on antiproliferative, anti-inflammatory or differentiation-modifying activity, or a combination of two or more of these actions. There is still a need for an efficient treatment of psoriasis.

The angiotensin II system is up regulated in psoriatic skin (Wollschläger, Das Renin-Agniotensin-System in menschlicher Haut, 2006). Higher concentration of angiotensin II, which is a product of rennin, influences both, the proliferation and inflammation observed with psoriasis.

It has now been found that renin inhibitors are particularly useful for the prevention, for the retardation of progression or for the treatment of psoriasis. In this context, a renin inhibitor is to be understood as a compound showing inhibition of renin at a minimum concentration of 1-7 nM according to the testing procedure desribed in the Traping assay (J. Nussberber et al, Hypertension, 2002; 39:e1-e8). In this context, prevention, retardation of progression and treatment is to be understood as reduced inflammation and inflammatory processes in the skin, less skin plaques and reduced thickness of the skin.

The invention therefore provides, for the treatment of psoriasis, renin inhibitors, in particular substituted piperidines and delta-amino-gamma-hydroxy-omega-(hetero)aryl alkanoic acid amide derivatives.

Substituted piperidines particularly useful in the context of the present invention are those compounds described in WO 97/09311, WO 2006/005741, WO 2006/103275, WO 2006/103277, WO 2007/082907 and WO 2008/040464, which references are herein incorporated in their entirety. Very particularly useful substituted piperidines are compounds.of the following groups A to F.

### Group A: Compounds of the general formula

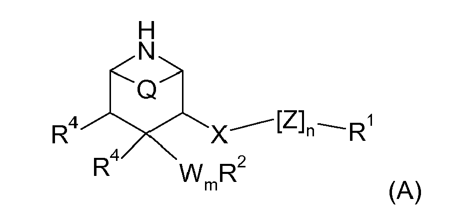

wherein R¹ is aryl or heterocyclyl;
R² is phenyl, naphthyl, acenaphthyl, cyclohexyl, pyridyl, pyrimidinyl, pyrazinyl, oxo-pyridinyl, diazinyl, triazolyl, thienyl, oxazolyl, oxadiazolyl, thiazolyl, pyrrolyl or furyl, which groups can be substituted by 1-3 halogen, hydroxy, cyano, trifluoromethyl, lower-alkyl, halo-lower-alkyl, hydroxy-lower-alkyl, lower-alkoxy-lower-alkyl, cyano-lower-alkyl, carboxy-lower-alkyl, lower-alkanoyloxy-lower-alkyl, lower-alkoxycarbonyloxy-lower-alkyl, lower-alkoxycarbonyl, or lower-alkoxy groups, or by lower-alkylenedioxy, and/or by a group L¹-T¹-L²-T²-L³-T³-L⁴-T⁴-L⁵-U.
L¹, L², L³, L⁴ and L⁵ independently of one another are a bond, C₁₋₈-alkylene, C₂₋₈-alkenylene or C₂₋₈-alkynylene or are absent;
T¹, T², T³ and T⁴ independently of one another are
   (a) a bond or are absent or are one of the groups
   (b) -CH(OH)-
   (c) -CH(OR⁶)₋
   (d) -CH(NR⁵R⁶)-
   (e) -CO-
   (f) -CR⁷R⁸-
   (g) -O- or -NR⁶-
   (h) -S(O)₀₋₂-
   (i) -SO₂NR⁶-
   (j) -NR⁶SO₂-
   (k) -CONR⁶₋
   (l) -NR⁶CO-
   (m) -O-CO-
   (n) -CO-O-
   (o) -O-CO-O-
   (p) -O-CO-NR⁶-
   (q) -NR⁶ -CO-NR⁶-
   (r) -NR⁶-CO-O-
   and the bonds emanating from (b), (d), (e) and (g)-(r) join to a C atom of the adjacent group and this C atom is saturated when the bond emanates from a hetero atom, and not more than two groups (b)-(f), three groups (g)-(h) and one group (i)-(R) are present;
R³ is hydrogen, hydroxy, lower-alkoxy or lower-alkenyloxy; and
R⁴ is hydrogen, lower-alkyl, lower-alkenyl, lower-alkoxy, hydroxy-lower-alkyl, lower-alkoxy-lower-alkyl, benzyl, oxo or a group R^{4a} - Z¹ X¹ - in which R^{4a} is
   (a) H-
   (b) lower-alkyl-
   (c) lower-alkenyl-
   (d) hydroxy-lower-alkyl-
   (e) polyhydroxy-lower-alkyl-
   (f) lower-alkyl-O-lower-alkyl-
   (g) aryl-
   (h) heterocyclyl-
   (i) arylalkyl-
   (j) heterocyclylalkyl-
   (k) aryloxyalkyl-
   (l) heterocyclyloxylalkyl-
   (m) (R⁵R⁶)-N-(CH₂)₁₋₃-
   (n) (R⁵R⁶)-N-
   (o) lower-alkyl-S(O)₀₋₂-
   (p) aryl-S(O)₀₋₂-
   (q) heterocyclyl-S(O)₀₋₂-
   (r) HO-SO₃- or salt thereof
   (s) H₂N-C(NH)-NH-
   (t) NC-,
   and the bonds emanating from (n)-(t) join to a C atom of the adjacent group and this C atom is saturated when the bond emanates from a hetero atom;
Z¹ is
   (a) a bond, is absent or is one of the groups
   (b) lower-alkylene-
   (c) lower-alkenylene-
   (d) -O-, -N(R¹¹)--, -S(O)₀₋₂-
   (e) -CO-
   (f) -O-CO-
   (g) -O-CO-O-
   (h) -O-CO-N(R¹¹)-,
   (i) -N(R¹¹)-CO-O-
   (j) -CO-N(R¹¹)-
   (k) -N(R¹¹)-CO-
   (l) -N(R¹¹)-CO-N(R¹¹)-
   (m) -CH(OR⁹)-,
   and the bonds emanating from (d) and (f)-(m) join to a C atom of the adjacent group and this C atom is saturated when the bond emanates from a hetero atom;
X¹ is
   (a) a bond, is absent or is one of the groups
   (b) -O-
   (c) -N(R¹¹)-,
   (d) -S(O))₀₋₂-
   (e) -(CH₂)₁₋₃-
   or R³ and R⁴ together are a bond;
R⁵ and R⁶ are hydrogen, lower-alkyl, lower-alkenyl, aryl-lower-alkyl or acyl or together with the N atom to which they are attached are a 5- or 6-membered heterocyclic ring which can contain an additional N atom or an O or S atom or a SO or SO₂ group and the additional N atom can be optionally substituted by lower-alkyl;
R⁷ and R⁸ together with the C atom to which they are attached are a 3-7 membered ring which can contain one or two O or S atoms or SO or SO₂ groups;
R⁹ is hydrogen, lower-alkyl, acyl or arylalkyl;
R¹⁰ is carboxyalkyl, alkoxycarbonylalkyl, alkyl or hydrogen;
R¹¹ is hydrogen or lower-alkyl;
U is hydrogen, lower-alkyl, cycloalkyl, cyano, optionally substituted cycloalkyl, aryl or heterocyclyl;
Q is ethylene or is absent;
X is a bond, oxygen, sulphur or a group -CH-R¹¹-, -CHOR⁹-, -O-CO-, - CO- or -C=NOR¹⁰- with the bond emanating from an oxygen or sulphur atom joining to a saturated C atom of group Z or to R¹ ;
W is oxygen or sulphur;
Z is lower-alkylene, lower-alkenylene, hydroxy-lower-alkylidene, -0-, -S-, -O-Alk-, -S-Alk-, -Alk-O- or -Alk-S-, in which Alk is lower alkylene;
   provided that
   a) X is -CH-R¹¹- and either R² contains a substituent L¹-T¹-L²-T²-L³-T³-L⁴-T⁴-L⁵-U or R⁴ is a substituent defined above other than hydrogen
      when Z is -0- or -S-;
   b) X is -CH-R¹¹- when Z is -O-Alk- or -S-Alk-; and
   c) Z is lower-alkenylene, -Alk-O- or -Alk-S- when X is a bond;
   n is 1 or, when X is -O-CO-, 0 or 1; and
   m is 0 or 1;
and the pharmaceutically acceptable salts thereof.

### Group B: Compounds of the general formula

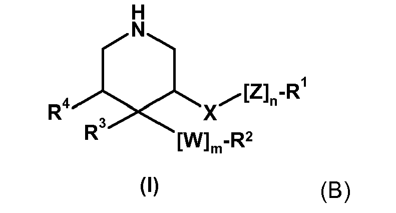

in which
(A) R¹ is aryl when R² is tetrazolyl or imidazolyl which may be substituted by 1-3 halogen, hydroxyl, cyano, trifluoromethyl, C₁₋₆-alkyl, halo-C₁₋₆-alkyl, hydroxy-C₁₋₆-alkyl, C₁₋₆-alkoxy-C₁₋₆-alkyl, cyano-C₁₋₆-alkyl, carboxy-C₁₋₆-alkyl, C₁₋₆-alkanoyloxy-C₁₋₆-alkyl, C₁₋₆-alkoxycarbonyloxy-C₁₋₆-alkyl, C₁₋₆-alkoxycarbonyl or C₁₋₆-alkoxy groups, or a C₁₋₆-alkylenedioxy group, and/or by an L1-T1-L2-T2-L3-T3-L4-T4-L5-U radical; or
(B) R¹ is aryl when X is -O-CH-R¹¹-CO-NR⁹-; or
(C) R¹ is aryl when Z is -alk-NR⁹- where alk denotes C₁₋₆-alkylene, and n is 1; or
(D) R¹ is phenyl which is substituted by 1-4 acetamidinyl-C₁₋₆-alkoxy, acetamidinyl-C₁₋₆-alkyl, acyl-C₁₋₆-alkoxy-C₁₋₆-alkyl, (N-acyl)-C₁₋₆-alkoxy-C₁₋₆-alkylamino, C₁₋₆-alkoxy, C₁₋₆-alkoxy-C₁₋₆-alkoxy, C₁₋₆-alkoxy-C₁₋₆-alkoxy-C₁₋₆-alkyl, C₁₋₆-alkoxy-C₁₋₆-alkyl, (N-C₁₋₆-alkoxy)-C₁₋₆-alkylaminocarbonyl-C₁₋₆-alkoxy, (N-C₁₋₆-alkoxy)-C₁₋₆-alkylaminocarbonyl-C₁₋₆-alkyl, C₁₋₆-alkoxy-C₁₋₆-alkylcarbamoyl, C₁₋₆-alkoxy-C₁₋₆alkylcarbonyl, C₁₋₆-alkoxy-C₁₋₆-alkylcarbonylamino, 1-C₁₋₆-alkoxy-C₁₋₆alkylimidazol-2-yl, 2-C₁₋₆-alkoxy-C₁₋₆-alkyl-4-oxoimidazol-1-yl, 1-C₁₋₆alkoxy-C₁₋₆-alkyltetrazol-5-yl, 5-C₁₋₆-alkoxy-C₁₋₆-alkyltetrazol-1-yl, 6-alkoxyaminocarbonyl-C₁₋₆-alkoxy, C₁₋₆-alkoxyaminocarbonyl-C₁₋₆alkyl, C₁₋₆-alkoxycarbonyl, C₁₋₆-alkoxycarbonyl-C₁₋₆-alkoxy, C₁₋₆alkoxycarbonyl-C₁₋₆-alkyl, C₁₋₆-alkoxycarbonylamino-C₁₋₆-alkoxy, C₁₋₆alkoxycarbonylamino-C₁₋₆-alkyl, C₁₋₆-alkyl, (N-C₁₋₆-alkyl)-C₁₋₆-alkoxy-C₁₋₆-alkylcarbamoyl, (N-C₁₋₆-alkyl)-C₁₋₆-alkoxy-C₁₋₆-alkylcarbonylamino, (N-C₁₋₆-alkyl)-C₁₋₆-alkoxycarbonylamino, (N-C₁₋₆-alkyl)-C₀₋₆alkylcarbonylamino-C₁₋₆-alkoxy, (N-C₁₋₆-alkyl)-C₀₋₆-alkylcarbonylamino-C₁₋₆-alkyl, (N-C₁₋₆-alkyl)-C₁₋₆-alkylsulphonylamino-C₁₋₆-alkoxy, (N-C₁₋₆-alkyl)-C₁₋₆-alkylsulphonylamino-C₁₋₆-alkyl, C₁₋₆-alkylamidinyl, C₁₋₆-alkylaminocarbonyl-C₁₋₆-alkoxy, di-C₁₋₆-alkylaminocarbonyl-C₁₋₆alkoxy, C₁₋₆-alkylaminocarbonyl-C₁₋₆-alkoxy-C₁₋₆-alkyl, C₁₋₆-alkylaminocarbonyl-C₁₋₆-alkyl, C₁₋₆-alkylaminocarbonylamino-C₁₋₆-alkoxy, C₁₋₆-alkylaminocarbonylamino-C₁₋₆-alkyl, di-C₁₋₆-alkylaminocarbonyl-C₁₋₆-alkyl, C₁₋₆-alkylamino-C₂₋₆-alkoxy, di-C₁₋₆-alkylamino-C₂₋₆-alkoxy, C₁₋₆-alkylamino-C₁₋₆-alkyl, di-C₁₋₆-alkylamino-C₁₋₆-alkyl, C₁₋₆alkylcarbamoyl, di-C₁₋₆-alkylcarbamoyl, C₀₋₆-alkylcarbonyl, C₀₋₆alkylcarbonylamino-C₁₋₆-alkoxy, C₀₋₆-alkylcarbonylamino, C₀₋₆-alkylcarbonylamino-C₁₋₆-alkyl, C₁₋₆-alkylcarbonyloxy-C₁₋₆-alkoxy, C₁₋₆alkylcarbonyloxy-C₁₋₆-alkyl, C₁₋₆-alkylsulphonyl, C₁₋₆-alkylsulphonyl-C₁₋₆-alkoxy, C₁₋₆-alkylsulphonyl-C₁₋₆-alkyl, C₁₋₆-alkylsulphonylamino-C₁₋₆-alkoxy, C₁₋₆-alkylsulphonylamino-C₁₋₆-alkyl, carbamoyl, carbamoyl-C₁₋₆-alkoxy, carbamoyl-C₁₋₆-alkyl, carboxy-C₁₋₆-alkoxy, carboxy-C₁₋₆-alkoxy-C₁₋₆-alkyl, carboxy-C₁₋₆-alkyl, cyano, cyano-C₁₋₆alkoxy, cyano-C₁₋₆-alkyl, C₃₋₆-cycloalkylcarbonylamino-C₁₋₆-alkoxy, C₃₋₆-cycloalkylcarbonylamino-C₁₋₆-alkyl, cyclopropyl-C₁₋₆-alkyl, O,N-dimethylhydroxylamino-C₁₋₆-alkyl, halo-C₁₋₆-alkoxy, halo-C₁₋₆alkyl, halogen, hydroxy-C₁₋₆-alkoxy-C₁₋₆-alkoxy, hydroxy-C₁₋₆-alkoxy-C₁₋₆-alkyl, hydroxy-C₁₋₆-alkyl, (N-hydroxy)-C₁₋₆-alkylaminocarbonyl-C₁₋₆-alkoxy, (N-hydroxy)-C₁₋₆-alkylaminocarbonyl-C₁₋₆-alkyl, (N-hydroxy)aminocarbonyl-C₁₋₆-alkoxy, (N-hydroxy)aminocarbonyl-C₁₋₆-alkyl, 2-oxooxazolidinyl-C₁₋₆-alkoxy, 2-oxooxazolidinyl-C₁₋₆-alkyl, O-methyloximyl-C₁₋₆-alkyl or trifluoromethyl; or
(E) R¹ is aryl which is substituted by 3-acetamidomethylpyrrolidinyl 3-C₁₋₆-alkoxy-C₁₋₆-alkylpyrrolidinyl, 3,4-dihydroxypyrrolidinyl, 2,6-dimethylmorpholinyl, 3,5-dimethylmorpholinyl, dioxanyl, dioxolanyl, 4,4-dioxothiomorpholinyl, dithianyl, dithiolanyl, 2-hydroxymethylpyrrolidinyl, 4-hydroxypiperidinyl, 3-hydroxypyrrolidinyl, imidazolylalkoxy, imidazolylalkyl, 2-methylimidazolylalkoxy, 2-methylimidazolylalkyl, 3-methyl[1,2,4]oxadiazol-5-ylalkoxy, 5-methyl[1,2,4]oxadiazol-3-ylalkoxy, 3-methyl-[1,2,4]oxadiazol-5ylalkyl, 5-methyl[1,2,4]oxadiazol-3-ylalkyl, 4-methylpiperazinyl, 5-methyltetrazol-1-ylalkoxy, 5-methyltetrazol-1-ylalkyl, morpholinyl, [1,2,4]oxadiazol-5-ylalkoxy, [1,2,4]oxadiazol-5-ylalkyl, oxazol-4ylalkoxy, oxazol-4-ylalkyl, 2-oxo-[1,3]oxazinyl, 2-oxooxazolidinyl, 2-oxoimidazolidinyl, 2-oxopyrrolidinyl, 4-oxopiperidinyl, 2-oxopyrrolidinylalkoxy, 2-oxopyrrolidinylalkyl, 2-oxotetrahydropyrimidinyl 4-oxothiomorpholinyl, piperazinyl, piperidinyl, pyrrolidinyl, pyrrolyl, [1,2,4]triazol-1-ylalkoxy, [1,2,4]triazol-4-ylalkoxy, [1,2,4]triazol-1ylalkyl, [1,2,4]triazol-4-ylalkyl, tetrazol-1-ylalkoxy, tetrazol-2-ylalkoxy, tetrazol-5-ylalkoxy, tetrazol-1-ylalkyl, tetrazol-2-ylalkyl, tetrazol-5ylalkyl, thiazol-4-ylalkoxy, thiazo-4-ylalkyl, thiomorpholinyl; or
(F) R¹ is heterocyclyl, optionally substituted, in particular as specified under (D) or (E), in particular benzo[1,3]dioxoyl, benzofuranyl, benzoxazolyl, dihydrobenzofuranyl, 3,4-dihydro-2H-benzo[1,4]oxazinyl, dihydro-3H-benzo[1,4]oxazinyl, dihydro-2H-benzo[1,4]thiazinyl, 2,3-dihydroindolyl, dihydro-1H-pyrido[2,3-b][1,4]oxazinyl, 1,1-dioxodihydro-2H-benzo[1,4]thiazinyl, indazolyl, indolyl, [1,5]naphthpyridyl, oxazolyl, 2-oxoazepanyl, 3-oxo-4H-benzo[1,4]oxazinyl, 2-oxobenzoxazolyl, 3-oxo-4H-benzo[1,4]thiazinyl, 2-oxodihydrobenzo[e][1,4]diazepinyl, 2-oxodihydrobenzo[d][1,3]oxazinyl, 2-oxodihydro-1H-quinazolinyl, 4-oxodihydroimidazolyl, 2-oxo-1,3-dihydroindolyl, 1-oxo-3H-isobenzofuranyl, 2-oxopiperidinyl 2-oxo-1H-pyrido[2,3-b][1,4]oxazinyl, 1-oxopyridyl, 2-oxotetrahydrobenzo[e][1,4]diazepinyl, 4-oxo-3H-thieno[2,3-d]pyrimidinyl, 5-oxo-4H-[1,2,4]triazinyl, phthalazinyl, pyrazolyl, 1 H-pyrrolizinyl, 1 H-pyrrolo[2,3-b]pyridyl, pyrrolyl, tetrahydro-quinoxalinyl, tetrahydropyranyl, triazinyl, imidazo[1,5-a]pyridinyl, tetrahydro-imidazo[1,5-a]pyridinyl or 1,1,3-trioxodihydro-2H-1λ⁶-benzo[1,4]thiazinyl;
   R² is phenyl, naphthyl, acenaphthyl, cyclohexyl, pyridyl, pyrimidinyl, pyrazinyl, oxopyridinyl, diazinyl, triazolyl, thienyl, oxazolyl, oxadiazolyl, thiazolyl, pyrrolyl, furyl, tetrazolyl or imidazolyl, which radicals may be substituted by 1-3 halogen, hydroxyl, cyano, trifluoromethyl, C₁₋₆-alkyl, halo-C₁₋₆-alkyl, hydroxy-C₁₋₆-alkyl, C₁₋₆-alkoxy-C₁₋₆-alkyl, cyano-C₁₋₆-alkyl, carboxy-C₁₋₆-alkyl, C₁₋₆-alkanoyloxy-C₁₋₆-alkyl, C₁₋₆-alkoxycarbonyloxy-C₁₋₆-alkyl, C₁₋₆-alkoxycarbonyl, or C₁₋₆-alkoxy groups, or a C₁₋₆-alkylenedioxy group, and/or by an L1-T1-L2-T2-L3-T3-L4-T4-L5-U radical;
   L1, L2, L3, L4 and L5 are each independently a bond, C₁₋₈-alkylene, C₂₋₈-alkenylene or C₂₋₈-alkynylene, or are absent;
   T1, T2, T3 and T4 are each independently
      (a) a bond, or are absent, or are one of the groups
      (b) -CH(OH)-
      (c) -CH(OR⁶)-
      (d) -CH(NR⁵R⁶)-
      (e) -CO-
      (f) -CR⁷R⁸-
      (g) -O- or -NR⁶-
      (h) -S(O)₀₋₂-
      (l) -SO₂NR⁶-
      (j) -NR⁶SO₂-
      (k) -CONR⁶-
      (l) -NR⁶CO-
      (m) -O-CO-
      (n) -CO-O-
      (o) -O-CO-O-
      (p) -O-CO-NR⁶-
      (q) -N(R⁶)-CO-N(R⁶)-
      (r) -N(R⁶)-CO-O-
      (s) pyrrolidinylene, piperidinylene or piperazinylene
      (t) -C(R¹¹)(R¹²)-,
      where the bonds starting from (b)-(t) lead to a saturated or aromatic carbon atom of the adjacent group if the bond starts from a heteroatom, and where not more than two groups (b)-(f), three groups (g)-(h) and one group (i)-(t) is/are present;
   R³ is hydrogen, hydroxyl, C₁₋₆-alkoxy or C₁₋₆-alkenyloxy;
   R⁴ is C₁₋₆-alkoxy, C₁₋₆-alkoxy-C₁₋₆-alkoxy, optionally N-mono- or N,N-di-C₁-C₆-alkylated amino, optionally N-mono- or N,N-di-C₁-C₆-alkylated amino-C₁₋₆-alkoxy, optionally N-C₁₋₆-alkylated C₁₋₆-alkoxycarbonylamino-C₁₋₆-alkoxy, optionally N-C₁₋₆-alkylated C₁₋₆-alkylcarbonylamino-C₁₋₆-alkoxy, optionally N-C₁₋₆-alkylated C₃₋₈-cycloalkyl-C₁₋₆-alkylcarbonylamino-C₁₋₆-alkoxy, C₁₋₆-alkylcarbonyl-C₁₋₆-alkoxy, C₁₋₆-alkylcarbonyloxy, aryl-C₁₋₆-alkoxy, aryloxy, optionally N-mono- or N,N-di-C₁-C₆-alkylated carbamoyl-C₁₋₆-alkoxy, optionally N-mono- or N,N-di-C₃-₈-cycloalkyl-C₁-C₆-alkylated carbamoyl-C₁₋₆-alkoxy, optionally N-mono- or N,N-di-C₁-C₆-alkylated carbamoyloxy, cyanoalkyloxy, C₃₋₈-cycloalkyloxy, C₃₋₈-cycloalkyl-C₁₋₆-alkoxy, C₃₋₈-cycloalkyloxy-C₁₋₆-alkoxy, hydroxyl, hydroxy-C₁₋₆-alkoxy, hydroxy-C₁₋₆-alkoxy-C₁₋₆-alkoxy, heterocyclyl-C₁₋₆-alkoxy, heterocyclyloxy, heterocyclyloxy-C₁₋₆-alkoxy or oxo;
   R⁵ and R⁶ are each independently hydrogen, C₁₋₆-alkyl, C₂₋₆-alkenyl, aryl-C₁₋₆-alkyl or acyl, or, together with the N atom to which they are bonded, are a 5- to 6-membered heterocyclic ring which may contain an additional N, O or S atom or an -SO- or -SO₂- group, where the additional N atom may optionally be substituted by C₁₋₆-alkyl radicals;
   R⁷ and R⁸, together with the carbon atom to which they are bonded, are a 3-7-membered ring which may contain one or two -O- or -S-atoms or-SO- or -SO₂- groups;
   R⁹ is hydrogen, C₁₋₆-alkyl, C₁₋₆-alkoxy-C₁₋₆-alkyl, acyl, aryl-C₁₋₆-alkyl, C₃₋₈-cycloalkyl or C₃₋₈-cycloalkyl-C₁₋₆-alkyl;
   R¹⁰ is carboxy-C₁₋₆-alkyl, C₁₋₆-alkoxycarbonyl-C₁₋₆-alkyl, C₁₋₆-alkyl or hydrogen;
   R¹¹ is hydrogen, halogen or C₁₋₆-alkyl;
   R¹² is hydrogen, halogen or C₁₋₆-alkyl;
   R¹¹ and R¹², together with the C-atom to which they are attached, may also be C₃₋₈-cycloalkyl;
   U is hydrogen, C₁₋₆-alkyl, cyano, trifluoromethyl, optionally substituted C₃₋₁₂-cycloalkyl, aryl, or heterocyclyl;
   X is a bond, oxygen or sulphur or is >CR¹¹R¹², >CHOR⁹, -O-CO-, >CO, >C=NOR¹⁰, -O-CR¹¹R¹²-, -O-CR¹¹R¹²-CO-NR⁹- -CO-NR⁹- or - NR⁹-, where a bond starting from a nitrogen, oxygen or sulphur atom leads to a saturated C atom of the Z group or to R¹;
   W is oxygen or sulphur;
   Z is C₁₋₆-alkylene, C₂₋₆-alkenylene, hydroxy-C₁₋₆-alkylidene, -0-, -N-, - S-, -O-alk-, -NR⁹-alk, -S-alk-, -alk-O-, -alk-S- or -alk-NR⁹-, where alk denotes C₁₋₆-alkylene; and where
      (a) if Z is -0- or-S-, X is -CR¹¹R¹²- and either R² contains an L1-T1-L2-T2-L3-T3-L4-T4-L5-U substituent or R⁴ is a substituent other than hydrogen as defined above;
      (b) if Z is -O-alk- or-S-alk-, X is -CR¹¹R¹²-; and (c) if X is a bond, Z is C₁₋₆-alkylene, C₂₋₆-alkenylene, -NR⁹-alk-, -alk-NR⁹-, -alk-O- or -alk-S-;
      n is 1 or, when X is -O-CO-, is 0 or 1;
      m is 0 or 1;
      and their pharmaceutically acceptable salts.

### Group C: Compounds of the general formula

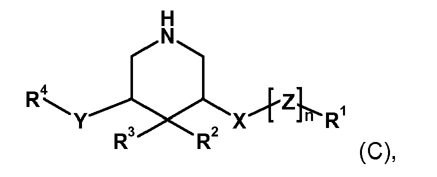

in which
(A) R¹ is aryl when R² is tetrazolyl or imidazolyl, each of which may be substituted by C₁₋₈alkoxy-C₁₋₈alkoxy-C₁₋₈alkyl, C₁₋₈alkoxy-C₁₋₈alkyl, aryloxy-C₁₋₈alkyl, heterocyclyloxy-C₁₋₈alkyl; or
(B) R¹ is aryl when X is -O-CHR⁵-CO-NR⁶-; or
(C) R¹ is aryl when Z is -Alk-NR⁶-, where Alk is C₁₋₈alkylene, and n is 1; or
(D) R¹ is aryl which is substituted by 1-4 acetamidinyl-C₁₋₈alkyl, acyl-C₁₋₈alkoxy-C₁₋₈alkyl, (N-acyl)-C₁₋₈alkoxy-C₁₋₈alkylamino, C₁₋₈alkoxy, C₁₋₈alkoxy-C₁₋₈alkoxy, C₁₋₈alkoxy-C₁₋₈alkoxy-C₁₋₈alkyl, C₁₋₈alkoxy-C₁₋₈alkyl, (N-C₁₋₈alkoxy)-C₁₋₈alkylaminocarbonyl-C₁₋₈alkoxy, (N-C₁₋₈alkoxy)-C₁₋₈alkylaminocarbonyl-C₁₋₈alkyl, C₁₋₈alkoxy-C₁₋₈alkylcarbamoyl, C₁₋₈alkoxy-C₁₋₈alkylcarbonyl, C₁₋₈alkoxy-C₁₋₈alkylcarbonylamino, 1-C₁₋₈alkoxy-C₁₋₈alkylimidazol-2-yl, 2-C₁₋₈alkoxy-C₁₋₈alkyl-4-oxoimidazol-1-yl, 1-C₁₋₈alkoxy-C₁₋₈alkyltetrazol-5-yl, 5-C₁₋₈alkoxy-C₁₋₈alkyltetrazol-1-yl, 6-alkoxy-aminocarbonyl-C₁₋₈alkoxy, C₁₋₈alkoxyaminocarbonyl-C₁₋₈alkyl, C₁₋₈alkoxycarbonyl, C₁₋₈alkoxycarbonyl-C₁₋₈alkoxy, C₁₋₈alkoxycarbonyl-C₁₋₈alkyl, C₁₋₈alkoxycarbonylamino-C₁₋₈alkoxy, C₁₋₈alkoxycarbonylamino-C₁₋₈alkyl, C₁₋₈alkyl, (N-C₁₋₈alkyl)-C₁₋₈alkoxy-C₁₋₈alkylcarbamoyl, (N-C₁₋₈alkyl)-C₁₋₈alkoxy-C₁₋₈alkylcarbonylamino, (N-C₁₋₈alkyl)-C₁₋₈alkoxycarbonylamino, (N-C₁₋₈alkyl)-C₀₋₈alkylcarbonylamino-C₁₋₈alkoxy, (N-C₁₋₈alkyl)-C₀₋₈alkylcarbonylamino-C₁₋₈alkyl, (N-C₁₋₈alkyl)-C₁₋₈alkylsulphonylamino-C₁₋₈alkoxy, (N-C₁₋₈alkyl)-C₁₋₈alkylsulphonylamino-C₁₋₈alkyl, C₁₋₈alkylamidinyl, C₁₋₈alkylaminocarbonyl-C₁₋₈alkoxy, di-C₁₋₈alkylaminocarbonyl-C₁₋₈alkoxy, C₁₋₈alkylaminocarbonyl-C₁₋₈alkoxy-C₁₋₈alkyl, C₁₋₈alkylaminocarbonyl-C₁₋₈alkyl, C₁₋₈alkylaminocarbonylamino-C₁₋₈alkoxy, C₁₋₈alkylamino-carbonylamino-C₁₋₈alkyl, di-C₁₋₈alkylaminocarbonyl-C₁₋₈alkyl, C₁₋₈alkylamino-C₂₋₈alkoxy, di-C₁₋₈alkylamino-C₂₋₈alkoxy, C₁₋₈alkylamino-C₁₋₈alkyl, di-C₁₋₈alkylamino-C₁₋₈alkyl, C₁₋₈alkylcarbamoyl, di-C₁₋₈alkylcarbamoyl, C₀₋₈alkylcarbonylamino-C₁₋₈alkoxy, C₀₋₈alkylcarbonylamino, C₀₋₈alkylcarbonylamino-C₁₋₈alkyl, C₁₋₈alkylcarbonyloxy-C₁₋₈alkoxy, C₁₋₈alkylcarbonyloxy-C₁₋₈alkyl, C₁₋₈alkylsulphonyl, C₁₋₈alkylsulphonyl-C₁₋₈alkoxy, C₁₋₈alkylsulphonyl-C₁₋₈alkyl, C₁₋₈alkylsulphonylamino-C₁₋₈alkoxy, C₁₋₈alkylsulphonylamino-C₁₋₈alkyl, carbamoyl, carbamoyl-C₁₋₈alkoxy, carbamoyl-C₁₋₈alkyl, carboxy-C₁₋₈alkoxy, carboxy-C₁₋₈alkoxy-C₁₋₈alkyl, carboxy-C₁₋₈alkyl, cyano, cyano-C₁₋₈alkoxy, cyano-C₁₋₈alkyl, C₃₋₈cycloalkyl-C₁₋₈alkoxy, C₃₋₈cycloalkyl-C₁₋₈alkyl, C₃₋₈cycloalkylcarbonylamino-C₁₋₈alkoxy, C₃-₈cycloalkylcarbonylamino-C₁₋₈alkyl, O,N-dimethylhydroxylamino-C₁₋₈alkyl, halogen, hydroxy-C₁₋₈alkoxy-C₁₋₈alkoxy, hydroxy-C₁₋₈alkoxy-C₁₋₈alkyl, hydroxy-C₁₋₈alkyl, (N-hydroxy)-C₁₋₈alkylaminocarbonyl-C₁₋₈alkoxy, (N-hydroxy)-C₁₋₈alkylaminocarbonyl-C₁₋₈alkyl, (N-hydroxy)aminocarbonyl-C₁₋₈alkoxy, (N-hydroxy)aminocarbonyl-C₁₋₈alkyl, 2-oxoxazolidinyl-C₁₋₈alkoxy, 2-oxoxazolidinyl-C₁₋₈alkyl, O-methyloximyl-C₁₋₈alkyl or trifluoromethyl; or
(E) R¹ is aryl which is substituted by 1-4 3-acetamidomethylpyrrolidinyl, 3-C₁₋₈alkoxy-C₁₋₈alkylpyrrolidinyl, 3,4-dihydroxypyrrolidinyl, 2,6-dimethylmorpholinyl, 3,5-dimethylmorpholinyl, dioxanyl, dioxolanyl, 4,4-dioxothiomorpholinyl, dithianyl, dithiolanyl, 2-hydroxymethylpyrrolidinyl, 4-hydroxypiperidinyl, 3-hydroxypyrrolidinyl, imidazolylalkoxy, imidazolylalkyl, 2-methylimidazolylalkoxy, 2-methylimidazolylalkyl, 3-methyl-[1,2,4]-oxadiazol-5-ylalkoxy, 5-methyl-[1,2,4]-oxadiazol-3-ylalkoxy, 3-methyl-[1,2,4]-oxadiazol-5-ylalkyl, 5-methyl-[1,2,4]-oxadiazol-3-ylalkyl, 4-methylpiperazinyl, 5-methyltetrazol-1-ylalkoxy, 5-methyltetrazol-1-ylalkyl, morpholinyl, [1,2,4]-oxadiazol-5-ylalkoxy, [1,2,4]-oxadiazol-5-ylalkyl, oxazol-4-ylalkoxy, oxazol-4-ylalkyl, 2-oxo-[1,3]-oxazinyl, 2-oxoxazolidinyl, 2-oxoimidazolidinyl, 2-oxopyrrolidinyl, 4-oxopiperidinyl, 2-oxopyrrolidinylalkoxy, 2-oxopyrrolidinylalkyl, 2-oxotetrahydropyrimidinyl 4-oxothiomorpholinyl, piperazinyl, piperidinyl, pyrrolidinyl, pyrrolyl, [1,2,4]-triazol-1-yl-alkoxy, [1,2,4]-triazol-4-ylalkoxy, [1,2,4]-triazol-1-ylalkyl, [1,2,4]-triazol-4-ylalkyl, tetrazol-1-ylalkoxy, tetrazol-2-ylalkoxy, tetrazol-5-ylalkoxy, tetrazol-1-ylalkyl, tetrazol-2-ylalkyl, tetrazol-5-ylalkyl, thiazol-4-ylalkoxy, thiazol-4-ylalkyl or thiomorpholinyl; or
(F) R¹ is heterocyclyl optionally substituted by oxo or oxide or as indicated under (D) or (E), especially azepanyl, benzo[1,3]dioxolyl, benzofuranyl, benzoimidazolyl, 4H-benzo[1,4]oxazinyl, benzoxazolyl, 4H-benzo[1,4]thiazinyl, 1H-quinolinyl, 2H-chromenyl, dihydrobenzo[e][1,4]diazepinyl, dihydrobenzofuranyl, 3,4-dihydro-2H-benzo[1,4]oxazinyl, dihydro-3H-benzo[1,4]oxazinyl, dihydrobenzo[d][1,3]oxazinyl, dihydro-2H-benzo[1,4]thiazinyl, dihydro-2H-1λ6-benzo[1,4]thiazinyl, dihydro-1H-quinazolinyl, 1a,7b-dihydro-1H-cyclopropa[c]chromenyl, dihydroimidazolyl, 1,3-dihydroindolyl, 2,3-dihydroindolyl, dihydro-1H-pyrido[2,3-b][1,4]oxazinyl, indazolyl, indolyl, 3H-isobenzofuranyl, [1,5]naphthyridyl, oxazolyl, phthalazinyl, piperidinyl, pyrazolyl, 1H-pyrido[2,3-b][1,4]oxazinyl, pyridyl, 1 H-pyrrolizinyl, 1 H-pyrrolo[2,3-b]pyridyl, pyrrolyl, tetrahydrobenzo[e][1,4]diazepinyl, 3H-thieno[2,3-d]pyrimidinyl, tetrahydro-quinoxalinyl, 1,1a,2,7b-tetrahydrocyclopropa[c]chromenyl, tetrahydropyranyl or triazinyl;
   R² is phenyl, naphthyl, acenaphthyl, cyclohexyl, pyridyl, pyrimidinyl, pyrazinyl, oxopyridinyl, diazinyl, triazolyl, thienyl, oxazolyl, oxadiazolyl, thiazolyl, pyrrolyl, furyl, tetrazolyl or imidazolyl, where the radicals are substituted by 1-3 C₂₋₈alkenyloxy-C₁₋₈alkyl, C₁₋₈alkoxy-C₁₋₈alkyl, C₁₋₈alkoxy-C₁₋₈alkylamino-C₁₋₈alkyl, C₁₋₈alkoxy-C₁₋₈alkyl-sulphanyl-C₁₋₈alkyl, C₁₋₈alkoxy-C₀₋₈alkyl-C₃₋₈cycloalkyl-C₀₋₈alkoxy-C₁₋₈alkyl, C₁₋₈alkyl, C₁₋₈alkylsulphanyl-C₁₋₈alkoxy-C₁₋₈alkyl, C₁₋₈alkyl-sulphanyl-C₁₋₈alkyl, C₁₋₈alkylsulphonyl-C₁₋₈alkoxy-C₁₋₈alkyl, C₃₋₈cycloalkyl-C₀₋₈alkoxy-C₁₋₈alkoxy-C₁₋₈alkyl, C₃₋₈cycloalkyl-C₀₋₈alkoxy-C₁₋₈alkyl, optionally halogen-substituted C₁₋₈alkoxy-C₁₋₈alkoxy-C₁₋₈alkyl, or (oxygen-heterocyclyl)-C₀₋₈alkoxy-C₁₋₈alkyl, and may, in addition to the aforementioned substituents, also be substituted by a maximum of 4 halogen;
   R³ is hydrogen, hydroxy, C₁₋₈alkoxy or C₂₋₈alkenyloxy;
   R⁴ is optionally halogen- and/or hydroxy-substituted C₁₋₈alkyl, optionally halogen- and/or hydroxy-substituted C₁₋₈alkoxy-C₁₋₈alkyl, optionally N-mono- or N,N-di-C₁₋₈-alkylated amino-C₁₋₈alkyl, optionally N-mono- or N,N-di-C₁₋₈-alkylated or optionally hydroxy-substituted amino-C₀₋₈alkylcarbonyl-C₁₋₈alkyl, hydroxy-C₀₋₈alkylcarbonyl-C₀₋₈alkyl, C₁₋₈alkoxy-C₀₋₈alkylcarbonyl-C₀₋₈alkyl, optionally N-C₁₋₈-alkylated C₁₋₈alkoxycarbonylamino-C₁₋₈alkyl, optionally N-C₁₋₈-alkylated C₁₋₈alkoxy-C₁₋₈alkylamino-C₁₋₈alkyl, optionally N-C₁₋₈-alkylated or optionally halogen-substituted C₁₋₈alkylcarbonylamino-C₁₋₈alkyl, cyano-C₁₋₈alkyl, optionally N-C₁₋₈-alkylated or optionally halogen-substituted C₃₋₈-cycloalkyl-C₀₋₈alkylcarbonylamino-C₁₋₈alkyl, optionally N-C₁₋₈-alkylated hydroxy-C₁₋₈alkylamino-C₁₋₈alkyl, hetero-cyclylcarbonyl-C₀₋₈alkylamino-C₁₋₈alkyl, C₁₋₈alkoxycarbonylamino-C₁₋₈alkyl, optionally N-C₁₋₈-alkylated or optionally halogen-substituted heterocyclyl-C₀₋₈alkylcarbonylamino-C₁₋₈alkyl, C₃₋₈cycloalkyl-C₀₋₈alkyl, C₃₋₈cycloalkyloxy-C₁₋₈alkyl, heterocyclyl-C₀₋₈-(optionally hydroxy-substituted)alkyl, optionally N-C₁₋₈-alkylated heterocyclyl-C₀₋₈alkylamino-C₀₋₈alkylcarbonyl-C₀₋₈alkyl, C₁₋₈alkylsulphonyl-C₁₋₈alkyl, C₂₋₈alkinyl, heterocyclyl-C₂₋₈alkinyl, optionally N-mono- or N,N-di-C₁₋₈-alkylated amino-C₂₋₈alkinyl, N-mono- or N,N-di-C₁₋₈-alkylated aminocarbonyl-C₂₋₈alkinyl, heterocyclylcarbonyl-C₀₋₈alkyl or heterocyclyloxy-C₁₋₈alkyl;
      and where
      (a) if Y is -O- and R² is not para-C₁₋₈alkyl-substituted phenyl, then R⁴ may additionally also be hydrogen, and
      (b) if Y is oxo, then R⁴ is absent;
      R⁵ is acyl, C₂₋₈alkenyl, C₁₋₈alkyl, aryl-C₁₋₈alkyl or hydrogen;
      R⁶ is acyl, C₁₋₈alkoxy-C₁₋₈alkyl, C₁₋₈alkyl or aryl-C₁₋₈alkyl or hydrogen;
      R⁷ is C₁₋₈alkoxycarbonyl-C₁₋₈alkyl, C₁₋₈alkyl, carboxy-C₁₋₈alkyl or hydrogen;
      X is a bond, oxygen or sulphur, where the bond originating from an oxygen or sulphur atom leads to a saturated C atom of the group Z, or is a group >CH-R⁵, >CHOR⁶, -O-CO-, >CO, >C=NOR⁷, -O-CHR⁵-or -O-CHR⁵-CO-NR⁶-;
      Y is -O-, oxo or a bond;
      Z is C₁₋₈alkylene, C₂₋₈alkenylene, hydroxy-C₁₋₈alkylidene, -O-, -S-, -O-Alk-, -S-Alk-, -Alk-O-,
      -Alk-S- or-Alk-NR⁶-, where Alk is C₁₋₈alkylene; and where (a) if Z is -O-Alk- or -S-Alk-, then X is -CHR⁵-; and (b) if X is a bond, then Z is C₂₋₈alkenylene, -Alk-O- or -Alk-S-;
      n is 1, or, if X is -O-CO- or -O-CHR⁵-CO-NR⁶-, is 0 or 1;
and the pharmaceutically acceptable salts thereof.

### Group D: Compounds of the general formulae

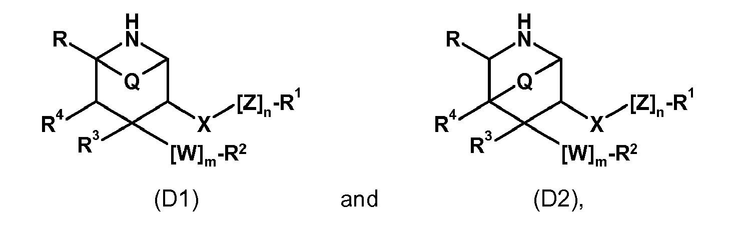

where
R is C₂₋₈-alkenyl, C₁₋₈-alkyl, C₂₋₈-alkynyl, C₀₋₈-alkyl-carbonyl-amino-C₁₋₈-alkyl, C₃₋₈-cycloalkyl-C₀₋₈-alkyl, C₁₋₈-alkyl-sulfonyl-₁₋₈-alkyl, optionally N-mono- or N,N-di-C₁₋₈-alkylated carbamoyl-C₀₋₈-alkyl, optionally O-C₁₋₈-alkylated carboxyl-C₀₋₈-alkyl, optionally N and/or N' mono-, di- or tri-C₁₋₈-alkylated ureido-C₁₋₈-alkyl or heterocyclyl-carbonyl-C₀₋₈-alkyl, each of said radicals may be substituted, preferably by 1-4 C₁₋₈-alkoxy, C₁₋₈-alkoxy-C₁₋₈-alkoxy, C₁₋₈-alkoxycarbonyl-(N-C₁₋₈-alkyl)-amino, C₁₋₈-alkyl, C₁₋₈-alkyl-carbonyl-(N-C₁₋₈-alkyl)-amino, C₁₋₈-alkyl-sulfanyl, C₁₋₈-alkyl-sulfinyl, aryl-C₀₋₈-alkoxy, which is optionally substituted by 1 or 2 aryl or C₁₋₈-alkoxy radicals, aryl, which is optionally substituted by 1 or 2 aryl or C₁₋₈-alkoxy radicals, aryl-amino, cyano, C₃₋₈-cycloalkoxy, halogen, heterocyclyl-C₀₋₈-alkyl, heterocyclyl-C₀₋₈-alkoxy, heterocyclyl-C₀₋₈-alkyl-amino, heterocyclyl-carbonyl, hydroxyl, optionally N-mono- or N,N-di-C₁₋₈-alkylated amino, optionally N-mono- or N,N-di-C₁₋₈-alkylated carbamoyl-C₀₋₈-alkyl, optionally N-mono- or N,N-di-C₁₋₈-alkylated carbamoyloxy, optionally N-mono- or N,N-di-C₁₋₈-alkylated sulfamoyl, optionally N-arylated or N-heterocyclyl-substituted carbamoyl, oxo, trifluoromethoxy or trifluoromethyl;
R¹ is aryl or heterocyclyl;
R² is acenaphthyl, cyclohexyl, diazinyl, furyl, imidazolyl, naphthyl, oxadiazolyl, oxazolyl, phenyl, pyrazinyl, pyridyl, pyrimidinyl, pyrrolyl, oxopyridinyl, tetrazolyl, thienyl, or triazolyl, each of said radicals may be substituted by 1-3 C₁₋₈-alkanoyloxy-C₁₋₈-alkyl, C₂₋₈-alkenyloxy, C₁₋₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, C₁₋₈-alkoxycarbonyl, C₁₋₈-alkoxycarbonyloxy-C₁₋₈-alkyl, C₁₋₈-alkyl, carboxy-C₁₋₈-alkyl, C₁₋₈-alkoxy-C₁₋₈-alkylsulfanyl, C₁₋₈-alkylsulfanyl, C₁₋₈-alkylsulfanyl-C₁₋₈-alkoxy, C₁₋₈-alkylsulfanyl-C₁₋₈-alkoxy-C₁₋₈-alkyl, C₁₋₈-alkylsulfanyl-C₁₋₈-alkyl, cyano, cyano-C₁₋₈-alkyl, C₃₋₈-cycloalkyl-C₀₋₆-alkoxy-C₁₋₈-alkoxy, C₁₋₆-alkoxy-C₀₋₆-alkyl-C₃₋₈-cycloalkyl-C₀₋₆-alkoxy-C₁₋₈-alkyl, C₃₋₈-cycloalkyl-C₀₋₆-alkoxy-C₁₋₈-alkyl, halo-C₁₋₈-alkyl, halogen, hydroxy-C₁₋₈-alkyl, hydroxyl, oxide, trifluoromethoxy or trifluoromethyl groups, or a C₁₋₈-alkylenedioxy group, and/or by an L1-T1-L2-T2-L3-T3-L4-T4-L5-U radical; L1, L2, L3, L4 and L5 are each independently a bond, C₁₋₈-alkylene, C₂₋₈-alkenylene or C₂₋₈-alkynylene, C₃₋₈-cycloalkene or are absent; T1, T2, T3 and T4 are each independently
   (a) a bond, or are absent, or are one of the groups
   (b) -CH(OH)-
   (c) -CH(OR⁶)-
   (d) -CH(NR⁵R⁶)-
   (e) -CO-
   (f) -CR⁷R⁸-
   (g) -O- or -NR⁶-
   (h) -S(O)₀₋₂-
   (i) -SO₂NR⁶-
   (j) -NR⁶SO₂-
   (k) -CONR⁶-
   (l) -NR⁶CO-
   (m) -O-CO-
   (n) -CO-O-
   (o) -O-CO-O-
   (p) -O-CO-NR⁶-
   (q) -N(R⁶)-CO-N(R⁶)-
   (r) -N(R⁶)-CO-O-
   (s) pyrrolidinylene, piperidinylene or piperazinylene
   (t) -C(R¹¹)(R¹²)-,
   where the bonds starting from (b)-(t) lead to a saturated or aromatic carbon atom of the adjacent group if the bond starts from a heteroatom, and where not more than two (b)-(f) groups, three (g)-(h) groups and one (i)-(t) group are present;
R³ is hydrogen, hydroxyl, C₁₋₈-alkoxy or C₂₋₈-alkenyloxy;
R⁴ is hydrogen, C₂₋₈-alkenyl, C₁₋₈-alkoxy, C₁₋₈-alkoxy-C₁₋₈-alkyl, C₁₋₈-alkoxy-C₁₋₈-alkoxy, C₁₋₈-alkyl, optionally (N-C₁₋₈-alkyl)-C₁₋₈-alkoxycarbonyl-amino-C₁₋₈-alkoxy, optionally (N-C₁₋₈-alkyl)-C₁₋₈-alkylcarbonyl-amino-C₁₋₈-alkoxy, optionally (N-mono- or N,N-di-C₁-C₈-alkyl)-amino-C₁₋₈-alkoxy, benzyl, C₃₋₈-cycloalkyloxy, C₃₋₈-cyclo-alkyloxy-C₁₋₈-alkoxy, heterocyclyl-C₀₋₈-alkoxy, heterocyclyloxy-C₁₋₈-alkoxy, hydroxy, hydroxy-C₁₋₈-alkoxy-C₁₋₈-alkoxy, hydroxy-C₁₋₈-alkyl, oxo or a
R^{4a}-Z1-X1- group where R^{4a} is
   (a) H-
   (b) C₁₋₈-alkyl-
   (c) C₂₋₈-alkenyl-
   (d) hydroxy-C₁₋₈-alkyl-
   (e) polyhydroxy-C₁₋₈-alkyl-
   (f) C₁₋₈-alkyl-O-C₁₋₈-alkyl-
   (g) aryl-
   (h) heterocyclyl-
   (i) arylalkyl-
   (j) heterocyclylalkyl-
   (k) aryloxyalkyl-
   (l) heterocyclyloxyalkyl-
   (m) (R⁵,R⁶)N-(CH₂)₁₋₃-
   (n) (R⁵,R⁶)N-
   (o) C₁₋₈-alkyl-S(O)₀₋₂-
   (p) aryl-S(O)₀₋₂-
   (q) heterocyclyl-S(O)₀₋₂-
   (r) HO-SO₃- or salts thereof
   (s) H₂N-C(NH)-NH-
   (t) NC-
   and the bonds starting from (n)-(t) lead to a carbon atom of the adjacent group and this carbon atom is saturated if the bond starts from a heteroatom;
Z1
   (a) is a bond, is absent, or is one of the groups
   (b) -C₁₋₈-alkylene-
   (c) -C₂₋₈-alkenylene-
   (d) -O-, -N(R¹¹)-, -S(O)₀₋₂-
   (e) -CO-
   (f) -O-CO-
   (g) -O-CO-O-
   (h) -O-CO-N(R¹¹)-
   (i) -N(R¹¹)-CO-O-
   (j) -CO-N(R¹¹)-
   (k) -N(R¹¹)-CO-
   (l) -N(R¹¹)-CO-N(R¹¹)-
   (m) -CH(OR⁹)-
   and the bonds starting from (d) and (f)-(m) lead to a carbon atom of the adjacent group and this carbon atom is saturated if the bond starts from a heteroatom;
X1
   (a) is a bond, is absent, or is one of the groups
   (b) -0-
   (c) -N(R¹¹)-
   (d) -S(O)₀₋₂-
   (e) -(CH₂)₁₋₃-;
or R³ and R⁴ in formula (I) together are a bond;
R⁵ and R⁶ are each independently hydrogen, C₁₋₈-alkyl, C₂₋₈-alkenyl, aryl-C₁₋₈-alkyl or acyl, or, together with the nitrogen atom to which they are bonded, are a 5- or 6-membered heterocyclic ring which may contain an additional nitrogen, oxygen or sulfur atom or a -SO-or -SO₂- group, and the additional nitrogen atom may optionally be substituted by C₁₋₈-alkyl radicals;
R⁷ and R⁸, together with the carbon atom to which they are bonded, are a 3-7-membered ring which may contain one or two -0- or -S - atoms or -SO- or -SO₂- groups;
R⁹ is hydrogen, C₁₋₈-alkyl, C₁₋₈-alkoxy-C₁₋₈-alkyl, acyl or arylalkyl; R¹⁰ is carboxyalkyl, alkoxycarbonylalkyl, alkyl or hydrogen;
R¹¹ is hydrogen or C₁₋₈-alkyl;
R¹² is hydrogen or C₁₋₈-alkyl;
Q is ethylene or is absent (formula I) or is ethylene or methylene (formula II);
U is hydrogen, C₁₋₈-alkyl, cyano, optionally substituted C₃₋₈-cycloalkyl, aryl or heterocyclyl;
W is oxygen or sulfur;
X is a bond, oxygen or sulfur, or is a >CH-R¹¹, >CHOR⁹, -O-CO-, >CO, >C=NOR¹⁰, -O-CHR¹¹- or -O-CHR¹¹-CO-NR⁹- group and the bond starting from an oxygen or sulfur atom leads to a saturated carbon atom of the Z group or to R¹;
Z is C₁₋₈-alkylene, C₂₋₈-alkenylene, hydroxy-C₁₋₈-alkylidene, -0-, -S-, -O-alk-, -S-alk-, -alk-O-, -alk-S- or -alk-NR⁹-, where alk is C₁₋₈-alkylene; and where
   (a) if Z is -0- or -S-, X is >CH-R¹¹ and either R² contains a L1-T1-L2-T2-L3-T3-L4-T4-L5-U substituent or R⁴ is a substituent other than hydrogen as defined above;
   (b) if Z is -O-alk- or -S-alk-, X is >CH-R¹¹; and
   (c) if X is a bond, Z is C₂₋₈-alkenylene, -alk-O- or -alk-S-;
   m is 0 or 1;
   n is 0 or 1;
and the pharmaceutically acceptable salts thereof.

### Group E: Compounds of the general formula

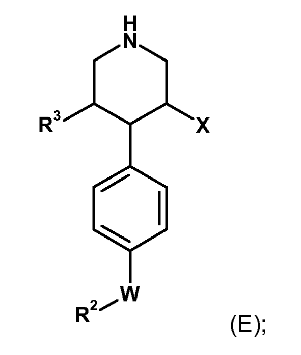

in which
(A) R¹ is heterocyclyl substituted by oxo or oxide or as indicated under (B) or (C), in particular azepanyl, benzo[1,3]dioxolyl, benzofuranyl, benzoimidazolyl, 4H-benzo[1,4]oxazinyl, benzooxazolyl, 4H-benzo[1,4]thiazinyl, quinolinyl, chromenyl, dihydrobenzo[e][1,4]diazepinyl, dihydrobenzofuranyl, 3,4-dihydro-2H-benzo[1,4]oxazinyl, dihydro-3H-benzo[1,4]oxazinyl, dihydrobenzo[d][1,3]oxazinyl, 3,4-dihydro-2H-benzo[1,4]thiazinyl, dihydro-2H-1λ6-benzo[1,4]thiazinyl, dihydro-1H-quinazolinyl, 1a,7b-dihydro-1H-cyclopropa[c]chromenyl, dihydroimidazolyl, 1,3-dihydroindolyl, 2,3-dihydroindolyl, dihydro-1H-pyrido[2,3-b][1,4]oxazinyl, indazolyl, indolyl, 3H-isobenzofuranyl, [1,5]naphthyridyl, oxazolyl, phthalazinyl, piperidinyl, pyrazolyl, 1H-pyrido[2,3-b][1,4]oxazinyl, pyridyl, 1 H-pyrrolizinyl, 1H-pyrrolo[2,3-b]pyridyl, pyrrolyl, tetrahydrobenzo[e][1,4]diazepinyl, 3H-thieno[2,3-d]pyrimidinyl, tetrahydro-quinoxalinyl, 1,1a,2,7b-tetrahydrocyclopropa[c]chromenyl, tetrahydropyranyl or triazinyl; or
(B) R¹ is aryl which is substituted by 1-4-acetamidinyl-C₁₋₈alkyl, acyl-C₁₋₈alkoxy-C₁₋₈alkyl, (N-acyl)-C₁₋₈alkoxy-C₁₋₈alkylamino, C₁₋₈alkoxy, C₁₋₈alkoxy-C₁₋₈alkoxy, C₁₋₈alkoxy-C₁₋₈alkoxy-C₁₋₈alkyl, C₁₋₈alkoxy-C₁₋₈alkyl, (N-C₁₋₈alkoxy)-C₁₋₈alkylaminocarbonyl-C₁₋₈alkoxy, (N-C₁₋₈alkoxy)-C₁₋₈alkylaminocarbonyl-C₁₋₈alkyl, C₁₋₈alkoxy-C₁₋₈alkylcarbamoyl, C₁₋₈alkoxy-C₁₋₈alkylcarbonyl, C₁₋₈alkoxy-C₁₋₈alkylcarbonylamino, 1-C₁₋₈alkoxy-C₁₋₈alkylimidazol-2-yl, 2-C₁₋₈alkoxy-C₁₋₈alkyl-4-oxoimidazol-1-yl, 1-C₁₋₈alkoxy-C₁₋₈-alkyltetrazol-5-yl, 5-C₁₋₈alkoxy-C₁₋₈-alkyltetrazol-1-yl, 6-alkoxy-aminocarbonyl-C₁₋₈alkoxy, C₁₋₈alkoxy-aminocarbonyl-C₁₋₈alkyl, C₁₋₈alkoxycarbonyl, C₁₋₈alkoxycarbonyl-C₁₋₈alkoxy, C₁₋₈alkoxycarbonyl-C₁₋₈alkyl, C₁₋₈alkoxycarbonylamino-C₁₋₈alkoxy, C₁₋₈alkoxycarbonylamino-C₁₋₈alkyl, C₁₋₈alkyl, (N-C₁₋₈alkyl)-C₁₋₈alkoxy-C₁₋₈alkylcarbamoyl, (N-C₁₋₈alkyl)-C₁₋₈alkoxy-C₁₋₈alkylcarbonylamino, (N-C₁₋₈alkyl)-C₁₋₈alkoxycarbonylamino, (N-C₁₋₈alkyl)-C₀₋₈alkylcarbonylamino-C₁₋₈alkoxy, (N-C₁₋₈alkyl)-C₀₋₈alkylcarbonylamino-C₁₋₈alkyl, (N-C₁₋₈alkyl)-C₁₋₈alkylsulfonylamino-C₁₋₈alkoxy, (N-C₁₋₈alkyl)-C₁₋₈alkylsulfonylamino-C₁₋₈alkyl, C₁₋₈alkylamidinyl, C₁₋₈alkylaminocarbonyl-C₁₋₈alkoxy, di-C₁₋₈alkylaminocarbonyl-C₁₋₈alkoxy, C₁₋₈alkylaminocarbonyl-C₁₋₈alkoxy-C₁₋₈alkyl, C₁₋₈alkylaminocarbonyl-C₁₋₈alkyl, C₁₋₈alkylaminocarbonylamino-C₁₋₈alkoxy, C₁₋₈alkyl-aminocarbonylamino-C₁₋₈alkyl, di-C₁₋₈alkylaminocarbonyl-C₁₋₈alkyl, C₁₋₈alkylamino-C₂₋₈alkoxy, di-C₁₋₈alkylamino-C₂₋₈alkoxy, C₁₋₈alkylamino-C₁₋₈alkyl, di-C₁₋₈alkylamino-C₁₋₈alkyl, C₁₋₈alkylcarbamoyl, di-C₁₋₈alkylcarbamoyl, C₀₋₈alkylcarbonylamino-C₁-₈alkoxy, C₀₋₈alkylcarbonylamino, C₀₋₈alkylcarbonylamino-C₁₋₈alkyl, C₁₋₈alkyl-carbonyloxy-C₁₋₈alkoxy, C₁₋₈alkylcarbonyloxy-C₁₋₈alkyl, C₁₋₈alkylsulfonyl, C₁₋₈alkylsulfonyl-C₁₋₈alkoxy, C₁₋₈alkylsulfonyl-C₁₋₈alkyl, C₁₋₈alkylsulfonylamino-C₁₋₈alkoxy, C₁₋₈alkylsulfonylamino-C₁₋₈alkyl, carbamoyl, carbamoyl-C₁₋₈alkoxy, carbamoyl-C₁₋₈alkyl, carboxy-C₁₋₈alkoxy, carboxy-C₁₋₈alkoxy-C₁₋₈alkyl, carboxy-C₁₋₈alkyl, cyano, cyano-C₁₋₈alkoxy, cyano-C₁₋₈alkyl, C₃₋₈cydoalkyl-C₁₋₈alkoxy, C₃₋₈cycloalkyl-C₁₋₈alkyl, C₃₋₈cycloalkylcarbonylamino-C₁₋₈alkoxy, C₃₋₈cycloalkylcarbonylamino-C₁₋₈alkyl, O,N-dimethylhydroxylamino-C₁₋₈alkyl, halogen, hydroxy-C₁₋₈alkoxy-C₁₋₈alkoxy, hydroxy-C₁₋₈alkoxy-C₁₋₈alkyl, hydroxy-C₁₋₈alkyl, (N-hydroxy)-C₁₋₈alkylaminocarbonyl-C₁₋₈alkoxy, (N-hydroxy)-C₁₋₈alkylaminocarbonyl-C₁₋₈alkyl, (N-hydroxy)aminocarbonyl-C₁₋₈alkoxy, (N-hydroxy)aminocarbonyl-C₁₋₈alkyl, 2-oxooxazolidinyl-C₁₋₈alkoxy, 2-oxooxazolidinyl-C₁₋₈alkyl, O-methyloximyl-C₁₋₈alkyl, polyhalO-C₁₋₈alkoxy or polyhalo-C₁₋₈alkyl; or
(C) R¹ is aryl which is substituted by 3-acetamidomethylpyrrolidinyl, 3-C₁₋₈alkoxy-C₁₋₈alkyl-pyrrolidinyl, 3,4-dihydroxypyrrolidinyl, 2,6-dimethylmorpholinyl, 3,5-dimethylmorpholinyl, dioxanyl, dioxolanyl, 4,4-dioxothiomorpholinyl, dithianyl, dithiolanyl, 2-hydroxymethylpyrrolidinyl, 4-hydroxypiperidinyl, 3-hydroxypyrrolidinyl, imidazolyl-alkoxy, imidazolylalkyl, 2-methylimidazolylalkoxy, 2-methylimidazolylalkyl, 3-methyl-[1,2,4]-oxadiazol-5-ylalkoxy, 5-methyl-[1,2,4]-oxadiazol-3-ylalkoxy, 3-methyl-[1,2,4]-oxadiazol-5-ylalkyl, 5-methyl-[1,2,4]-oxadiazol-3-ylalkyl, 4-methylpiperazinyl, 5-methyltetrazol-1-ylalkoxy, 5-methyltetrazol-1-ylalkyl, morpholinyl, [1,2,4]-oxadiazol-5-ylalkoxy, [1,2,4]-oxadiazol-5-ylalkyl, oxazol-4-ylalkoxy, oxazol-4-ylalkyl, 2-oxo-[1,3]oxazinyl, 2-oxooxazolidinyl, 2-oxoimidazolidinyl, 2-oxopyrrolidinyl, 4-oxo-piperidinyl, 2-oxopyrrolidinylalkoxy, 2-oxopyrrolidinylalkyl, 2-oxotetrahydropyrimidinyl 4-oxothiomorpholinyl, piperazinyl, piperidinyl, pyrrolidinyl, pyrrolyl, [1,2,4]-triazol-1-yl-alkoxy, [1,2,4]-triazol-4-ylalkoxy, [1,2,4]-triazol-1-ylalkyl, [1,2,4]-triazol-4-ylalkyl, tetrazol-1-ylalkoxy, tetrazol-2-ylalkoxy, tetrazol-5-ylalkoxy, tetrazol-1-ylalkyl, tetrazol-2-ylalkyl, tetrazol-5-ylalkyl, thiazol-4-ylalkoxy, thiazol-4-ylalkyl or thiomorpholinyl; or
(D) R¹ is aryl when X is -O-CHR⁶-CO-NR⁴-R¹ or -O-CHR⁶-CO-NR⁴-Z, where Z is Alk-R¹ where Alk is C₁₋₈alkylene; or
   (E) R¹ is aryl when X is -O-Z, where Z is Alk-NR⁴-R¹ or X is -Z, where Z is -Alk-NR⁴-R¹, where Alk is C₁₋₈alkylene;
   R² a) is absent when W is cyano; or
      b) is C₁₋₈alkyl, C₂₋₈alkenyl, C₂₋₈alkynyl, C₁₋₈alkoxy-C₁₋₈alkyl, C₁₋₈alkoxy-C₃₋₈cycloalkyl-C₁₋₈-alkyl, C₁₋₈alkylsulfanyl-C₁₋₈alkyl, C₁₋₈alkylsulfonyl-C₁₋₈alkyl when W is -O- or -S-;
   R³ a) is halogen- and/or hydroxy-substituted C₁₋₈alkoxy, halogen-and/or hydroxy-substituted C₁₋₈alkoxy-C₁₋₈alkoxy, branched C₁₋₈alkoxy-C₁₋₈alkoxy, optionally N-mono- or N,N-di-C₁₋₈-alkylated amino-C₁₋₈alkoxy, optionally N-C₁₋₈alkylated C₁₋₈alkoxy-C₁₋₈alkylamino-C₁₋₈alkoxy, optionally N-mono- or N,N-di-C₁₋₈alkylated amino-C₀₋₈-alkylcarbonyl-C₁₋₈alkoxy, hydroxy-C₀₋₈-alkylcarbonyl-C₀₋₈alkoxy, C₁₋₈alkoxy-C₀₋₈alkylcarbonyl-C₀₋₈alkoxy, C₁₋₈-alkylcarbonylamino-C₁₋₈alkoxy, cyano-C₁₋₈alkoxy, substituted C₃₋₈cydoalkyl-C₀₋₈alkoxy, heterocyclyl-C₀₋₈alkoxy, optionally N-C₁₋₈alkylated heterocyclyl-C₀₋₈alkylamino-C₀₋₈alkylcarbonyl-C₀₋₈alkoxy, C₁₋₈alkyl-sulfonyl-C₁₋₈alkoxy, C₂₋₈alkynyloxy, heterocyclyl-C₂₋₈alkynyloxy, optionally N-mono- or N,N-di-C₁₋₈alkylated amino-C₂₋₈alkynyloxy, N-mono- or N,N-di-C₁₋₈alkylated aminocarbonyl-C₂₋₈alkynyloxy, heterocyclyl-carbonyl-C₀₋₈alkoxy, optionally N-mono- or N,N-di-C₁₋₈alkylated amino-C₁₋₈alkyl, optionally N-C₁₋₈alkylated C₁₋₈alkoxy-C₁₋₈alkylamino-C₁₋₈alkyl, optionally N-mono- or N,N-di-C₁₋₈alkylated and optionally hydroxy-substituted amino-C₀₋₈alkyl-carbonyl-C₀₋₈alkyl, optionally N-C₁₋₈alkylated heterocyclyl-C₀₋₈alkylamino-C₀₋₈alkylcarbonyl-C₀₋₈alkyl, optionally halogen- or hydroxy-substituted C₁₋₈alkoxy-C₁₋₈alkyl, optionally halogen- or hydroxy-substituted hydroxy-C₁₋₈alkyl, optionally N-C₁₋₈alkylated hydroxy-C₁₋₈alkylamino-C₁₋₈alkyl, hetero-cyclylcarbonyl-C₀₋₈alkyl, heterocyclylcarbonyl-C₀₋₈alkylamino-C₁₋₈alkyl, heterocyclyl-C₁₋₈alkyl, C₁₋₈alkoxycarbonylamino-C₁₋₈alkyl, optionally halogen-substituted heterocyclyl-C₀₋₈alkylcarbonylamino-C₁₋₈alkyl, optionally halogen-substituted C₃₋₈cydoalkyl-C₀₋₈alkylcarbonylamino-C₁₋₈alkyl or optionally halogen-substituted C₁₋₈alkylcarbonylamino-C₁₋₈alkyl; or additionally
      b) is hydroxy, unsubstituted C₁₋₈alkoxy, unsubstituted, unbranched C₁₋₈alkoxy-C₁₋₈-alkoxy or unsubstituted C₃₋₈cycloalkyl-C₀₋₈alkoxy if -W-R² is not C₁₋₈alkoxy;
   R⁴ is acyl, C₁₋₈alkoxy-C₁₋₈alkyl, C₁₋₈alkyl, aryl-C₁₋₈alkyl, C₃₋₈cycloalkyl-C₀₋₈alkyl, or hydrogen;
   R⁵ is C₁₋₈alkoxycarbonyl-C₁₋₈alkyl, C₁₋₈alkyl, carboxy-C₁₋₈alkyl or hydrogen;
   R⁶ is acyl, C₂₋₈alkenyl, C₁₋₈alkyl, aryl-C₁₋₈alkyl or hydrogen;
   X is Z, -O-Z or -S-Z, where the bond originating from an oxygen or sulfur atom leads to a saturated C atom of the group Z, or is a group -CHR⁶-Z, -CHOR⁴-Z, -O-CO-Z, -O-CO-R¹, -CO-Z, -C=NOR⁵-Z, -O-CHR⁶-Z, -O-CHR⁶-CO-NR⁴-Z, -O-CHR⁶-CO-NR⁴-R¹, or -O-CHR⁶-R¹;
   W is -O-, -S- or cyano;
   Z is C₁₋₈-Alk-R¹, C₂₋₈alkenylene-R¹, hydroxy-substituted -Alk-R¹, -OR¹, -S-R¹, -O-Alk-R¹,
   -S-Alk-R¹, -Alk-O-R¹, -Alk-S-R¹ or -Alk-NR⁴-R¹, where Alk is C₁₋₈alkylene; and where
      (a) X is -CH-R⁶-Z if Z is -O-R¹ or -S-R¹
      (b) X is -CH-R⁶-Z if Z is -O-Alk-R¹ or -S-Alk-R¹; and
      (c) Z is C₂₋₈alkenylene-R¹, -Alk-O-R¹, -Alk-S-R¹ or -Alk-NR⁴-R¹ if X is Z;
and the pharmaceutically acceptable salts thereof.

### Group F: Compounds of the general formula

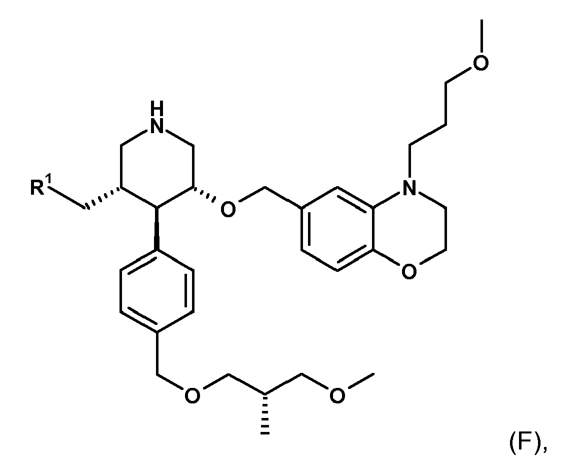

in which
R¹ is straight-chain C₁₋₈-alkanoyloxy, straight-chain C₁₋₈-alkoxy, straight-chain C₁₋₈-alkoxy-straight-chain-C₁₋₈-alkoxy, straight-chain C₁₋₈-alkoxycarbonylamino, straight-chain C₀₋₈-alkylcarbonylamino, optionally N-mono- or N,N-di-C₁₋₈-alkylated amino or hydroxy or straight-chain omega-hydroxy-C₁₋₈-alkyl;
and the pharmaceutically acceptable salts thereof.

Delta-amino-gamma-hydroxy-omega-(hetero)aryl alkanoic acid amide derivatives particularly useful in the context of the present invention are those compounds described in EP 678503, WO 2005/090305, WO 2006/095020 and WO 2007/031558., which references are herein incorporated in their entirety. Very particularly useful delta-amino-gamma-hydroxy-omega-(hetero)aryl alkanoic acid amide derivatives are compounds.of the following groups G to J.

### Group G: Compounds of the general formula

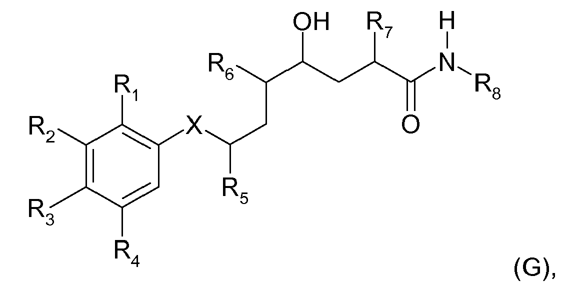

wherein
R₁ is hydrogen, hydroxy, lower alkoxy, cycloalkoxy, lower alkoxy-lower alkoxy or free or esterified or amidareal carboxy-lower alkoxy, R₂ is hydrogen, lower alkyl, cycloalkyl, lower alkoxy-lower alkyl, lower alkoxy-lower alkoxy-lower alkyl, cycloalkoxy-lower alkyl, hydroxy, optionally lower alkanoylated, halogenated or sulfonylated hydroxy-lower alkoxy; amino-lower alkyl that is unsubstituted or substituted by lower alkyl, by lower alkanoyl and/or by lower alkoxycarbonyl; optionally hydrogenated heteroaryl-lower alkyl; amino-lower alkoxy that is substituted by lower alkyl, by lower alkanoyl and/or by lower alkoxycarbonyl; oxo-lower alkoxy, lower alkoxy, cycloalkoxy, lower alkenyloxy, cycloalkoxy-lower alkoxy, lower alkoxy-lower alkoxy, lower alkoxy-lower alkenyl, lower alkenyloxy-lower alkoxy, lower alkoxy-lower alkenyloxy, lower alkenyloxy-lower alkyl, lower alkanoyl-lower alkoxy, optionally S-oxidised lower alkylthio-lower alkoxy, lower alkylthio-(hydroxy)-lower alkoxy, aryl-lower alkoxy, optionally hydrogenated heteroaryl-lower alkoxy, cyano-lower alkoxy, free or esterified or amidated carboxy-lower alkoxy or free or esterified or amidated carboxy-lower alkyl,
R₃ is optionally halogenated lower alkyl, lower alkoxy-lower alkyl, cycloalkoxy-lower alkyl, hydroxy-lower alkyl, optionally S-oxidised lower alkylthio-lower alkyl, optionally hydrogenareal heteroarylthio-lower alkyl, optionally hydrogenated heteroaryl-lower alkyl; amino-lower alkyl that is unsubstituted or N-mono- or N,N-di-lower alkylated, N-lower alkanoylated or N-lower alkanesulfonylated or N,N-disubstituted by lower alkylene, by unsubstituted or N'-lower alkylated or N'-lower alkanoylated aza-lower alkylene, by oxa-lower alkylene or by optionally S-oxidised thia-lower alkylene; cyano-lower alkyl, free or esterified or amidated carboxy-lower alkyl, cycloalkyl, aryl, hydroxy, lower alkoxy, cycloalkoxy, lower alkoxy-lower alkoxy, cycloalkoxy-lower alkoxy, hydroxy-lower alkoxy, aryl-lower alkoxy, optionally halogenated lower alkoxy, optionally S-oxidised lower alkylthio-lower alkoxy, optionally hydrogenated heteroaryl-lower alkoxy, optionally hydrogenated heteroarylthio-lower alkoxy; amino-lower alkoxy that is unsubstituted or N-mono- or N,N-di-lower alkylated, N-lower alkanoylated or N-lower alkanesulfonylated or substituted by lower alkylene, by unsubstituted or N'-lower alkylated or N'-lower alkanoylated aza-lower alkylene, by oxa-lower alkylene or by optionally S-oxidised thia-lower alkylene; cyano-lower alkoxy or free or esterified or amidated carboxy lower alkoxy, or together with R4 is lower alkylenedioxy or a fused-on benzo or cyclohexeno ring, R₄ together with R₃ is lower alkylenedioxy or a fused-on benzo or cyclohexeno ring, or is hydrogen, lower alkyl, hydroxy, lower alkoxy or cycloalkoxy,
X is methylene or hydroxymethylene,
R₅ is lower alkyl or cycloalkyl,
R₆ is unsubstituted or N-mono- or N,N-di-lower alkylated or N-lower alkanoylated amino,
R₇ is lower alkyl, lower alkenyl, cycloalkyl or aryl-lower alkyl, and
R₈ is lower alkyl, cycloalkyl, free or aliphatically esterideal or etherideal hydroxy-lower alkyl; amino-lower alkyl that is unsubstituted or N-lower alkanoylated or N-mono- or N,N-di-lower alkylated or N,N-disubstituted by lower alkylene, by hydroxy-, lower alkoxy- or lower alkanoyloxy-lower alkylene, by unsubstituted or N'-lower alkanoylated or N'-lower alkylated aza-lower alkylene, by oxa-lower alkylene or by optionally S-oxidised thia-lower alkylene; free or esterified or amidated carboxy-lower alkyl, free or esterified or amidated dicarboxy-lower alkyl, free or esterideal or amidated carboxy-(hydroxy)-lower alkyl, free or esterified or amidated carboxycycloalkyl-lower alkyl, cyano-lower alkyl, lower alkanesulfonyl-lower alkyl, unsubstituted or N-mono- or N,N-di-lower alkylated thiocarbamoyl-lower alkyl, unsubstituted or N-mono- or N,N-di-lower alkylated sulfamoyl-lower alkyl, or a heteroaryl radical bonded via a carbon atom and optionally hydrogenated and/or oxo-substituted, or lower alkyl substituted by a heteroaryl radical bonded via a carbon atom and optionally hydrogenated and/or oxo-substituted,
and the pharmaceutically acceptable salts thereof,

### Group H: Compounds of the general formula

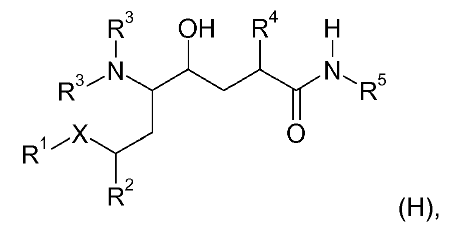

where
X is -CH₂- or >CH-OH;
   (A) R¹ is an optionally substituted heterocyclyl radical or an optionally substituted polycyclic, unsaturated hydrocarbon radical where X is hydroxymethylene; or
   (B) R¹ is a heterocyclyl radical or a polycyclic, unsaturated hydrocarbon radical which is substituted by one to four radicals selected from C₁-C₆-alkyl, C₃₋₈-cycloalkyl, C₃₋₈-cycloalkoxy, C₃₋₈-cycloalkoxy-C₁₋₆-alkyl, C₃₋₈-cycloalkoxy-C₁₋₆-alkoxy, C₁-C₆-alkylamino, di-C₁-C₆-alkylamino, amino-C₁₋₆-alkyl, amino-C₂₋₇-alkoxy, polyhalo-C₁₋₆-alkyl, polyhalo-C₂₋₇-alkoxy, nitro, amino, oxo, oxide, C₂-C₆-alkenyl, C₁-C₆-alkoxy, C₁-C₆-alkanoyloxy, hydroxy, halogen, cyano, carbamoyl, carboxyl, C₁-C₆-alkylenedioxy, phenyl, phenoxy, phenylthio, phenyl-C₁-C₆-alkyl or phenyl-C₁-C₆-alkoxy, pyridylcarbonylamino-C₁₋₆-alkyl, C₂₋₇-alkenyloxy, C₁₋₆-alkoxy-C₁₋₆-alkoxy, C₁₋₆-alkoxy-C₁₋₆-alkoxy-C₁₋₆-alkyl, methoxybenzyloxy, hydroxybenzyloxy, methylenedioxybenzyloxy, dioxolanyl-C₁₋₆-alkoxy, C₃₋₈-cycloalkyl-C₁₋₆-alkyl, C₃₋₈-cycloalkyl-C₁₋₆-alkoxy, hydroxy-C₂₋₇-alkoxy, carbamoyloxy-C₂₋₇-alkoxy, pyridylcarbamoyloxy-C₂₋₇-alkoxy, benzoyloxy-C₂₋₇-alkoxy, C₁₋₆-alkoxycarbonyl, C₁₋₆-alkylcarbonylamino, C₁₋₆-alkylcarbonylamino-C₁₋₆-alkyl, C₁₋₆-alkylcarbonylamino-C₂₋₇-alkoxy, (N-C₁₋₆-alkyl)-C₁₋₆-alkylcarbonylamino-C₁₋₆-alkyl, (N-C₁₋₆-alkyl)-C₁₋₆-alkylcarbonylamino-C₂₋₇-alkoxy, C₃₋₈-cycloalkylcarbonyl-amino-C₁₋₆-alkyl, C₃₋₈-cycloalkylcarbonylamino-C₂₋₇-alkoxy, C₁₋₆-alkoxy-C₁₋₆-alkyl, hydroxy-C₁-₆-alkyl, hydroxy-C₂₋₇-alkoxy-C₁₋₆-alkyl, hydroxy-C₂₋₇-alkoxy-C₁₋₆-alkoxy, C₁₋₆-alkoxycarbonylamino-C₁₋₆-alkyl, C₁₋₆-alkoxycarbonylamino-C₂₋₇-alkoxy, C₁₋₆-alkylaminocarbonylamino-C₁₋₆-alkyl, C₁₋₆-alkylaminocarbonylamino-C₂₋₇-alkoxy, C₁₋₆-alkylaminocarbonyl-C₁₋₆-alkyl, C₁₋₆-alkylaminocarbonyl-C₁₋₆-alkoxy, C₁₋₆-alkylaminocarbonyl-C₁₋₆-alkoxy-C₁₋₆-alkyl, di-C₁₋₆-alkylaminocarbonyl-C₁₋₆-alkyl, di-C₁₋₆-alkylaminocarbonyl-C₁₋₆-alkoxy, C₁₋₆-alkylcarbonyl-oxy-C₁₋₆-alkyl, C₁₋₆-alkylcarbonyloxy-C₂₋₆-alkoxy, cyano-C₁₋₆-alkyl, cyano-C₁₋₆-alkoxy, 2-oxooxazolidinyl-C₁₋₆-alkyl, 2-oxooxazolidinyl-C₁₋₆-alkoxy, C₁₋₆-alkoxycarbonyl-C₁₋₆-alkyl, C₁₋₆-alkoxycarbonyl-C₁₋₆-alkoxy, C₁₋₆-alkylsulphonylamino-C₁₋₆-alkyl, C₁₋₆-alkylsulphonylamino-C₁₋₆-alkoxy, (N-C₁₋₆-alkyl)-C₁₋₆-alkylsulphonylamino-C₁₋₆-alkyl, (N-C₁₋₆-alkyl)-C₁₋₆-alkylsulphonylamino-C₂₋₇-alkoxy, C₁₋₆-alkylamino-C₁₋₆-alkyl, C₁₋₆-alkylamino-C₂₋₇-alkoxy, di-C₁₋₆-alkylamino-C₁₋₆-alkyl, di-C₁₋₆-alkylamino-C₂₋₇-alkoxy, C₁₋₆-alkylsulphonyl-C₁₋₆-alkyl, C₁₋₆-alkylsulphonyl-C₁₋₆-alkoxy, carboxy-C₁₋₆-alkyl, carboxy-C₁₋₆-alkoxy, carboxy-C₁₋₆-alkoxy-C₁₋₆-alkyl, C₁₋₆-alkoxy-C₁₋₆-alkylcarbonyl, acyl-C₁₋₆-alkoxy-C₁₋₆-alkyl, (N-C₁₋₆-alkyl)-C₁₋₆-alkoxy-carbonylamino, (N-hydroxy)-C₁₋₆-alkylaminocarbonyl-C₁₋₆-alkyl, (N-hydroxy)-C₁₋₆-alkylaminocarbonyl-C₁₋₆-alkoxy, (N-hydroxy)aminocarbonyl-C₁₋₆-alkyl, (N-hydroxy)aminocarbonyl-C₁₋₆-alkoxy, C₁₋₆-alkoxyaminocarbonyl-C₁₋₆-alkyl, 6-alkoxyaminocarbonyl-C₁₋₆-alkoxy, (N-C₁₋₆-alkoxy)-C₁₋₆-alkylaminocarbonyl-C₁₋₆-alkyl, (N-C₁₋₆-alkoxy)-C₁₋₆-alkylaminocarbonyl-C₁₋₆-alkoxy, (N-acyl)-C₁₋₆-alkoxy-C₁₋₆-alkylamino, C₁₋₆-alkoxy-C₁₋₆-alkylcarbamoyl, (N-C₁₋₆-alkyl)-C₁₋₆-alkoxy-C₁₋₆-alkylcarbamoyl, C₁₋₆-alkoxy-C₁₋₆-alkylcarbonyl, C₁₋₆-alkoxy-C₁₋₆-alkylcarbonylamino, (N-C₁₋₆-alkyl)-C₁₋₆-alkoxy-C₁₋₆-alkylcarbonylamino, 1-C₁₋₆-alkoxy-C₁₋₆-alkylimidazol-2-yl, 1-C₁₋₆-alkoxy-C₁₋₆-alkyltetrazol-5-yl, 5-C₁₋₆-alkoxy-C₁₋₆-alkyltetrazol-1-yl, 2-C₁₋₆-alkoxy-C₁₋₆-alkyl-4-oxoimidazol-1-yl, carbamoyl-C₁₋₆-alkyl, carbamoyl-C₁₋₆-alkoxy, C₁₋₆-alkylcarbamoyl, di-C₁₋₆-alkylcarbamoyl, C₁₋₆-alky-Isulphonyl, C₁₋₆-alkylamidinyl, acetamidinyl-C₁₋₆-alkyl, O-methyl-oximyl-C₁₋₆-alkyl, O,N-dimethylhydroxylamino-C₁₋₆-alkyl, C₃₋₆-cycloalkyl-C₁₋₆-alkanoyl, aryl-C₁₋₆-alkanoyl or heterocyclyl-C₁₋₆-alkanoyl, each of which is optionally substituted by halogen, C₁-C₆-alkyl, C₁₋₆-alkoxy, hydroxy, C₁-C₆-alkylamino, di-C₁-C₆-alkylamino, C₁₋₆-alkoxycarbonyl, hydroxy-C₁₋₆-alkyl or trifluoromethyl, and also pyridyl, pyridyloxy, pyridylthio, pyridylamino, pyridyl-C₁₋₆-alkyl, pyridyl-C₁₋₆-alkoxy, pyrimidinyl, pyrimidinyloxy, pyrimidinylthio, pyrimidinylamino, pyrimidinyl-C₁₋₆-alkyl, pyrimidinyl-C₁₋₆-alkoxy, thienyl, thienyl-C₁₋₆-alkyl, thienyl-C₁₋₆-alkoxy, furyl, furyl-C₁₋₆-alkyl or furyl-C₁₋₆-alkoxy, piperidinoalkyl, piperidinoalkoxy, piperidinoalkoxyalkyl, morpholinoalkyl, morpholinoalkoxy, morpholinoalkoxyalkyl, piperazinoalkyl, piperazinoalkoxy, piperazinoalkoxyalkyl, [1,2,4]triazol-1-ylalkyl, [1,2,4]triazol-1-ylalkoxy, [1,2,4]triazol-4-ylalkyl, [1,2,4]triazol-4-ylalkoxy, [1,2,4]oxadiazol-5-ylalkyl, [1,2,4]oxadiazol-5-ylalkoxy, 3-methyl[1,2,4]oxadiazol-5-ylalkyl, 3-methyl[1,2,4]oxadiazol-5-ylalkoxy, 5-methyl[1,2,4]oxadiazol-3-ylalkyl, 5-methyl[1 ,2,4]oxadiazol-3-ylalkoxy, tetrazol-1-ylalkyl, tetrazol-1-ylalkoxy, tetrazol-2-ylalkyl, tetrazol-2-ylalkoxy, tetrazol-5-ylalkyl, tetrazol-5-ylalkoxy, 5-methyltetrazol-1-ylalkyl, 5-methyltetrazol-1-ylalkoxy, thiazol-4-ylalkyl, thiazol-4-ylalkoxy, oxazol-4-ylalkyl, oxazol-4-ylalkoxy, 2-oxopyrrolidinylalkyl, 2-oxopyrrolidinylalkoxy, imidazolylalkyl, imidazolyl-alkoxy, 2-methylimidazolylalkyl, 2-methylimidazolylalkoxy or N-methylpiperazinoalkyl, N-methylpiperazinoalkoxy, N-methylpiperazinoalkoxyalkyl, dioxolanyl, dioxanyl, dithiolanyl, dithianyl, pyrrolidinyl, piperidinyl, piperazinyl, pyrrolyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 3-acetamidomethylpyrrolidinyl, 3-C₁₋₆-alkoxy-C₁₋₆-alkylpyrrolidinyl, 4-hydroxypiperidinyl, 4-oxo-piperidinyl, 3,5-dimethylmorpholinyl, 4,4-dioxothiomorpholinyl, 4-oxo-thiomorpholinyl, 2,6-dimethylmorpholinyl, 2-oxoimidazolidinyl, 2-oxooxazolidinyl, 2-oxopyrrolidinyl, 2-oxo[1,3]oxazinyl, 2-oxotetrahydropyrimidinyl, each of which is optionally substituted by halogen, C₁₋₆-alkyl, C₁₋₆-alkoxy or dihydroxy-C₁₋₆-alkylaminocarbonyl, and the -O-CH₂CH(OH)CH₂NRₓ radical where NRₓ is a mono- or di-C₁₋₆-alkylamino, piperidino, morpholino, piperazino or N-methylpiperazino radical,
      where, in the case that R¹ is naphthyl or cyclohexenophenyl, at least the ring system not bonded to X is substituted as specified; or
   (C) R¹ is pyrazinyl, triazolyl, imidazolyl, benzthiazolyl, pyranyl, tetrahydropyranyl, azetidinyl, morpholinyl, quinazolinyl, quinoxalinyl, isoquinolyl, benzo[b]thienyl, isobenzofuranyl, benzimidazolyl, 2-oxobenzimidazolyl, oxazolyl, thiazolyl, pyrrolyl, pyrazolyl, triazinyl, dihydrobenzofuranyl, 2-oxodihydrobenzo[d][1,3]oxazinyl, 4-oxodihydroimidazolyl, 5-oxo-4H-[1,2,4]triazinyl, 3-oxo-4H-benzo[1,4]thiazinyl, tetrahydroquinoxalinyl, 1,1,3-trioxodihydro-2H-1λ⁶-benzo[1,4]thiazinyl, 1-oxopyridyl, dihydro-3H-benzo[1,4]oxazinyl, 2-oxotetrahydrobenzo[e][1,4]diazepinyl, 2-oxodihydrobenzo[e][1,4]-diazepinyl, 1H-pyrrolizinyl, phthalazinyl, 1-oxo-3H-isobenzofuranyl, 4-oxo-3H-thieno[2,3-d]pyrimidinyl, 3-oxo-4H-benzo[1,4]oxazinyl, [1,5]naphthyridyl, dihydro-2H-benzo[1,4]thiazinyl, 1,1-dioxodihydro-2H-benzo[1,4]thiazinyl, 2-oxo-1H-pyrido[2,3-b][1,4]oxazinyl, dihydro-1H-pyrido[2,3-b][1,4]oxazinyl, 1 H-pyrrolo[2,3-b]pyridyl, benzo-[1,3]dioxolyl, benzooxazolyl, 2-oxobenzooxazolyl, 2-oxo-1,3-dihydroindolyl, 2,3-dihydroindolyl, indazolyl, benzofuranyl, dioxolanyl, dioxanyl, dithiolanyl, dithianyl, pyrrolidinyl, piperidinyl, piperazinyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, tetrahydropyranyl, 2-oxoimidazolidinyl, 2-oxooxazolidinyl, 2-oxo-piperidinyl, 2-oxopyrrolidinyl, 2-oxo[1,3]oxazinyl, 2-oxoazepanyl, or 2-oxotetrahydropyrimidinyl;
R² is C₁-C₆-alkyl or C₃-C₆-cycloalkyl;
R³ are each independently H, C₁-C₆-alkyl, C₁₋₆-alkoxycarbonyl or C₁-C₆-alkanoyl;
R⁴ is C₁-C₆-alkyl, C₃-C₆-cycloalkyl, C₂-C₆-alkenyl or unsubstituted or substituted aryl-C₁-C₆-alkyl;
R⁵ is C₁-C₆-alkyl, C₁-C₆-hydroxyalkyl, C₁-C₆-alkoxy-C₁-C₆-alkyl, C₁-C₆-alkanoyloxy-C₁-C₆-alkyl, C₁-C₆-aminoalkyl, C₁-C₆-alkylamino-C₁-C₆-alkyl, C₁-C₆-dialkylamino-C₁-C₆-alkyl, C₁-C₆-alkanoylamido-C₁-C₆-alkyl, HO(O)C-C₁-C₆-alkyl, C₁-C₆-alkyl-O-(O)C-C₁-C₆-alkyl, H₂N-C(O)-C₁-C₆-alkyl, C₁-C₆-alkyl-HN-C(O)-C₁-C₆-alkyl, (C₁-C₆-alkyl)₂N-C(O)-C₁-C₆-alkyl, C₂-C₈-alkenyl, C₂-C₈-alkynyl, cyano-C₁-C₆-alkyl, halo-C₁-C₆-alkyl, optionally substituted aryl-C₀₋C₆-alkyl, optionally substituted C₃-C₈-cydoalkyl-C₀₋C₆-alkyl or optionally substituted heterocyclyl-C₀₋C₆-alkyl, ,
and the pharmaceutically acceptable salts thereof.

### Group I: Compounds of the general formula

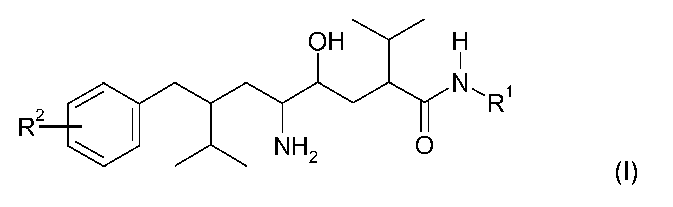

in which
R¹ is a) saturated heterocyclyl which is optionally substituted one or more times by C₁₋₈- alkanoyl, C₂₋₆alkenyl, C₂₋₆alkynyl, C₁₋₆alkoxy, C₁₋₆alkoxy-C₁₋₆alkoxy, C₁₋₆alkoxy-C₁-₆alkyl, C₁₋₆alkoxycarbonylamino, C₁₋₆alkyl, C₀₋₆alkylcarbonylamino, C₁₋₆alkylcarbonyl-oxy, C₁₋₆alkylenedioxy, optionally N-mono or N,N-di-C₁₋₆-alkylated amino, aryl, optionally N-mono or N,N-di-C₁₋₆-alkylated carbamoyl, optionally esterified carboxy, cyano, C₃₋₈cydoalkoxy, C₃₋₈cycloalkyl-C₀₋₆alkyl, halogen, halo-C₁₋₆alkoxy, halo-C₁₋₆alkyl, heteroaryl, unsaturated, partially saturated or saturated heterocyclyl, hydroxy, nitro, oxide or oxo which is bonded via a C atom; or
   b) azepanyl-, azetidinyl-, aziridinyl-, dioxanyl-, dioxepanyl-, dioxolanyl-, dithianyl-, dithiolanyl-, furanyl-, oxathianyl-, oxepanyl-, tetrahydropyranyl-, tetrahydrofuranyl-, tetrahydrothiophenyl-, tetrahydrothiopyranyl-, thiepanyl- or bicyclic saturated heterocyclyl-C₁₋₄alkyl each of which is optionally substituted one or more times by C₁₋₈alkanoyl, C₂₋₆alkenyl, C₂₋₆alkynyl, C₁₋₆alkoxy, C₁₋₆alkoxy-C₁₋₆alkoxy, C₁₋₆alkoxy-C₁₋₆alkyl, C₁₋₆alkoxycarbonylamino, C₁₋₆alkyl, C₀₋₆alkylcarbonylamino, C₁₋₆alkylcarbonyloxy, C₁-₆alkylenedioxy, optionally N-mono or N,N-di-C₁₋₆-alkylated amino, aryl, optionally N-mono or N,N-di-C₁₋₆-alkylated carbamoyl, optionally esterified carboxy, cyano, C₃₋₈cycloalkoxy, C₃₋₈cycloalkyl-C₀₋₆alkyl, halogen, halo-C₁₋₆alkoxy, halo-C₁₋₆alkyl, heteroaryl, unsaturated, partially saturated or saturated heterocyclyl, hydroxy, nitro, oxide or oxo; or
   c) C₂₋₈alkynyl which is optionally substituted one or more times by C₁₋₈alkanoyl, C₂₋₆alkenyl, C₂₋₆alkynyl, C₁₋₆alkoxy, C₁₋₆alkoxy-C₁₋₆alkoxy, C₁₋₆alkoxy-C₁₋₆alkyl, C₁₋₆alkoxycarbonylamino, C₁₋₆alkyl, C₀₋₆alkylcarbonylamino, C₁₋₆alkylcarbonyloxy, C₁₋₆alkylenedioxy, optionally N-mono or N,N-di-C₁₋₆-alkylated amino, aryl, optionally N-mono or N,N-di-C₁₋₆-alkylated carbamoyl, optionally esterified carboxy, cyano, C₃₋₈cydoalkoxy, C₃₋₈cycloalkyl-C₀₋₆alkyl, halogen, halo-C₁₋₆-alkoxy, halo-C₁₋₆alkyl, heteroaryl, unsaturated, partially saturated or saturated heterocyclyl, hydroxy, nitro or oxo; or
R² is, independently of one another, 1-4 radicals selected from: C₁₋₈alkanoyl, C₁₋₈alkanoyl-C₂₋₄alkoxy bearing the alkanoyl group in a position higher than C₁₋₈alkanoylamino-C₁₋₄alkoxy, N-C₁₋₄alkanoylamino-C₁₋₄alkyl, C₁₋₈alkanoyloxy-C₁₋₄alkyl, N'-C₂₋₈alkanoylpiperazino-C₁₋₄alkoxy, N'-C₂₋₈alkanoylpiperazino-C₁₋₄alkyl, C₁₋₈alkanesulphonyl-C₁₋₄alkoxy, C₁₋₈alkanesulphonyl-C₁₋₄alkyl, C₁₋₈alkanesulphonylamino-C₁₋₄alkoxy, C₁₋₄alkanesulphonylamino-C₁₋₄alkyl, C₁₋₈alkanesulphonyl-C₁₋₄(hydroxy)alkoxy, C₂₋₈alkenyloxy, C₂₋₈alkenyloxy-C₁₋₄alkoxy, C₂₋₈alkenyloxy-C₁₋₄alkyl, C₁₋₈alkoxy, C₁₋₆alkoxy-C₁₋₆alkanoyl, C₁₋₄alkoxy-C₂₋₄alkenyl, C₁₋₄alkoxy-C₂₋₄alkenyloxy, C₁₋₄alkoxy-C₁₋₄alkoxy, C₁₋₄alkoxy-C₁₋₄alkoxy-C₁₋₄alkyl, C₁₋₄alkoxy-C₁₋₄alkyl, C₁₋₄alkoxycarbonyl-C₁₋₄alkoxy, C₁₋₄alkoxycarbonyl-C₁₋₄alkyl, C₁₋₈alkoxycarbonylamino-C₁₋₄alkoxy, C₁₋₈alkoxycarbonylamino-C₁₋₄alkyl, C₁₋₈alkyl, C₁₋₄alkylamino, N,N-di-C₁₋₄alkylamino, C₁₋₄alkylamino-C₁₋₄alkoxy, N,N-di-C₁₋₄alkylamino-C₁₋₄alkoxy, C₁₋₄alkylamino-C₁₋₄alkyl, N,N-di-C₁₋₄alkylamino-C₁₋₄alkyl, N-mono- or N,N-di-C₁₋₄alkylcarbamoyl-C₁₋₄alkoxy, N-mono- or N,N-di-C₁₋₄alkylcarbamoyl-C₁₋₄alkyl, N'-C₁₋₄alkylpiperazino-C₁₋₄alkoxy, N'-C₁₋₄alkyl-piperazino-C₁₋₄alkyl, C₁₋₄alkylthio-C₁₋₄alkoxy, C₁₋₄alkylthio-C₁₋₄(hydroxy)alkoxy, C₁₋₄alkylthio-C₁₋₄alkyl, amino-C₂₋₄alkoxy, amino-C₁₋₄alkyl, carbamoyl-C₁₋₄alkoxy, carbamoyl-C₁₋₈alkyl, carboxy-C₁₋₄alkoxy, carboxy-C₁₋₄alkyl, cyano-C₁₋₄alkoxy, cyano-C₁₋₄alkyl, C₃₋₈-cycloalkoxy, C₃₋₈cycloalkoxy-C₁₋₄alkoxy, C₃₋₈cycloalkoxy-C₁₋₄alkyl, C₃₋₈cycloalkyl, S,S-dioxothiomorpholino-C₁₋₄alkoxy, S.S-dioxothio-morpholino-C₁₋₄alkyl, halogen, halo-C₁₋₄alkoxy, halo-C₁₋₄alkyl, halo-C₂₋₈(hydroxy)alkoxy, hydroxy, hydroxy-C₂₋₈alkoxy, hydroxy-C₂₋₈alkyl, imidazolylthio-C₁₋₄alkoxy, imidazolylthio-C₁₋₄alkyl, optionally N-oxidized morpholino-C₁₋₄alkoxy, morpholino-C₁₋₄alkyl, S-oxothio-morpholino-C₁₋₄alkoxy, S-oxothiomorpholino-C₁₋₄alkyl, piperazino-C₁₋₄alkoxy, piperazino-C₁₋₄alkyl, piperidino-C₁₋₄alkoxy, piperidino-C₁₋₄alkyl, optionally partially hydrogenated pyridyl- or N-oxidopyridyl-C₁₋₄alkoxy, optionally partially hydrogenated pyridyl- or N-oxido-pyridyl-C₁₋₄alkyl, optionally N-oxidized pyridylthio-C₁₋₄alkoxy, optionally N-oxidized pyridylthio-C₁₋₄alkyl, pyrimidinylthio-C₁₋₄alkoxy, pyrimidinylthio-C₁₋₄alkyl, pyrrolidino-C₁₋₄alkoxy, pyrrolidino-C₁₋₄alkyl, thiazolinylthio-C₁₋₄alkoxy, thiazolinylthio-C₁₋₄alkyl, thiazolyl-C₁₋₄alkoxy, thiazolylthio-C₁₋₄alkoxy, thiazolylthio-C₁₋₄alkyl, thio-morpholino-C₁₋₄alkoxy, thiomorpholino-C₁₋₄alkyl, trifluoro-C₁₋₈alkanesulphonyl-C₁₋₄alkoxy, trifluoro-C₁₋₈alkanesulphonylamino-C₁₋₄alkyl, phenyl or naphthyl which is unsubstituted or mono-, di- or trisubstituted by C₁₋₄alkoxy, C₁₋₄alkyl, C₁₋₄alkylamino, di-C₁₋₄alkylamino, halogen, hydroxy and/or trifluoromethyl, and phenyl- or naphthyl-C₁₋₄alkoxy which is unsubstituted or mono-, di- or trisubstituted by C₁₋₄alkoxy, C₁₋₄alkyl, C₁₋₄alkylamino, di-C₁₋₄alkylamino, halogen, hydroxy and/or trifluoromethyl,
and the pharmaceutically acceptable salts thereof.

### Group J: Compounds of the general formula

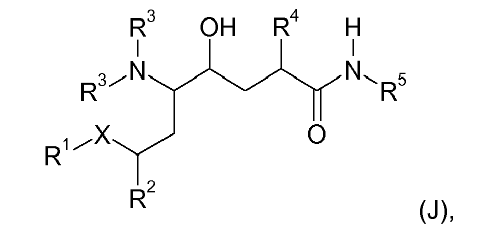

in which
- X: is -CH₂- ;
- R¹: is a mono- to tetrasubstituted, mono- or bicyclic, unsaturated hetero- cyclic radical having 1 to 4 nitrogen atoms, where the substituents of the said radicals are selected independently of one another from the group consisting of acetamidinyl-C₁₋₆alkyl, 3-acetamidomethyl- pyrrolidinyl, acyl-C₁₋₆alkoxy-C₁₋₆alkyl, (N-acyl)-C₁₋₆alkoxy-C₁₋₆alkyl- amino, C₁₋₆alkanoyl, C₁₋₆alkanoyloxy, C₂₋₆alkenyl, C₂₋₆alkenyloxy, C₂₋₆alkenyloxy-C₁₋₆alkyl, C₁₋₆alkoxy, C₁₋₆alkoxy-C₁₋₆alkoxy, C₁₋₆alkoxy-C₁₋₆alkoxy-C₁₋₆alkyl, C₁₋₆alkoxy-C₁₋₆alkyl, (N-C₁₋₆alkoxy)- C₁₋₆alkylaminocarbonyl-C₁₋₆alkoxy, (N-C₁₋₆alkoxy)-C₁₋₆alkylamino- carbonyl-C₁₋₆alkyl, C₁₋₆alkoxy-C₁₋₆alkylcarbamoyl, C₁₋₆alkoxy- C₁₋₆alkylcarbonyl, C₁₋₆alkoxy-C₁₋₆alkylcarbonylamino, 1-C₁₋₆alkoxy- C₁₋₆alkylimidazol-2-yl, 2-C₁₋₆alkoxy-C₁₋₆alkyl-4-oxoimidazol-1-yl, 3-C₁₋₆alkoxy-C₁₋₆alkylpyrrolidinyl, 1-C₁₋₆alkoxy-C₁₋₆alkyltetrazol-5-yl, 5-C₁₋₆alkoxy-C₁₋₆alkyltetrazol-1-yl, C₁₋₆alkoxyaminocarbonyl- C₁₋₆alkoxy, C₁₋₆alkoxyaminocarbonyl-C₁₋₆alkyl, C₁₋₆alkoxycarbonyl, C₁₋₆alkoxycarbonyl-C₁₋₆alkoxy, C₁₋₆alkoxycarbonyl-C₁₋₆alkyl, C₁₋₆alkoxycarbonylamino, C₁₋₆alkoxycarbonylamino-C₁₋₆alkoxy, C₁₋₆alkoxycarbonylamino-C₁₋₆alkyl, C₁₋₆alkyl, (N-C₁₋₆alkyl)-C₁₋₆alkoxy- C₁₋₆alkylcarbamoyl, (N-C₁₋₆alkyl)-C₁₋₆alkoxy-C₁₋₆alkylcarbonylamino, (N-C₁₋₆alkyl)-C₁₋₆alkoxycarbonylamino, (N-C₁₋₆alkyl)-C₀₋₆alkyl- carbonylamino-C₁₋₆alkoxy, (N-C₁₋₆alkyl)-C₀₋₆alkylcarbonylamino- C₁₋₆alkyl, (N-C₁₋₆alkyl)-C₁₋₆alkylsulphonylamino-C₁₋₆alkoxy, (N-C₁₋₆alkyl)-C₁₋₆alkylsulphonylamino-C₁₋₆alkyl, C₁₋₆alkylamidinyl, C₁₋₆alkylamino, di-C₁₋₆alkylamino, C₁₋₆alkylamino-C₂₋₆alkoxy, di-C₁₋₆alkylamino-C₂₋₆alkoxy, C₁₋₆alkylamino-C₁₋₆alkyl, C₁₋₆alkyl- aminocarbonyl, C₁₋₆alkylaminocarbonyl-C₁₋₆alkoxy, di-C₁₋₆alkyl- aminocarbonyl-C₁₋₆alkoxy, C₁₋₆alkylaminocarbonyl-C₁₋₆alkoxy- C₁₋₆alkyl, C₁₋₆alkylaminocarbonyl-C₁₋₆alkyl, C₁₋₆alkylaminocarbonyl- amino-C₁₋₆alkoxy, C₁₋₆alkylaminocarbonylamino-C₁₋₆alkyl, di- C₁₋₆alkylaminocarbonyl-C₁₋₆alkyl, di-C₁₋₆alkylamino-C₁₋₆alkyl, C₁₋₆alkylcarbamoyl, di-C₁₋₆alkylcarbamoyl, C₀₋₆alkylcarbonylamino, C₀₋₆alkylcarbonylamino-C₁₋₆alkoxy, C₀₋₆alkylcarbonylamino-C₁₋₆alkyl, C₁₋₆alkylcarbonyloxy-C₁₋₆alkoxy, C₁₋₆alkylcarbonyloxy-C₁₋₆alkyl, C₁₋₆alkylenedioxy, C₁₋₆alkylsulphonyl, C₁₋₆alkylsulphonyl-C₁₋₆alkoxy, C₁₋₆alkylsulphonyl-C₁₋₆alkyl, C₁₋₆alkylsulphonylamino-C₁₋₆alkoxy, C₁₋₆alkylsulphonylamino-C₁₋₆alkyl, amino, amino-C₂₋₇alkoxy, amino- C₁₋₆alkyl, aryl-C₁₋₆alkanoyl, benzoyloxy-C₂₋₆alkoxy, carbamoyl, carbamoyl-C₁₋₆alkoxy, carbamoyl-C₁₋₆alkyl, carboxy, carboxy- C₁₋₆alkoxy, carboxy-C₁₋₆alkoxy-C₁₋₆alkyl, carboxy-C₁₋₆alkyl, cyano, cyano-C₁₋₆alkoxy, cyano-C₁₋₆alkyl, C₃₋₈cydoalkyl-C₁₋₆alkanoyl, C₃₋₈cycloalkyl-C₀₋₆alkoxy, C₃₋₈cycloalkyl-C₀₋₆alkyl, C₃₋₈cycloalkyl- carbonylamino, C₃₋₈cycloalkylcarbonylamino-C₁₋₆alkoxy, C₃₋₈cyclo- alkylcarbonylamino-C₁₋₆alkyl, 3,4-dihydroxypyrrolidinyl, O,N-dimethyl- hydroxylamino-C₁₋₆alkyl, 2,6-dimethylmorpholinyl, 3,5-dimethyl- morpholinyl, dioxanyl, dioxolanyl, dioxolanyl-C₁₋₆alkoxy, 4,4-dioxo- thiomorpholinyl, dithianyl, dithiolanyl, optionally C₁₋₆alkoxy-, C₁₋₆alkyl-, dihydroxy-C₁₋₆alkylaminocarbonyl- or halogen-substituted furyl, furyl-C₁₋₆alkoxy, furyl-C₁₋₆alkyl, pyridyl, pyridyl-C₁₋₆alkoxy, pyridyl-C₁₋₆alkyl, pyridylamino, pyridyloxy, pyridylthio, pyrimidinyl, pyrimidinyl-C₁₋₆alkoxy, pyrimidinyl-C₁₋₆alkyl, pyrimidinylamino, pyrimidinyloxy, pyrimidinylthio, thienyl, thienyl-C₁₋₆alkoxy or thienyl- C₁₋₆alkyl, halogen, heterocyclyl-C₁₋₆alkanoyl, hydroxy, hydroxy- C₂₋₆alkoxy, hydroxy-C₂₋₆alkoxy-C₁₋₆alkoxy, hydroxy-C₂₋₆alkoxy- C₁₋₆alkyl, hydroxy-C₁₋₆alkyl, (N-hydroxy)-C₁₋₆alkylaminocarbonyl- C₁₋₆alkoxy, (N-hydroxy)-C₁₋₆alkylaminocarbonyl-C₁₋₆alkyl, (N-hydroxy)aminocarbonyl-C₁₋₆alkoxy, (N-hydroxy)aminocarbonyl- C₁₋₆alkyl, hydroxybenzyloxy, 2-hydroxymethylpyrrolidinyl, 4-hydroxypiperidinyl, 3-hydroxypyrrolidinyl, imidazolyl-C₁₋₆alkoxy, imidazolyl-C₁₋₆alkyl, methoxybenzyloxy, methylenedioxybenzyloxy, 2-methylimidazolyl-C₁₋₆alkoxy, 2-methylimidazolyl-C₁₋₆alkyl, 3-methyl- [1,2,4]-oxadiazol-5-yl-C₁₋₆alkoxy, 5-methyl-[1,2,4]-oxadiazol-3-yl- C₁₋₆alkoxy, 3-methyl-[1,2,4]-oxadiazol-5-yl-C₁₋₆alkyl, 5-methyl-[1,2,4]- oxadiazol-3-yl-C₁₋₆alkyl, O-methyloximyl-C₁₋₆alkyl, 4-methyl- piperazinyl, N-methylpiperazino-C₁₋₆alkoxy, N-methylpiperazino- C₁₋₆alkoxy-C₁₋₆alkyl, N-methylpiperazino-C₁₋₆alkyl, 5-methyltetrazol-1- yl-C₁₋₆alkoxy, 5-methyltetrazol-1-yl-C₁₋₆alkyl, morpholinyl, morpholino-C₁₋₆alkoxy, morpholino-C₁₋₆alkoxy-C₁₋₆alkyl, morpholino- C₁₋₆alkyl, nitro, [1,2,4]-oxadiazol-5-yl-C₁₋₆alkoxy, [1,2,4]-oxadiazol-5- yl-C₁₋₆alkyl, oxazol-4-yl-C₁₋₆alkoxy, oxazol-4-yl-C₁₋₆alkyl, oxide, oxo, 2-oxoimidazolidinyl, 2-oxo-[1,3]oxazinyl, 2-oxooxazolidinyl, 2-oxo- oxazolidinyl-C₁₋₆alkoxy, 2-oxooxazolidinyl-C₁₋₆alkyl, 4-oxopiperidinyl, 2-oxopyrrolidinyl, 2-oxopyrrolidinyl-C₁₋₆alkoxy, 2-oxopyrrolidinyl- C₁₋₆alkyl, 2-oxotetrahydropyrimidinyl, 4-oxothiomorpholinyl, optionally C₁₋₆alkoxy-, C₁₋₆alkoxycarbonyl-, C₁₋₆alkyl-, C₁₋₆alkylamino-, di- C₁₋₆alkylamino-, halogen-, hydroxy-, hydroxy-C₁₋₆alkyl-, or trifluoro- methyl-substituted phenoxy, phenyl, phenyl-C₁₋₆alkoxy, phenyl- C₁₋₆alkyl or phenylthio, piperazinyl, piperazino-C₁₋₆alkoxy, piperazino- C₁₋₆alkoxy-C₁₋₆alkyl, piperazino-C₁₋₆alkyl, piperidinyl, piperidino- C₁₋₆alkoxy, piperidino-C₁₋₆alkoxy-C₁₋₆alkyl, polyhalogen-C₁₋₆alkoxy, polyhalogen-C₁₋₆alkyl, pyridylcarbamoyloxy-C₁₋₆alkoxy, pyridyl- carbonylamino-C₁₋₆alkyl, pyrrolidinyl, pyrrolyl, tetrazol-1-yl-C₁₋₆alkoxy, tetrazol-2-yl-C₁₋₆alkoxy, tetrazol-5-yl-C₁₋₆alkoxy, tetrazol-1-yl- C₁₋₆alkyl, tetrazol-2-yl-C₁₋₆alkyl, tetrazol-5-yl-C₁₋₆alkyl, thiazol-4-yl- C₁₋₆alkoxy, thiazol-4-yl-C₁₋₆alkyl, thiomorpholinyl, [1,2,4]-triazol-1-yl- C₁₋₆alkoxy, [1,2,4]-triazol-4-yl-C₁₋₆alkoxy, [1,2,4]-triazol-1-yl-C₁₋₆alkyl, [1,2,4]-triazol-4-yl-C₁₋₆alkyl and the radical -O-CH₂CH(OH)CH₂NRx, where NRx is a mono- or di-C₁₋₆alkylamino, N-methylpiperazino, morpholino, piperazino or piperidino radical, and where in the case where R¹ is a bicyclic heterocyclic ring system, at least the ring not directly bonded to X is substituted as indicated;
- R²: is C₁₋₆alkyl or C₃₋₆cycloalkyl;
- R³: is independently of one another H, C₁₋₆alkyl, C₁₋₆alkoxycarbonyl or C₁₋₆alkanoyl;
- R⁴: is C₂₋₆alkenyl, C₁₋₆alkyl, unsubstituted or substituted aryl-C₁₋₆alkyl or C₃₋₈cycloalkyl;
- R⁵: is -Lₘ-R⁶;
- L: is C₁₋₈alkylene which is optionally substituted by 1-4 halogen, or a linker:

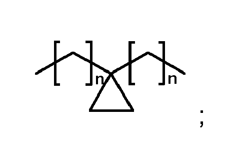

n = 0, 1 or 2;
m = 0 or 1;
   - R⁶: is a radical composed of 2 cyclic systems selected from bicyclo[x.y.z]alkyl, spiro[o.p]alkyl, mono- or bioxabicyclo[x.y.z]alkyl or mono- or bioxaspiro[o.p]alkyl, all of which may be substituted by 1-3 substituents selected from C₁₋₆alkyl, C₁₋₆alkoxy, cyano, halogen, C₁₋₆alkoxy-C₁₋₆alkyl, hydroxy-C₁₋₆alkyl or dialkylamino, or if m = 0: is also saturated C₃₋₈heterocyclyl which comprises 1-2 oxygen atoms, substituted by 1-3 substituents selected from C₁₋₆alkyl, C₁₋₆alkoxy, cyano, halogen, C₁₋₆alkoxy-C₁₋₆alkyl, hydroxy-C₁₋₆alkyl or dialkylamino, or if m = 1: is also saturated C₃₋₈heterocyclyl which comprises 1-2 oxygen atoms, optionally substituted by 1-3 substituents selected from C₁₋₆alkyl, C₁₋₆alkoxy, cyano, halogen, C₁₋₆-alkoxy-C₁₋₆alkyl, hydroxy- C₁₋₆alkyl or dialkylamino;

o = 2, 3, 4, 5 or 6
p = 2, 3, 4, 5 or 6
x = 1, 2, 3, 4 or 5;
y = 1, 2, 3, 4 or 5;
z = 0, 1, 2, 3, 4 or 5; where x ≥ y ≥ z;
and the pharmaceutically acceptable salts thereof.

The compounds of the above formulae may be present in the form of optically pure enantiomers, diastereomers, diastereomer mixtures, racemates, diastereomeric racemates, mixtures of diastereomeric racemates or as meso compounds. The invention encompasses all of theses forms. Diastereomer mixtures, diastereomeric racemates or mixtures of diastereomeric racemates may be separated by customary methods, for example by column chromatography, thin-layer chromatography, HPLC and the like.

The term "pharmaceutically acceptable salts" encompasses salts with inorganic or organic acids, such as hydrochloric acid, hydrobromic acid, nitric acid, sulfuric acid, phosphoric acid, citric acid, formic acid, fumaric acid, maleic acid, acetic acid, succinic acid, tartaric acid, methanesulfonic acid, p-toluenesulfonic acid and the like.

Salts of compounds having salt-forming groups are in particular acid addition salts, salts with bases, or, in the presence of a plurality of salt-forming groups, in some cases also mixed salts or internal salts.

The compounds of the above formulae having an acidic group, for example a carboxyl or sulfo group, may form salts, for example with suitable bases, such as non-toxic metal salts derived from metals of group Ia, Ib, IIa and IIb of the Periodic Table of the Elements, for example alkali metal, in particular lithium, sodium, or potassium, salts, alkaline earth metal salts, for example magnesium or calcium salts, and also zinc salts and ammonium salts, including those salts which are formed with organic amines, such as optionally hydroxy-substituted mono-, di- or trialkylamines, in particular mono-, di- or tri(lower alkyl)amines, or with quaternary ammonium bases, e.g. methyl-, ethyl-, diethyl- or triethylamine, mono-, bis- or tris(2-hydroxy(lower alkyl))amines, such as ethanol-, diethanol- or triethanolamine, tris(hydroxymethyl)methylamine or 2-hydroxy-tert-butylamine, N,N-di(lower alkyl)-N-(hydroxy(lower alkyl))amine, such as N,N-di-N-dimethyl-N-(2-hydroxyethyl)amine, or N-methyl-D-glucamine, or quaternary ammonium hydroxides such as tetrabutylammonium hydroxide. The compounds of the formula I having a basic group, for example an amino group, may form acid addition salts, for example with suitable inorganic acids, e.g. hydrohalic acid such as hydrochloric acid, hydrobromic acid, sulfuric acid with replacement of one or both protons, phosphoric acid with replacement of one or more protons, e.g. orthophosphoric acid or metaphosphoric acid, or pyrophosphoric acid with replacement of one or more protons, or with organic carboxylic, sulfonic or phosphonic acids or N-substituted sulfamic acids, e.g. acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid, isonicotinic acid, and also amino acids, for example the alpha-amino acids mentioned above, and also methanesulfonic acid, ethanesulfonic acid, 2-hydroxyethanesulfonic acid, ethane-1 ,2-disulfonic acid, benzenesulfonic acid, 4-methylbenzenesulfonic acid, naphthalene-2-sulfonic acid, 2- or 3-phosphoglycerate, glucose 6-phosphate, N-cyclohexylsulfamic acid (with formation of the cyclamates) or with other acidic organic compounds such as ascorbic acid. Compounds of formula (I) having acidic and basic groups may also form internal salts.

Salts obtained may be converted to other salts in a manner known per se, acid addition salts, for example, by treating with a suitable metal salt such as a sodium, barium or silver salt, of another acid in a suitable solvent in which an inorganic salt which forms is insoluble and thus separates out of the reaction equilibrium, and base salts by release of the free acid and salt reformation.

The compounds of the above formulae, including their salts, may also be obtained in the form of hydrates or solvates, incorporating a solvent used in the crystallization process.

The above-specified compound groups are not to be regarded as closed, but rather it is possible in a sensible manner, for example in order to replace general by more specific definitions, to exchange parts of these compound groups with one another or for the definitions given or to omit them.

The compounds of the above formulae may also be prepared in optically pure form. The separation into antipodes may be effected by methods known per se, either preferably at a synthetically early stage by salt formation with an optically active acid, for example (+)- or (-)-mandelic acid, and separation of the diastereomeric salts by fractional crystallization, or preferably at a rather late stage by derivatization with a chiral auxiliary building block, for example (+)- or (-)-camphanoyl chloride, and separation of the diastereomeric products by chromatography and/or crystallization and subsequent cleavage of the bond to the chiral auxiliary. To determine the absolute configuration of the compound present, the pure diastereomeric salts and derivatives may be analysed with common spectroscopic methods, of which X-ray spectroscopy on single crystals constitutes a particularly suitable method.

The compounds of the above formulae also include those compounds in which one or more atoms are replaced by their stable, non-radioactive isotopes; for example, a hydrogen atom by deuterium.

Prodrug derivatives of the compounds described in the present context are derivatives thereof which, on *in vivo* application, release the original compound by a chemical or physiological process. A prodrug may be converted to the original compound, for example, when a physiological pH is attained or by enzymatic conversion. Prodrug derivatives may, for example, be esters of freely available carboxylic acids, S- and O-acyl derivatives of thiols, alcohols or phenols, where the acyl group is as defined in the present context. Preference is given to pharmaceutically acceptable ester derivatives which are converted by solvolysis in physiological medium to the original carboxylic acid, for example lower alkyl esters, cycloalkyl esters, lower alkenyl esters, benzyl esters, mono- or disubstituted lower alkyl esters such as lower ω-(amino, mono- or dialkylamino, carboxyl, lower alkoxycarbonyl)alkyl esters or such as lower α-(alkanoyloxy, alkoxycarbonyl or dialkylaminocarbonyl)alkyl esters; as such, pivaloyloxymethyl esters and similar esters are utilized in a conventional manner.

Owing to the close relationship between a free compound, a prodrug derivative and a salt compound, a certain compound in this invention also encompasses its prodrug derivative and salt form, where this is possible and appropriate.

The compounds of the above formulae, or the pharmaceutically acceptable salts thereof, are useful as medicines for the prevention, retardation of progression or treatment of psoriasis, for example in the form of pharmaceutical compositions. The pharmaceutical compositions may be administered enterally, such as orally, for example in the form of tablets, film-coated tablets, sugar-coated tablets, hard and soft gelatin capsules, solutions, emulsions or suspensions, rectally, for example in the form of suppositories, topical on the skin such as dermally, for example in the form of ointments, crèmes, lotions, sprays, ear drops or patches. The administration may also be parenteral, such as intramuscular or intravenous, for example in the form of injection solutions.

To prepare tablets, film-coated tablets, sugar-coated tablets and hard gelatin capsules, the compounds of the formula (I) or formula (Ia) or the pharmaceutically acceptable salts thereof may be processed with pharmaceutically inert, inorganic or organic excipients. Such excipients used, for example for tablets, film-coated tablets and hard gelatin capsules, may be lactose, corn starch, or derivatives thereof, talc or stearic acid or salts thereof.
Suitable excipients for soft gelatin capsules are, for example, vegetable oils, waxes, fats and semisolid and liquid polyols.
Suitable excipients for preparing solutions and syrups are, for example, water, polyols, sucrose, invert sugar and glucose.
Suitable excipients for injection solutions are, for example, water, alcohols, polyols, glycerol, vegetable oils, bile acids and lecithin.
Suitable excipients for suppositories are, for example, natural or hardened oils, waxes, fats and semisolid or liquid polyols.
The pharmaceutical compositions may additionally also comprise preservatives, solubilizers, viscosity-increasing substances, stabilizers, wetting agents, emulsifiers, sweeteners, colorants, flavourings, salts for altering the osmotic pressure, buffers, coatings or antioxidants. The compositions may also comprise other therapeutically valuable substances.

The dose may vary within wide limits and has of course to be adapted to the individual circumstances in each individual case. In general, for oral administration, a daily dose of about 3 mg to about 3 g, preferably about 10 mg to about 1 g, for example about 300 mg, per adult (70 kg), divided into preferably 1-3 individual doses which may, for example, be of equal size, may be appropriate, although the upper limit specified may also be exceeded if this should be found to be appropriate; typically, children receive a lower dose according to their age and body weight.

The compounds which follow illustrate the compounds useful in the present invention. Unless stated otherwise, the absolute stereochemistry of the 3-hydroxy(or alkoxy)-4-phenyl-5-alkoxypiperidine unit is (3S,4S,5R) (or 3S,4R,5R, depending on the 3-alkoxy group).

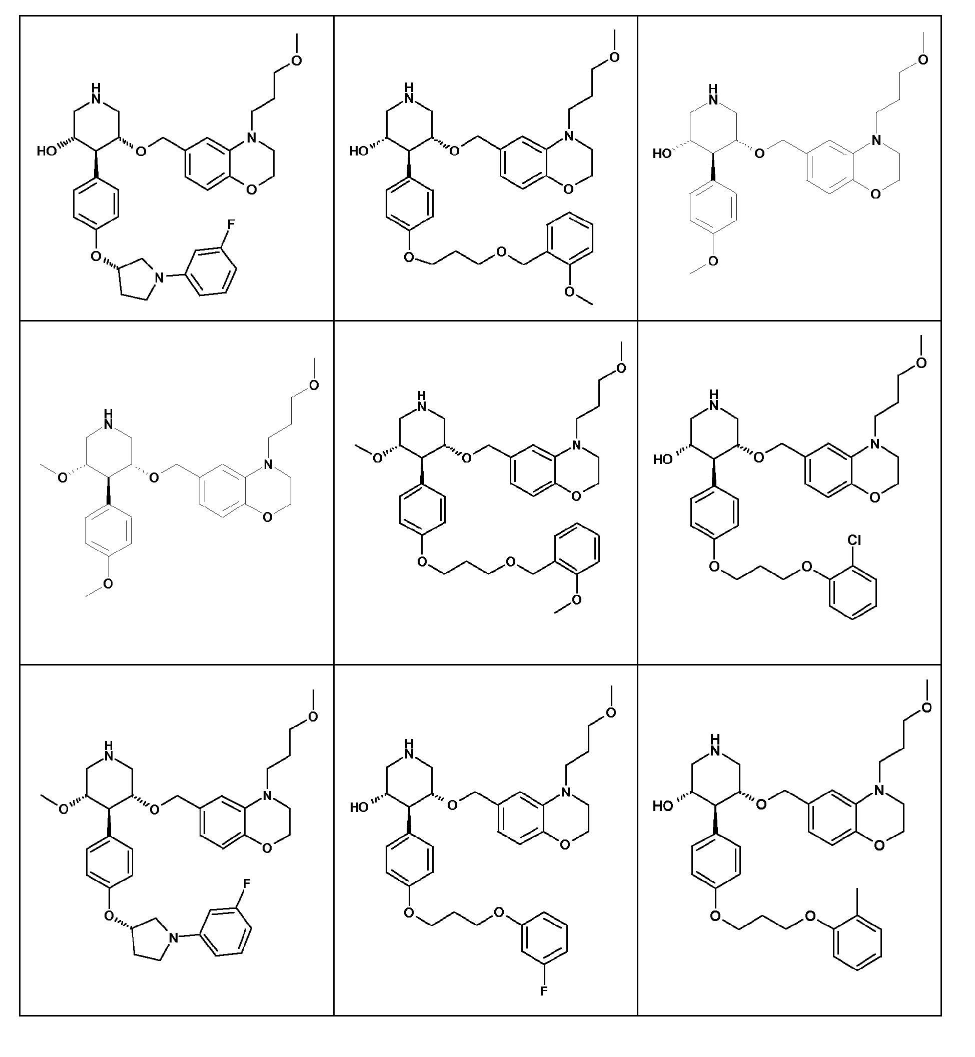

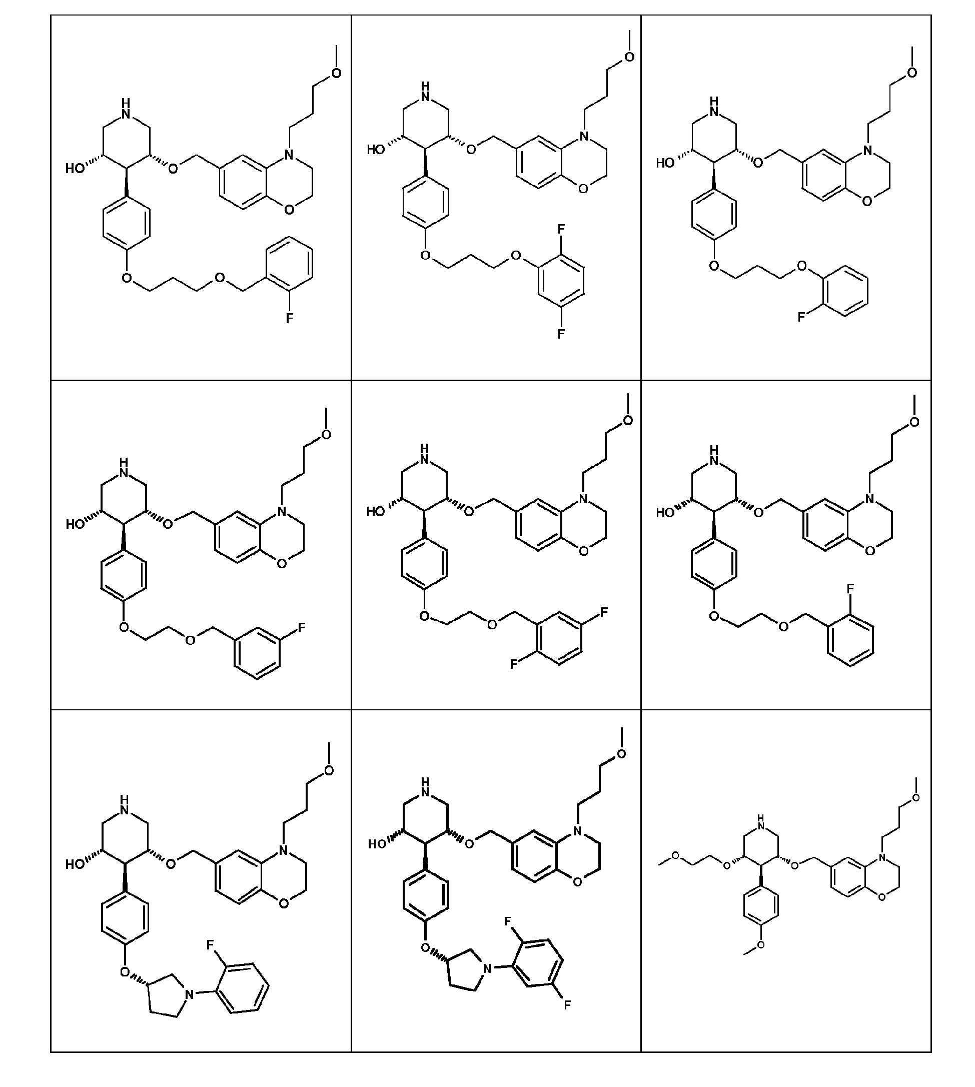

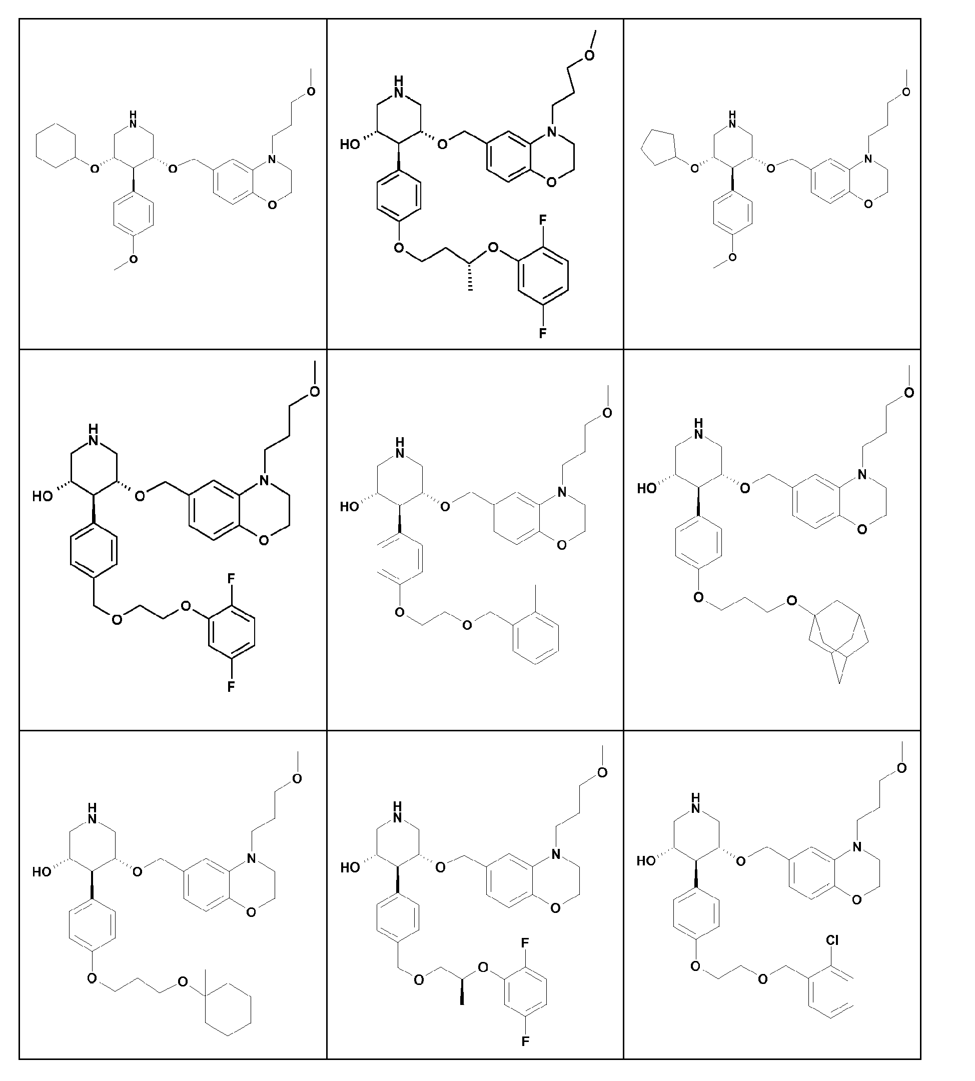

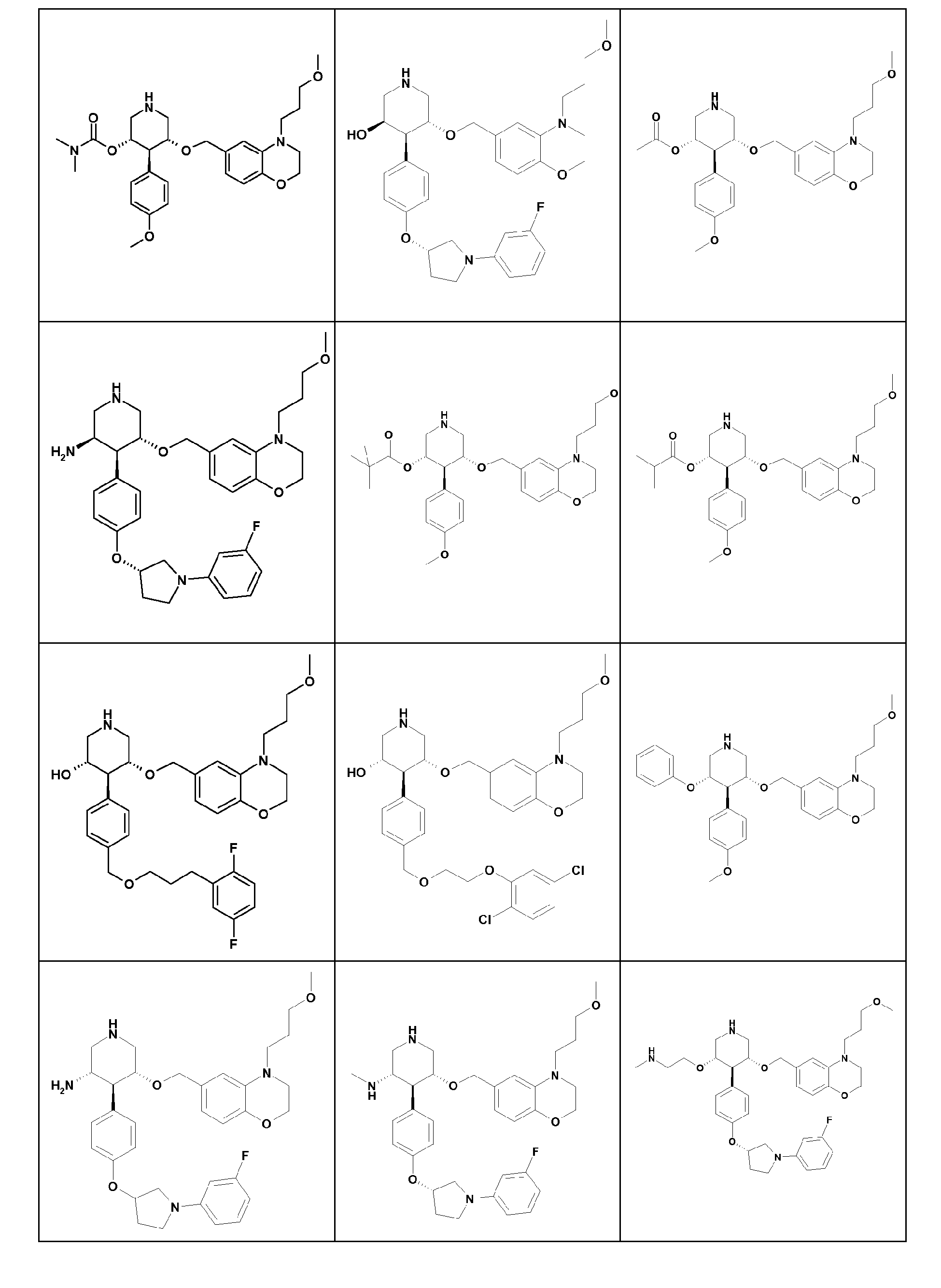

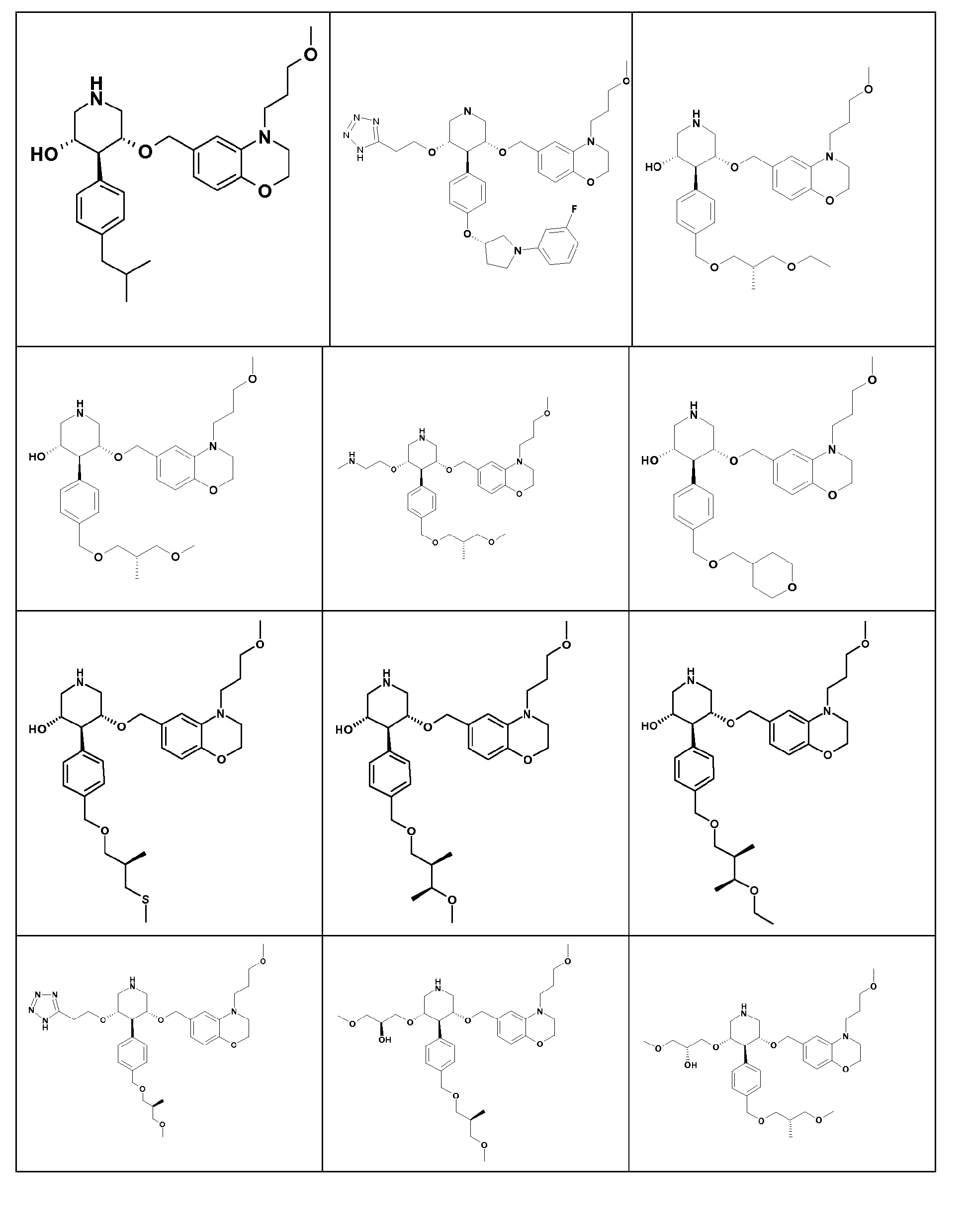

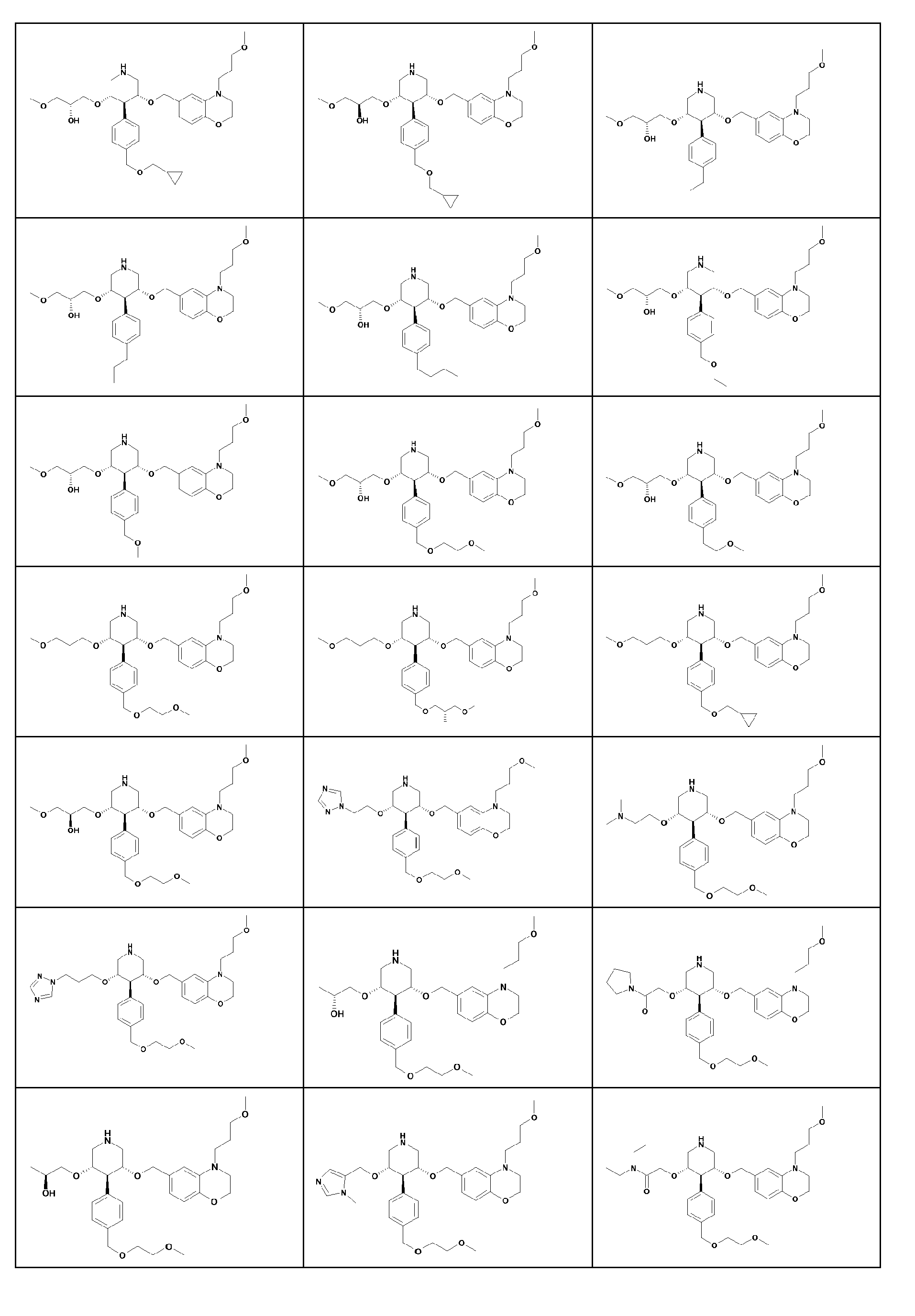

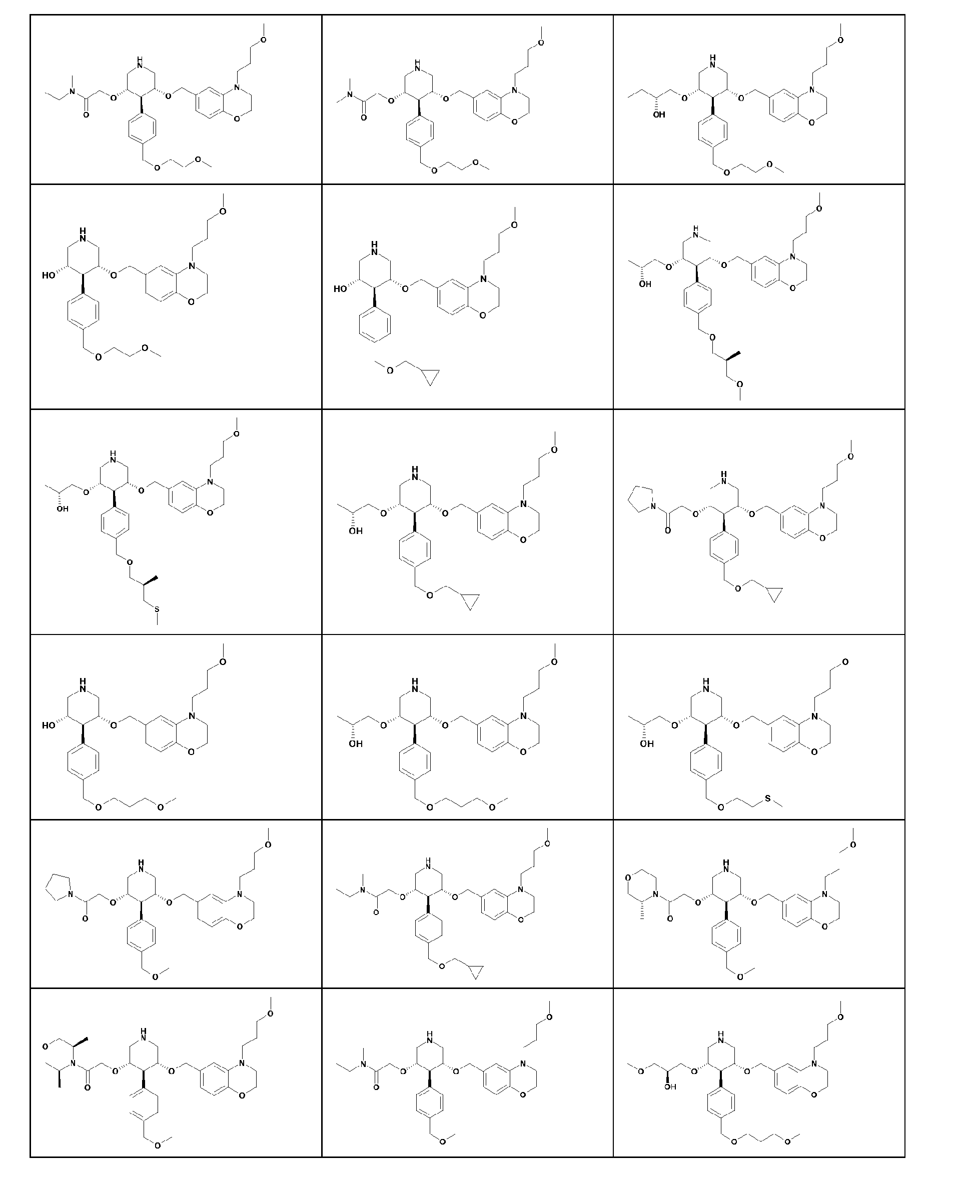

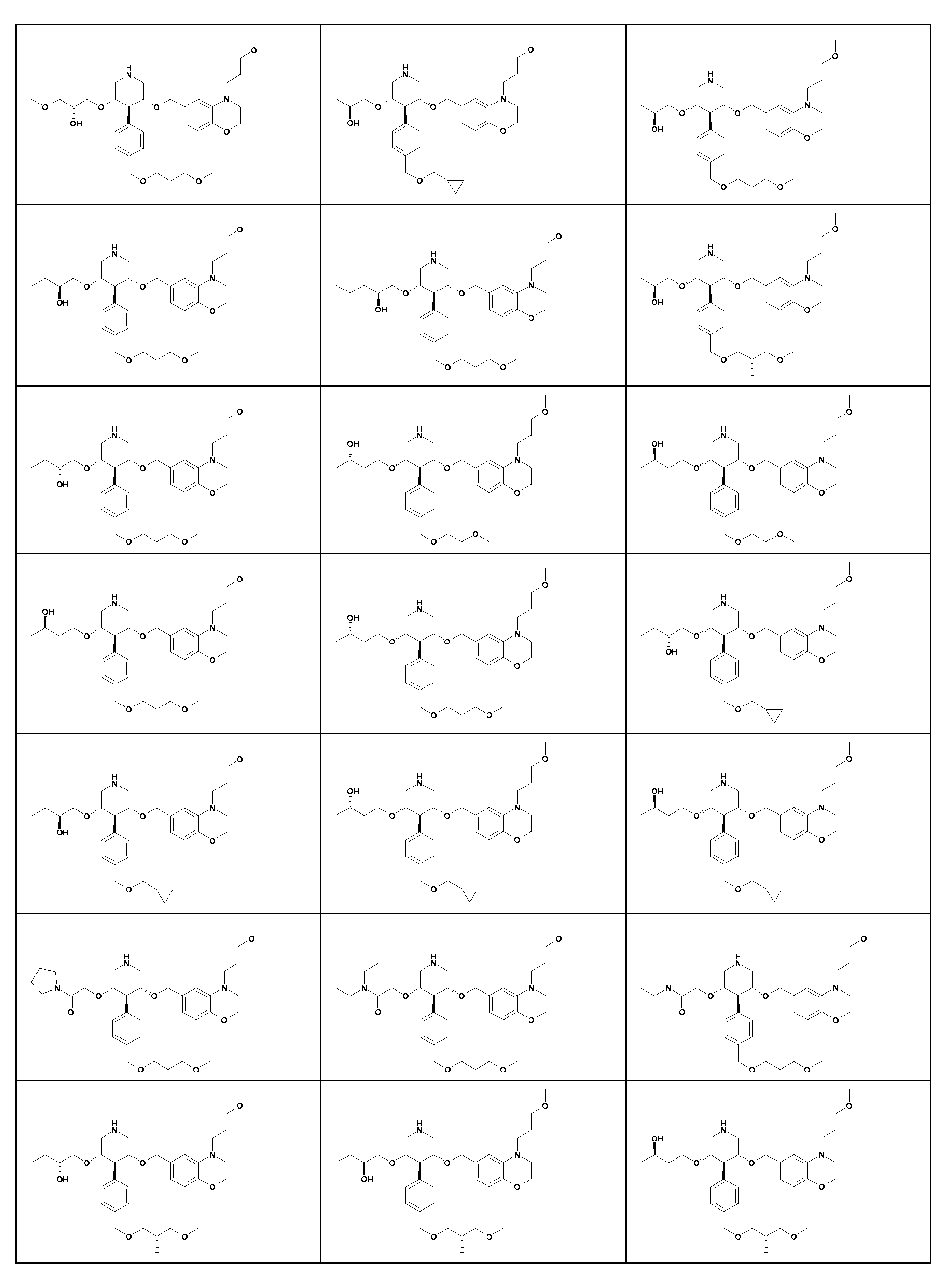

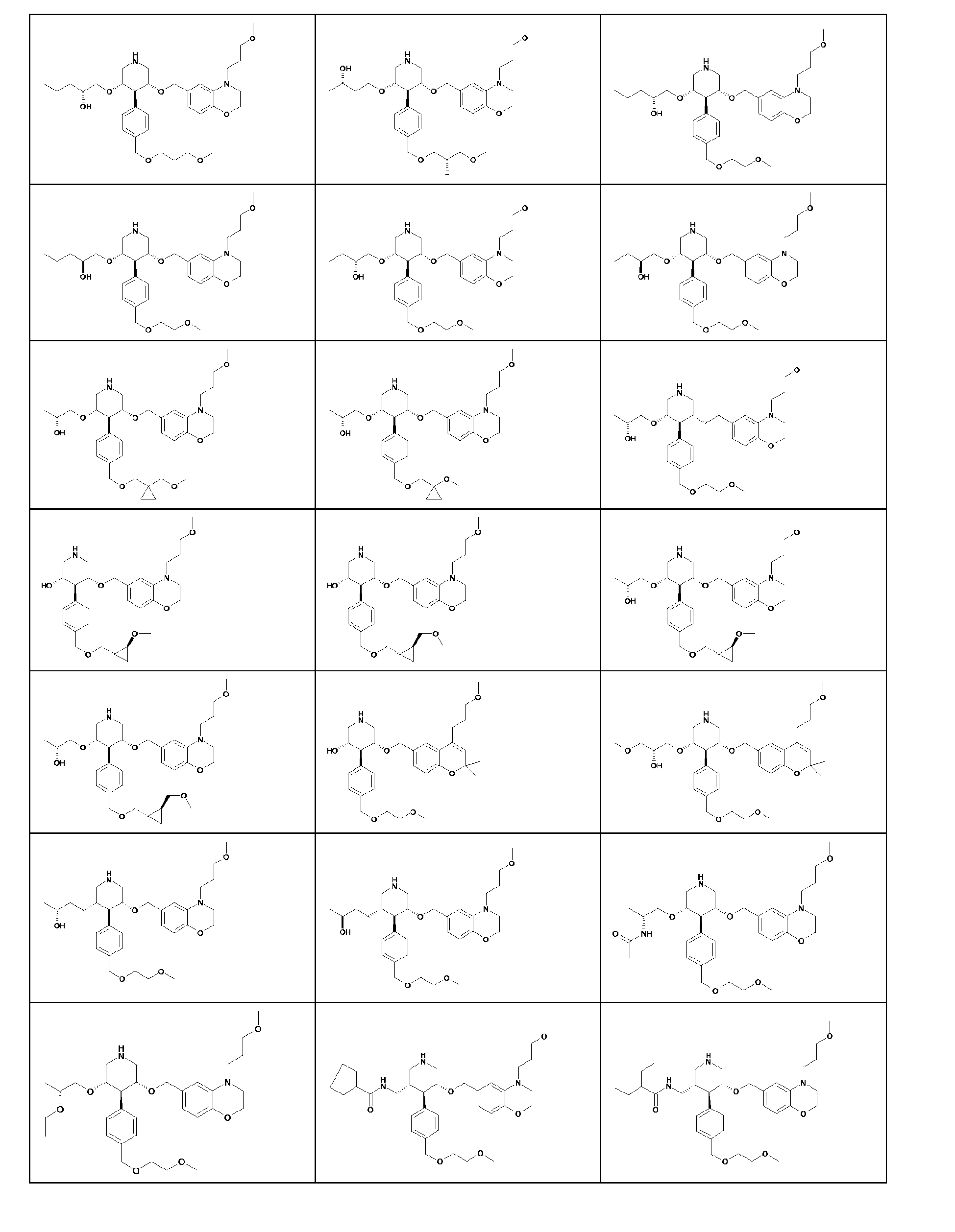

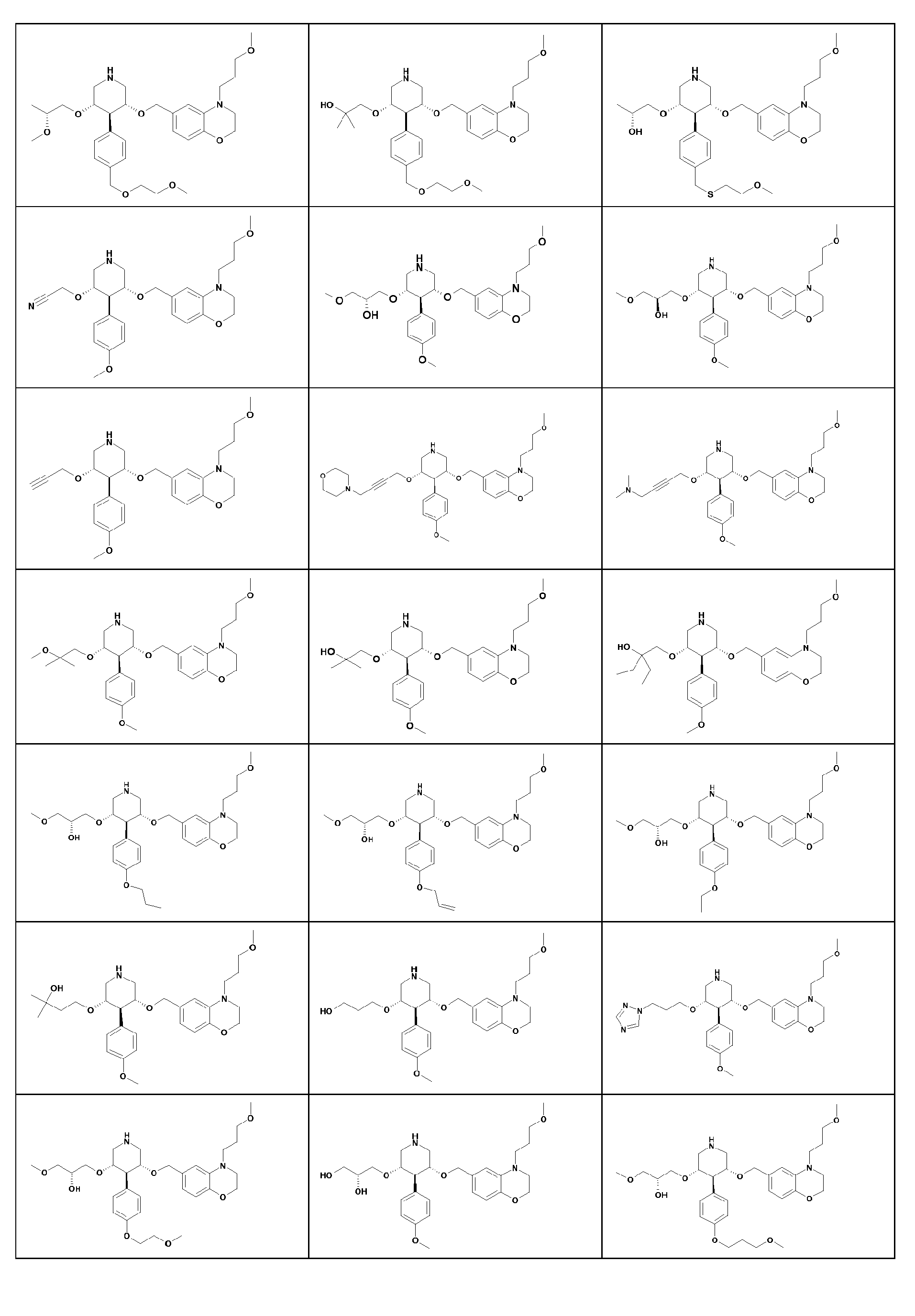

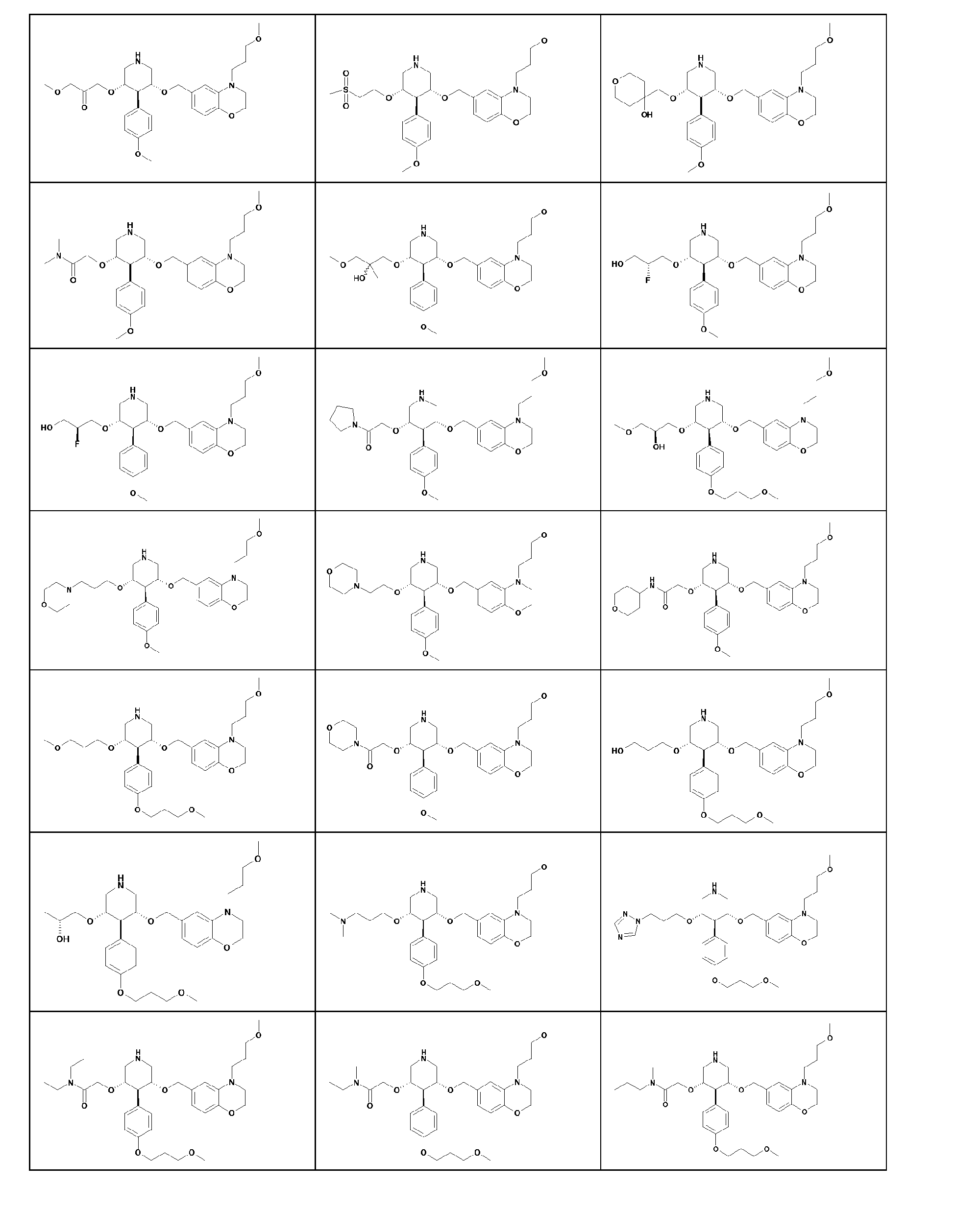

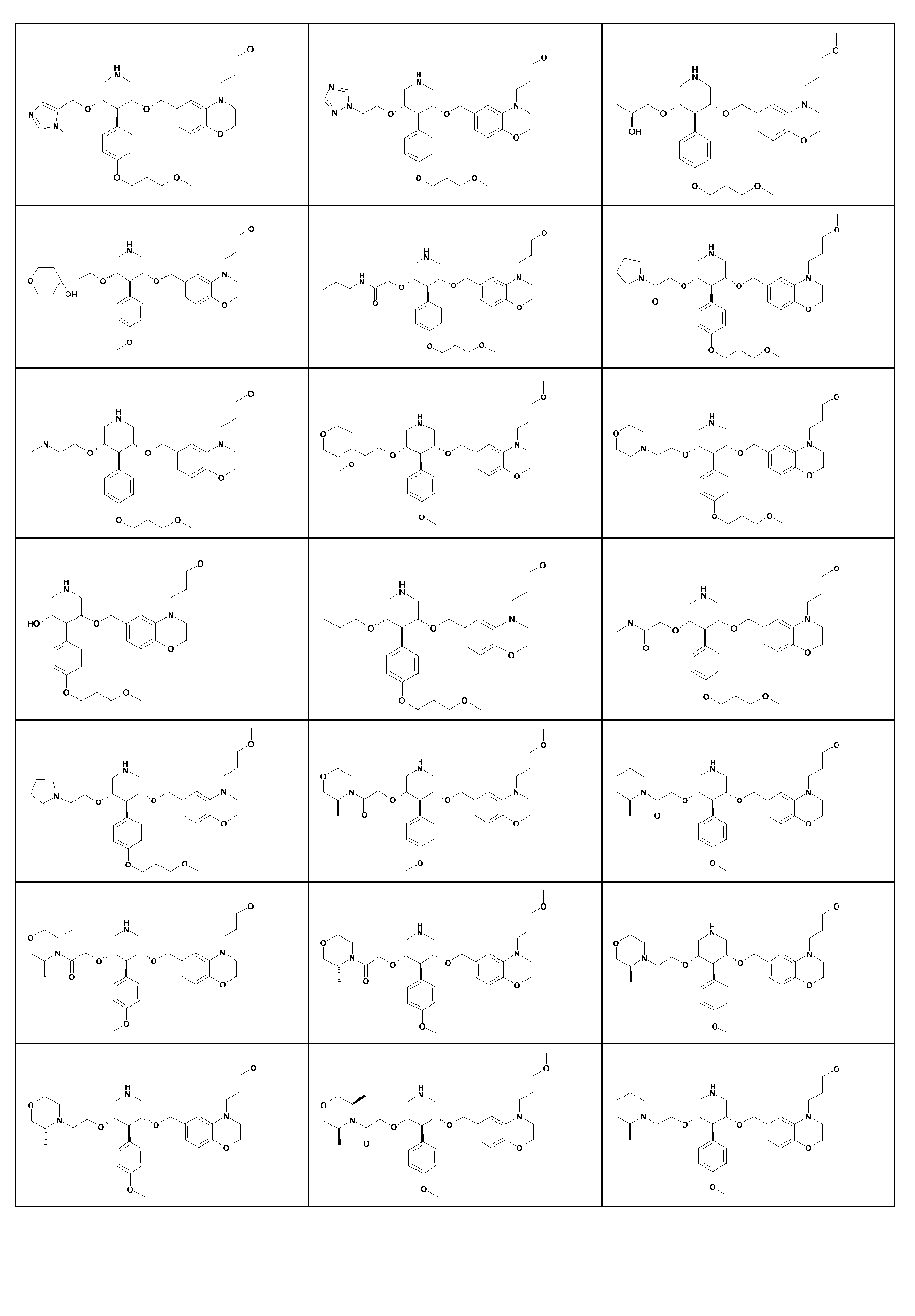

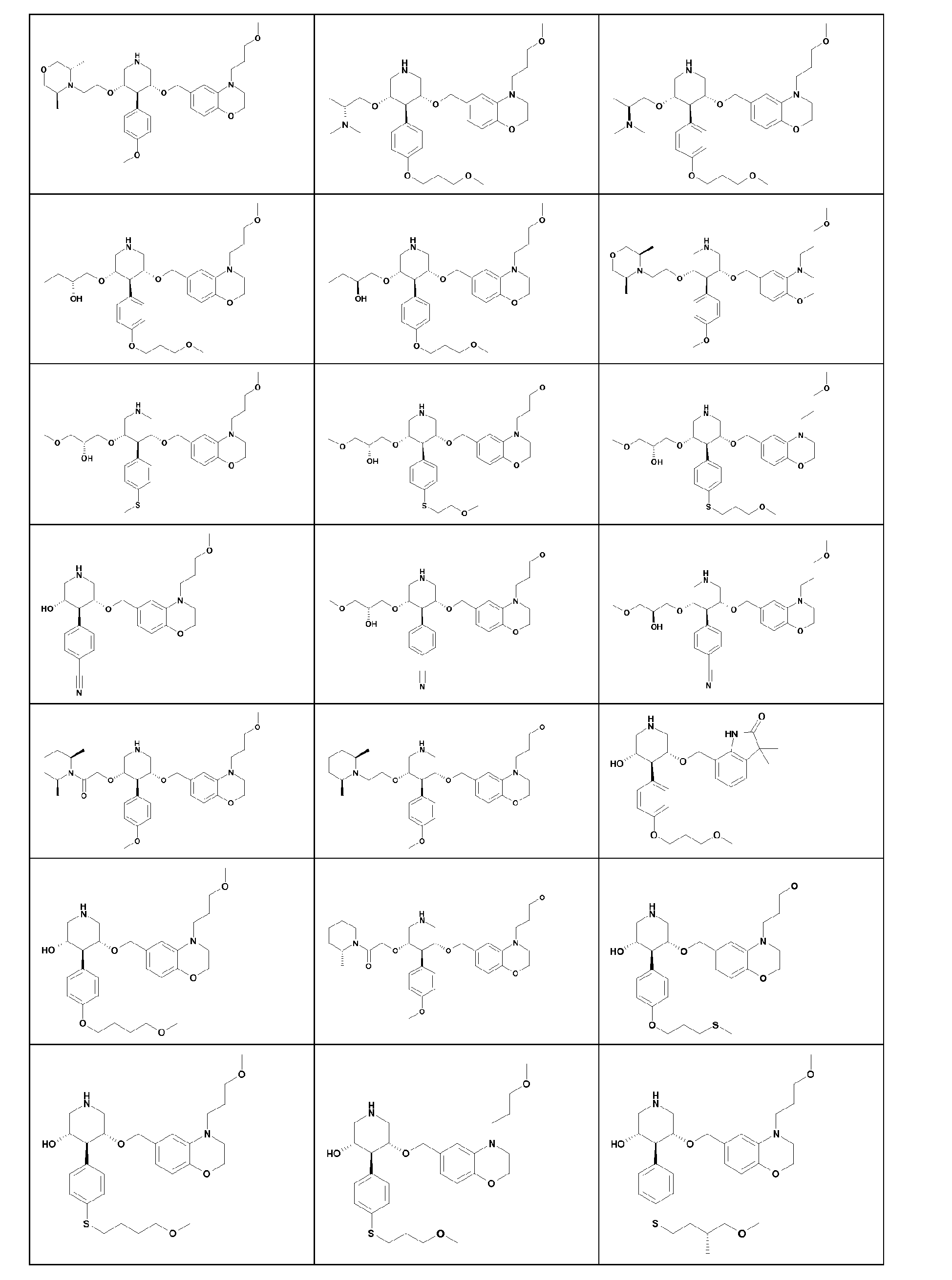

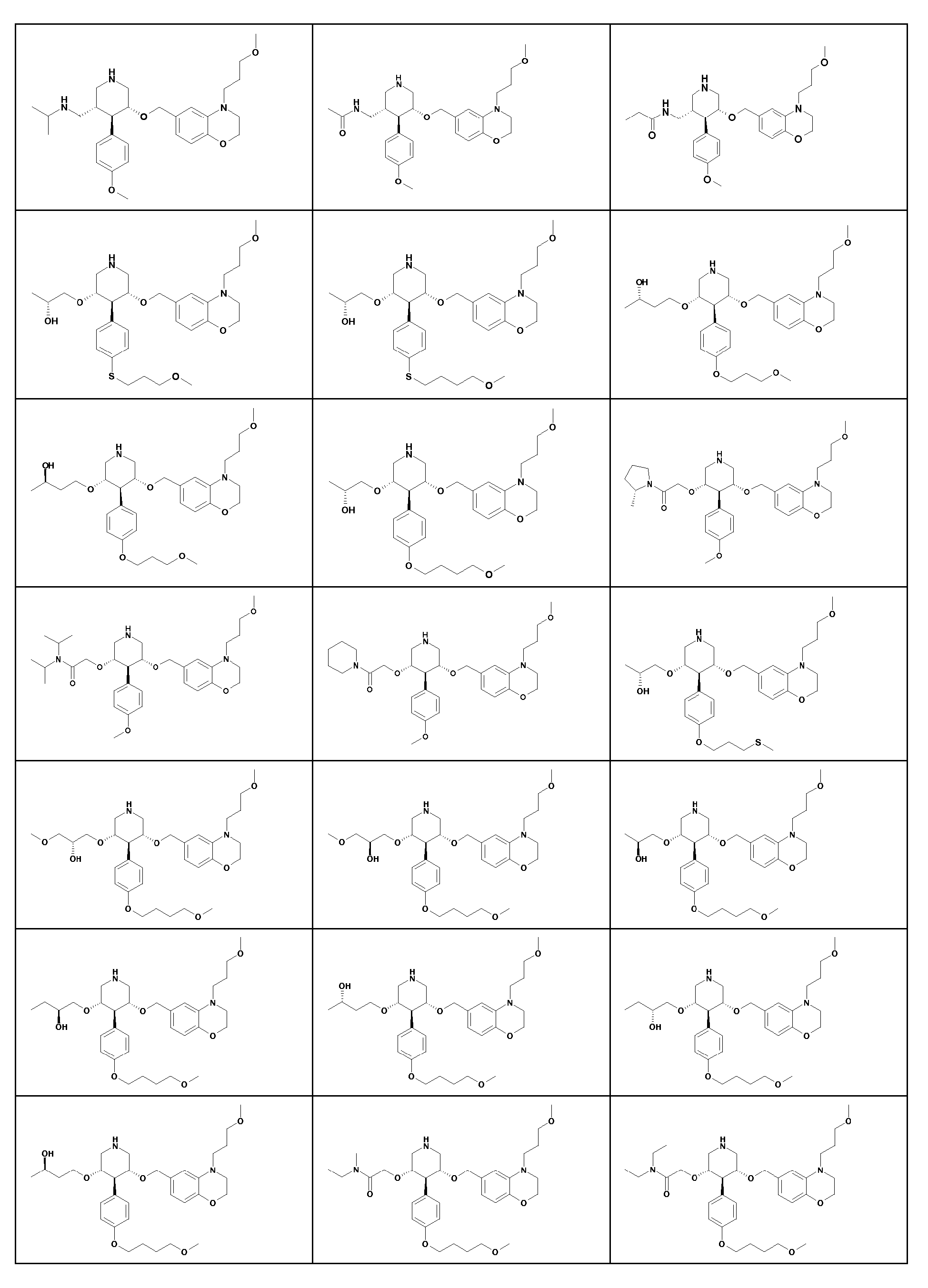

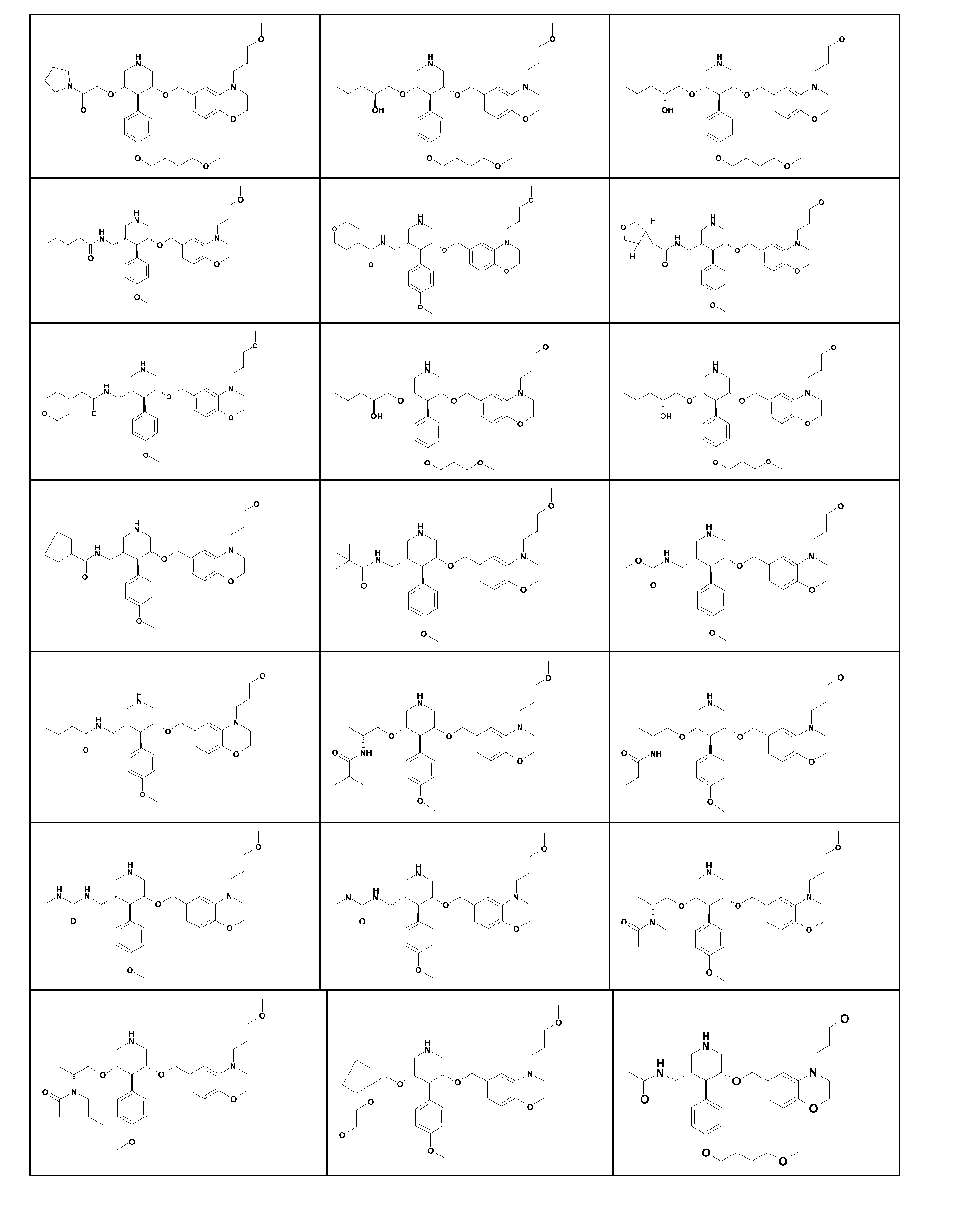

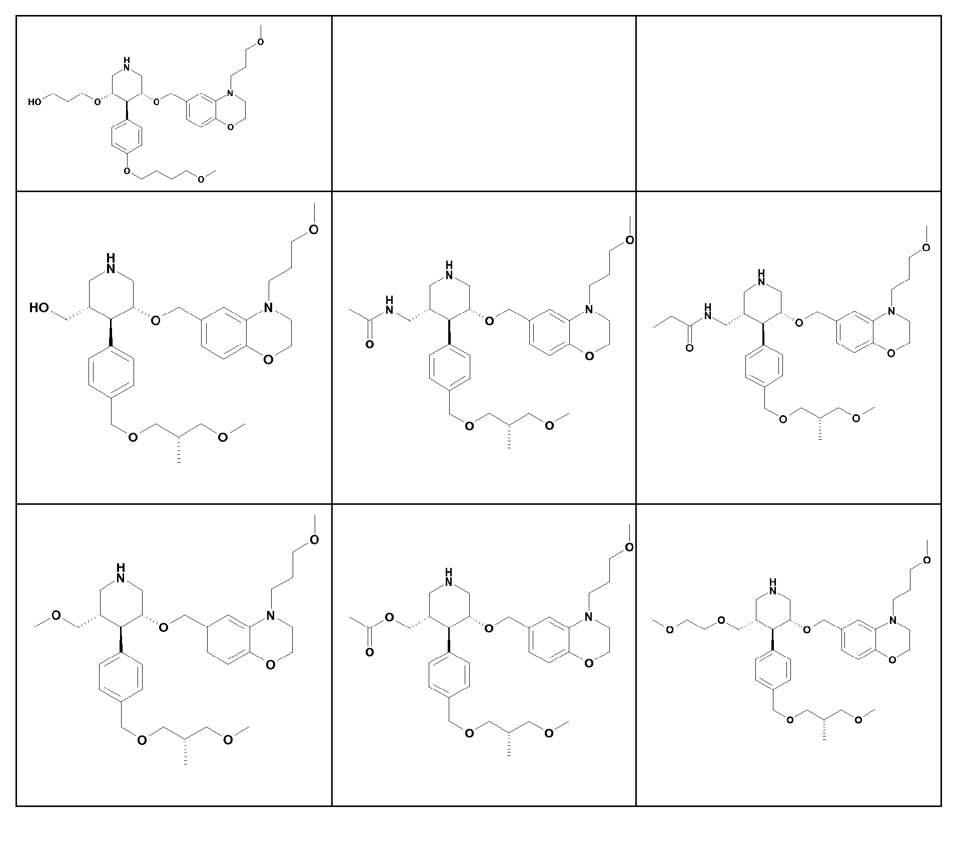

The following tests describe the determination of the antipsoriatic effect of the compounds according to the present invention. The tests are based on A. Pol et al. "A simple technique for high-throughput screening of drugs that modulate normal and psoriasis-like differentiation in cultured human keratinocytes"in Skin Pharmacol. Appl. Skin Physiol. 2002;15:252-261.

### SKALP ELISA of Culture Supernatants

A sandwich-type ELISA is used to determine SKALP levels in supernatants of cultured keratinocytes. Briefly, 96-well flat-bottom microtitre plates is coated overnight with the mouse monoclonal antibody Trab-2F. Plates are blocked with 1% bovine serum albumin and probed with the test sample for 2 h at 37 °C. Subsequently, the biotinylated monoclonal antibody Trab-20 is added, and the plates are incubated for

1 h at 37°C. Finally, the plates are incubated with avidin-biotin complex which is followed by a 30-min incubation at room temperature. Between all steps, the plates are washed with PBS/0.05% Tween-20.
o-Phenylene diamine dihydrochloride is added as chromogenic substrate for a 15-min incubation at room temperature. The enzyme reaction is stopped by addition of 4 M H₂SO₄. Absorbance is measured at 492 nm. A SKALP calibration curve (0.5-12.5 ng/ml) is prepared from a stock of full-length SKALP obtained by purification from cell culture supernatants. Concentrations of SKALP are read from this curve and expressed as nanograms secreted SKALP per millilitre culture medium.

### CK10 ELISA of Fixed Keratinocytes

A solid-phase ELISA will be used for semi-quantitative determination of CK10 levels in cultured keratinocytes. In brief, cells will be fixed with acetone:methanol (1:1) for 20 min. Plateswill be then blocked with 1% normal horse serum/1% bovine serum albumin/0.05% Tween-20 in PBS for 30 min at 37 °C. Subsequently, an anti-CK-10 mouse monoclonal antibody will be added, and plates will be incubated for 2 h at 37°C. Then biotinylated antimouse IgGwill be added, and plates will be incubated for 1.5 h at 37 °C. Subsequently, plates will be incubated with avidin-biotin complex during 1 h at 37°C. Plates will be washed between all steps with PBS/0.05% Tween-20. Finally, o-phenylene diamine dihydrochloride will be added as chromogenic substrate for a 15-min incubation at room temperature. Addition of 4 M H2S04 to stop the enzyme reaction will be omitted to allow subsequent cell number measurements as described below.
Absorbance was measured at 492 nm. Absorbance of cultures stained without primary antiserum will be used as a blank.

### Cell Number Measurements Using PicoGreen dsDNA Quantitation Reagent

Agents to be tested for their potential effect on keratinocyte differentiation are added to postconfluent keratinocyte cell cultures with low proliferative activity. However, in order to study possible drug effects on cell number during the additional 72 h of culture, cell number quantitation are performed using PicoGreen dsDNA Quantitation Reagent. Directly following CK10 ELISA, cells are washed with PBS, and 100 µl/well of a freshly prepared 2.5 mg/ml trypsin (Sigma) solution in PBS is added. Plates are incubated for 30 min at 37°C. Next, without removal of the trypsin solution, an additional 100 µl/well of a 1:200 dilution of PicoGreen in PBS is added. Subsequently, sample fluorescence is measured using a fluorescence microplate reader (excitation/emission 485/520 nm).

In the above tests:
a) the compounds of the present invention suppress the expression of the psorias-associated SKALP marker (at least not more than 50% SKALP compared to the untreated control at a concentration of 10⁻⁵ M);
b) within the concentration range tested (10⁻⁷ M to 10⁻⁵ M), the compounds of the present invention show a limited (less than 20% lower relative CK10 level compared to the untreated control) inhibition of CK10 expression;
c) within the concentration range tested (10⁻⁷ M to 10⁻⁵ M), the compounds of the present invention show a limited (less than 10% reduction of cell numbers compared to the untreated control) cytotoxic effect.

## Claims

1. A renin inhibitor for the prevention, for the retardation of progression or for the treatment of psoriasis.

2. A renin inhibitor according to Claim 1 selected from substituted piperidines and delta-amino-gamma-hydroxy-omega-(hetero)aryl alkanoic acid amide derivatives.

3. A renin inhibitor according to Claim 1 or 2 being a compound of the general formula 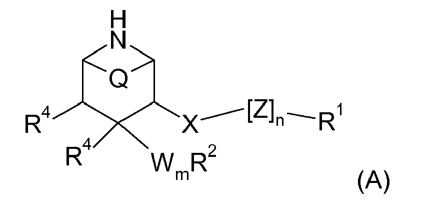 wherein R¹ is aryl or heterocyclyl;
R² is phenyl, naphthyl, acenaphthyl, cyclohexyl, pyridyl, pyrimidinyl, pyrazinyl, oxopyridinyl, diazinyl, triazolyl, thienyl, oxazolyl, oxadiazolyl, thiazolyl, pyrrolyl or furyl, which groups can be substituted by 1-3 halogen, hydroxy, cyano, trifluoromethyl, lower-alkyl, halo-lower-alkyl, hydroxy-lower-alkyl, lower-alkoxy-lower-alkyl, cyano-lower-alkyl, carboxy-lower-alkyl, lower-alkanoyloxy-lower-alkyl, lower-alkoxycarbonyloxy-lower-alkyl, lower-alkoxycarbonyl, or lower-alkoxy groups, or by lower-alkylenedioxy, and/or by a group L¹-T¹-L²-T²-L³-T³-L⁴-T⁴-L⁵-U;
L¹, L², L³, L⁴ and L⁵ independently of one another are a bond, C₁₋₈-alkylene, C₂₋₈-alkenylene or C₂₋₈-alkynylene or are absent;
T¹, T², T³ and T⁴ independently of one another are
(a) a bond or are absent or are one of the groups
(b) -CH(OH)-
(c) -CH(OR⁶)₋
(d) -CH(NR⁵R⁶)-
(e) -CO-
(f) -CR⁷R⁸-
(g) -O- or -NR⁶-
(h) -S(O)₀₋₂-
(i) -SO₂NR⁶-
(j) -NR⁶SO₂-
(k) -CONR⁶₋
(l) -NR⁶CO-
(m) -O-CO-
(n) -CO-O-
(o) -O-CO-O-
(p) -O-CO-NR⁶-
(q) -NR⁶ -CO-NR⁶-
(r) -NR⁶-CO-O-
and the bonds emanating from (b), (d), (e) and (g)-(r) join to a C atom of the adjacent group and this C atom is saturated when the bond emanates from a hetero atom, and not more than two groups (b)-(f), three groups (g)-(h) and one group (i)-(R) are present;
R³ is hydrogen, hydroxy, lower-alkoxy or lower-alkenyloxy; and
R⁴ is hydrogen, lower-alkyl, lower-alkenyl, lower-alkoxy, hydroxy-lower-alkyl, lower-alkoxy-lower-alkyl, benzyl, oxo or a group R^{4a} -Z¹ -X¹ - in which R^{4a} is
(a) H-
(b) lower-alkyl-
(c) lower-alkenyl-
(d) hydroxy-lower-alkyl-
(e) polyhydroxy-lower-alkyl-
(f) lower-alkyl-O-lower-alkyl-
(g) aryl-
(h) heterocyclyl-
(i) arylalkyl-
(j) heterocyclylalkyl-
(k) aryloxyalkyl-
(l) heterocyclyloxylalkyl-
(m) (R⁵R⁶)-N-(CH₂)₁₋₃-
(n) (R⁵R⁶)-N-
(o) lower-alkyl-S(O)₀₋₂-
(p) aryl-S(O)₀₋₂-
(q) heterocyclyl-S(O)₀₋₂-
(r) HO-SO₃- or salt thereof
(s) H₂N-C(NH)-NH-
(t) NC-,
and the bonds emanating from (n)-(t) join to a C atom of the adjacent group and this C atom is saturated when the bond emanates from a hetero atom;
Z¹ is
(a) a bond, is absent or is one of the groups
(b) lower-alkylene-
(c) lower-alkenylene-
(d) -O-, -N(R¹¹)--, -S(O)₀₋₂-
(e) -CO-
(f) -O-CO-
(g) -O-CO-O-
(h) -O-CO-N(R¹¹)-,
(i) -N(R¹¹)-CO-O-
(j) -CO-N(R¹¹)-
(k) -N(R¹¹)-CO-
(l) -N(R¹¹)-CO-N(R¹¹)-
(m) -CH(OR⁹)₋,
and the bonds emanating from (d) and (f)-(m) join to a C atom of the adjacent group and this C atom is saturated when the bond emanates from a hetero atom;
X¹ is
(a) a bond, is absent or is one of the groups
(b) -O-
(c) -N(R¹¹)-,
(d) -S(O))₀₋₂-
(e) -(CH₂)₁₋₃-
or R³ and R⁴ together are a bond;
R⁵ and R⁶ are hydrogen, lower-alkyl, lower-alkenyl, aryl-lower-alkyl or acyl or together with the N atom to which they are attached are a 5- or 6-membered heterocyclic ring which can contain an additional N atom or an O or S atom or a SO or SO₂ group and the additional N atom can be optionally substituted by lower-alkyl;
R⁷ and R⁸ together with the C atom to which they are attached are a 3-7 membered ring which can contain one or two O or S atoms or SO or SO₂ groups;
R⁹ is hydrogen, lower-alkyl, acyl or arylalkyl;
R¹⁰ is carboxyalkyl, alkoxycarbonylalkyl, alkyl or hydrogen;
R¹¹ is hydrogen or lower-alkyl;
U is hydrogen, lower-alkyl, cycloalkyl, cyano, optionally substituted cycloalkyl, aryl or heterocyclyl;
Q is ethylene or is absent;
X is a bond, oxygen, sulphur or a group -CH-R¹¹-, -CHOR⁹-, -O-CO-, -CO- or - C=NOR¹⁰- with the bond emanating from an oxygen or sulphur atom joining to a saturated C atom of group Z or to R¹ ;
W is oxygen or sulphur;
Z is lower-alkylene, lower-alkenylene, hydroxy-lower-alkylidene, -0-, -S-, -O-Alk-, -S-Alk-, -Alk-O- or -Alk-S-, in which Alk is lower alkylene; provided that
a) X is -CH-R¹¹- and either R² contains a substituent L¹-T¹-L²-T²-L³-T³-L⁴-T⁴-L⁵-U or R⁴ is a substituent defined above other than hydrogen when Z is -0- or -S-;
b) X is -CH-R¹¹- when Z is -O-Alk- or -S-Alk-; and
c) Z is lower-alkenylene, -Alk-O- or -Alk-S- when X is a bond;
n is 1 or, when X is -O-CO-, 0 or 1; and
m is 0 or 1;
or a pharmaceutically acceptable salt thereof.

4. A renin inhibitor according to Claim 1 or 2 being a compound of the general formula 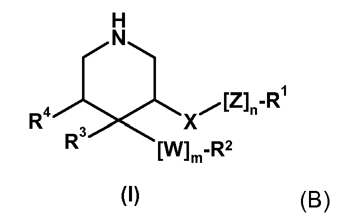 in which
(A) R¹ is aryl when R² is tetrazolyl or imidazolyl which may be substituted by 1-3 halogen, hydroxyl, cyano, trifluoromethyl, C₁₋₆-alkyl, halo-C₁₋₆-alkyl, hydroxy-C₁₋₆-alkyl, C₁₋₆-alkoxy-C₁₋₆-alkyl, cyano-C₁₋₆-alkyl, carboxy-C₁₋₆-alkyl, C₁₋₆-alkanoyloxy-C₁₋₆-alkyl, C₁₋₆-alkoxycarbonyloxy-C₁₋₆-alkyl, C₁₋₆-alkoxycarbonyl or C₁₋₆-alkoxy groups, or a C₁₋₆-alkylenedioxy group, and/or by an L1-T1-L2-T2-L3-T3-L4-T4-L5-U radical; or
(B) R¹ is aryl when X is -O-CH-R¹¹-CO-NR⁹-; or
(C) R¹ is aryl when Z is -alk-NR⁹- where alk denotes C₁₋₆-alkylene, and n is 1;
or
(D) R¹ is phenyl which is substituted by 1-4 acetamidinyl-C₁₋₆-alkoxy, acetamidinyl-C₁₋₆-alkyl, acyl-C₁₋₆-alkoxy-C₁₋₆-alkyl, (N-acyl)-C₁₋₆-alkoxy-C₁₋₆-alkylamino, C₁₋₆-alkoxy, C₁₋₆-alkoxy-C₁₋₆-alkoxy, C₁₋₆-alkoxy-C₁₋₆-alkoxy-C₁₋₆-alkyl, C₁₋₆-alkoxy-C₁₋₆-alkyl, (N-C₁₋₆-alkoxy)-C₁₋₆-alkylaminocarbonyl-C₁₋₆-alkoxy, (N-C₁₋₆-alkoxy)-C₁₋₆-alkylaminocarbonyl-C₁₋₆-alkyl, C₁₋₆-alkoxy-C₁₋₆-alkylcarbamoyl, C₁₋₆-alkoxy-C₁₋₆-alkylcarbonyl, C₁₋₆-alkoxy-C₁₋₆-alkylcarbonylamino, 1-C₁₋₆-alkoxy-C₁₋₆-alkylimidazol-2-yl, 2-C₁₋₆-alkoxy-C₁₋₆-alkyl-4-oxoimidazol-1-yl, 1-C₁₋₆-alkoxy-C₁₋₆-alkyltetrazol-5-yl, 5-C₁₋₆-alkoxy-C₁₋₆-alkyltetrazol-1-yl, 6-alkoxyaminocarbonyl-C₁₋₆-alkoxy, C₁₋₆-alkoxyaminocarbonyl-C₁₋₆-alkyl, C₁₋₆-alkoxycarbonyl, C₁₋₆-alkoxycarbonyl-C₁₋₆-alkoxy, C₁₋₆-alkoxycarbonyl-C₁₋₆-alkyl, C₁₋₆-alkoxycarbonylamino-C₁₋₆-alkoxy, C₁₋₆-alkoxycarbonylamino-C₁₋₆-alkyl, C₁₋₆-alkyl, (N-C₁₋₆-alkyl)-C₁₋₆-alkoxy-C₁₋₆-alkylcarbamoyl, (N-C₁₋₆-alkyl)-C₁₋₆-alkoxy-C₁₋₆-alkylcarbonylamino, (N-C₁₋₆-alkyl)-C₁₋₆-alkoxycarbonylamino, (N-C₁₋₆-alkyl)-C₀₋₆-alkylcarbonylamino-C₁₋₆-alkoxy, (N-C₁₋₆-alkyl)-C₀₋₆-alkylcarbonylamino-C₁₋₆-alkyl, (N-C₁₋₆-alkyl)-C₁₋₆-alkylsulphonylamino-C₁₋₆-alkoxy, (N-C₁₋₆-alkyl)-C₁₋₆-alkylsulphonylamino-C₁₋₆-alkyl, C₁₋₆-alkylamidinyl, C₁₋₆-alkylaminocarbonyl-C₁₋₆-alkoxy, di-C₁₋₆-alkylaminocarbonyl-C₁₋₆-alkoxy, C₁₋₆-alkylaminocarbonyl-C₁₋₆-alkoxy-C₁₋₆-alkyl, C₁₋₆-alkylaminocarbonyl-C₁₋₆-alkyl, C₁₋₆-alkylaminocarbonylamino-C₁₋₆-alkoxy, C₁₋₆-alkylaminocarbonylamino-C₁₋₆-alkyl, di-C₁₋₆-alkylaminocarbonyl-C₁₋₆-alkyl, C₁₋₆-alkylamino-C₂₋₆-alkoxy, di-C₁₋₆-alkylamino-C₂₋₆-alkoxy, C₁₋₆-alkylamino-C₁₋₆-alkyl, di-C₁₋₆-alkylamino-C₁₋₆-alkyl, C₁₋₆-alkylcarbamoyl, di-C₁₋₆-alkylcarbamoyl, C₀₋₆-alkylcarbonyl, C₀₋₆-alkylcarbonylamino-C₁₋₆-alkoxy, C₀₋₆-alkylcarbonylamino, C₀₋₆-alkylcarbonylamino-C₁₋₆-alkyl, C₁₋₆-alkylcarbonyloxy-C₁₋₆-alkoxy, C₁₋₆-alkylcarbonyloxy-C₁₋₆-alkyl, C₁₋₆-alkylsulphonyl, C₁₋₆-alkylsulphonyl-C₁₋₆-alkoxy, C₁₋₆-alkylsulphonyl-C₁₋₆-alkyl, C₁₋₆-alkylsulphonylamino-C₁₋₆-alkoxy, C₁₋₆-alkylsulphonylamino-C₁₋₆-alkyl, carbamoyl, carbamoyl-C₁₋₆-alkoxy, carbamoyl-C₁₋₆-alkyl, carboxy-C₁₋₆-alkoxy, carboxy-C₁₋₆-alkoxy-C₁₋₆-alkyl, carboxy-C₁₋₆-alkyl, cyano, cyano-C₁₋₆-alkoxy, cyano-C₁₋₆-alkyl, C₃₋₆-cycloalkylcarbonylamino-C₁₋₆-alkoxy, C₃₋₆-cycloalkylcarbonylamino-C₁₋₆-alkyl, cyclopropyl-C₁₋₆-alkyl, O,N-dimethylhydroxyl-amino-C₁₋₆-alkyl, halo-C₁₋₆-alkoxy, halo-C₁₋₆-alkyl, halogen, hydroxy-C₁₋₆-alkoxy-C₁₋₆-alkoxy, hydroxy-C₁₋₆-alkoxy-C₁₋₆-alkyl, hydroxy-C₁₋₆-alkyl, (N-hydroxy)-C₁₋₆-alkylaminocarbonyl-C₁₋₆-alkoxy, (N-hydroxy)-C₁₋₆-alkylaminocarbonyl-C₁₋₆-alkyl, (Nhydroxy)aminocarbonyl-C₁₋₆-alkoxy, (N-hydroxy)aminocarbonyl-C₁₋₆-alkyl, 2-oxooxazolidinyl-C₁₋₆-alkoxy, 2-oxooxazolidinyl-C₁₋₆-alkyl, O-methyloximyl-C₁₋₆-alkyl or trifluoromethyl; or
(E) R¹ is aryl which is substituted by 3-acetamidomethylpyrrolidinyl 3-C₁₋₆-alkoxy-C₁₋₆-alkylpyrrolidinyl, 3,4-dihydroxypyrrolidinyl, 2,6-dimethylmorpholinyl, 3,5-dimethylmorpholinyl, dioxanyl, dioxolanyl, 4,4-dioxothiomorpholinyl, dithianyl, dithiolanyl, 2-hydroxymethylpyrrolidinyl, 4-hydroxypiperidinyl, 3-hydroxypyrrolidinyl, imidazolyl-alkoxy, imidazolylalkyl, 2-methylimidazolylalkoxy, 2-methylimidazolylalkyl, 3-methyl[1,2,4]oxadiazol-5-ylalkoxy, 5-methyl[1,2,4]oxadiazol-3-ylalkoxy, 3-methyl-[1,2,4]oxadiazol-5-ylalkyl, 5-methyl[1,2,4]oxadiazol-3-ylalkyl, 4-methylpiperazinyl, 5-methyltetrazol-1-ylalkoxy, 5-methyltetrazol-1-ylalkyl, morpholinyl, [1,2,4]oxadiazol-5-ylalkoxy, [1,2,4]oxadiazol-5-ylalkyl, oxazol-4-ylalkoxy, oxazol-4-ylalkyl, 2-oxo-[1,3]oxazinyl, 2-oxooxazolidinyl, 2-oxoimidazolidinyl, 2-oxopyrrolidinyl, 4-oxopiperidinyl, 2-oxopyrrolidinylalkoxy, 2-oxopyrrolidinylalkyl, 2-oxotetrahydropyrimidinyl 4-oxothiomorpholinyl, piperazinyl, piperidinyl, pyrrolidinyl, pyrrolyl, [1,2,4]triazol-1-ylalkoxy, [1,2,4]triazol-4-ylalkoxy, [1,2,4]triazol-1-ylalkyl, [1,2,4]triazol-4-ylalkyl, tetrazol-1-ylalkoxy, tetrazol-2-ylalkoxy, tetrazol-5-ylalkoxy, tetrazol-1-ylalkyl, tetrazol-2-ylalkyl, tetrazol-5-ylalkyl, thiazol-4-ylalkoxy, thiazo-4-ylalkyl, thiomorpholinyl; or
(F) R¹ is heterocyclyl, optionally substituted, in particular as specified under (D) or (E), in particular benzo[1,3]dioxoyl, benzofuranyl, benzoxazolyl, dihydrobenzofuranyl, 3,4-dihydro-2H-benzo[1,4]oxazinyl, dihydro-3H-benzo[1,4]oxazinyl, dihydro-2H-benzo[1,4]thiazinyl, 2,3-dihydroindolyl, dihydro-1H-pyrido[2,3-b][1,4]oxazinyl, 1,1-dioxodihydro-2H-benzo[1,4]thiazinyl, indazolyl, indolyl, [1,5]naphthpyridyl, oxazolyl, 2-oxoazepanyl, 3-oxo-4H-benzo[1,4]oxazinyl, 2-oxobenzoxazolyl, 3-oxo-4H-benzo[1,4]thiazinyl, 2-oxodihydrobenzo[e][1,4]diazepinyl, 2-oxodihydro-benzo[d][1,3]oxazinyl, 2-oxodihydro-1H-quinazolinyl, 4-oxodihydroimidazolyl, 2-oxo-1,3-dihydroindolyl, 1-oxo-3H-isobenzofuranyl, 2-oxopiperidinyl 2-oxo-1H-pyrido[2,3-b][1,4]oxazinyl, 1-oxopyridyl, 2-oxotetrahydrobenzo[e][1,4]diazepinyl, 4-oxo-3H-thieno[2,3-d]pyrimidinyl, 5-oxo-4H-[1,2,4]triazinyl, phthalazinyl, pyrazolyl, 1 H-pyrrolizinyl, 1 H-pyrrolo[2,3-b]pyridyl, pyrrolyl, tetrahydroquinoxalinyl, tetrahydropyranyl, triazinyl, imidazo[1,5-a]pyridinyl, tetrahydro-imidazo[1,5-a]pyridinyl or 1,1,3-trioxodihydro-2H-1λ⁶-benzo[1,4]thiazinyl;
R² is phenyl, naphthyl, acenaphthyl, cyclohexyl, pyridyl, pyrimidinyl, pyrazinyl, oxopyridinyl, diazinyl, triazolyl, thienyl, oxazolyl, oxadiazolyl, thiazolyl, pyrrolyl, furyl, tetrazolyl or imidazolyl, which radicals may be substituted by 1-3 halogen, hydroxyl, cyano, trifluoromethyl, C₁₋₆-alkyl, halo-C₁₋₆-alkyl, hydroxy-C₁₋₆-alkyl, C₁₋₆-alkoxy-C₁₋₆-alkyl, cyano-C₁₋₆-alkyl, carboxy-C₁₋₆-alkyl, C₁₋₆-alkanoyloxy-C₁₋₆-alkyl, C₁₋₆-alkoxycarbonyloxy-C₁₋₆-alkyl, C₁₋₆-alkoxycarbonyl, or C₁₋₆-alkoxy groups, or a C₁₋₆-alkylenedioxy group, and/or by an L1-T1-L2-T2-L3-T3-L4-T4-L5-U radical;
L1, L2, L3, L4 and L5 are each independently a bond, C₁₋₈-alkylene, C₂₋₈-alkenylene or C₂₋₈-alkynylene, or are absent;
T1, T2, T3 and T4 are each independently
(a) a bond, or are absent, or are one of the groups
(b) -CH(OH)-
(c) -CH(OR⁶)-
(d) -CH(NR⁵R⁶)-
(e) -CO-
(f) -CR⁷R⁸-
(g) -O- or -NR⁶-
(h) -S(O)₀₋₂-
(i) -SO₂NR⁶-
(j) -NR⁶SO₂-
(k) -CONR⁶-
(l) -NR⁶CO-
(m) -O-CO-
(n) -CO-O-
(o) -O-CO-O-
(p) -O-CO-NR⁶-
(q) -N(R⁶)-CO-N(R⁶)-
(r) -N(R⁶)-CO-O-
(s) pyrrolidinylene, piperidinylene or piperazinylene
(t) -C(R¹¹)(R¹²)-,
where the bonds starting from (b)-(t) lead to a saturated or aromatic carbon atom of the adjacent group if the bond starts from a heteroatom, and where not more than two groups (b)-(f), three groups (g)-(h) and one group (i)-(t) is/are present;
R³ is hydrogen, hydroxyl, C₁₋₆-alkoxy or C₁₋₆-alkenyloxy;
R⁴ is C₁₋₆-alkoxy, C₁₋₆-alkoxy-C₁₋₆-alkoxy, optionally N-mono- or N,N-di-C₁-C₆-alkylated amino, optionally N-mono- or N,N-di-C₁-C₆-alkylated amino-C₁₋₆-alkoxy, optionally N-C₁₋₆-alkylated C₁₋₆-alkoxycarbonylamino-C₁₋₆-alkoxy, optionally N-C₁₋₆-alkylated C₁₋₆-alkylcarbonylamino-C₁₋₆-alkoxy, optionally N-C₁₋₆-alkylated C₃₋₈-cycloalkyl-C₁₋₆-alkylcarbonylamino-C₁₋₆-alkoxy, C₁₋₆-alkylcarbonyl-C₁₋₆-alkoxy, C₁₋₆-alkylcarbonyloxy, aryl-C₁₋₆-alkoxy, aryloxy, optionally N-mono- or N,N-di-C₁-C₆-alkylated carbamoyl-C₁₋₆-alkoxy, optionally N-mono- or N,N-di-C₃₋₈-cycloalkyl-C₁-C₆-alkylated carbamoyl-C₁₋₆-alkoxy, optionally N-mono- or N,N-di-C₁-C₆-alkylated carbamoyloxy, cyanoalkyloxy, C₃₋₈-cycloalkyloxy, C₃₋₈-cycloalkyl-C₁₋₆-alkoxy, C₃₋₈-cycloalkyloxy-C₁₋₆-alkoxy, hydroxyl, hydroxy-C₁₋₆-alkoxy, hydroxy-C₁₋₆-alkoxy-C₁₋₆-alkoxy, heterocyclyl-C₁₋₆-alkoxy, heterocyclyloxy, heterocyclyloxy-C₁₋₆-alkoxy or oxo;
R⁵ and R⁶ are each independently hydrogen, C₁₋₆-alkyl, C₂₋₆-alkenyl, aryl-C₁₋₆-alkyl or acyl, or, together with the N atom to which they are bonded, are a 5- to 6-membered heterocyclic ring which may contain an additional N, O or S atom or an -SO- or -SO₂-group, where the additional N atom may optionally be substituted by C₁₋₆-alkyl radicals;
R⁷ and R⁸, together with the carbon atom to which they are bonded, are a 3-7-membered ring which may contain one or two -O- or -S- atoms or-SO- or -SO₂-groups;
R⁹ is hydrogen, C₁₋₆-alkyl, C₁₋₆-alkoxy-C₁₋₆-alkyl, acyl, aryl-C₁₋₆-alkyl, C₃₋₈-cycloalkyl or C₃₋₈-cycloalkyl-C₁₋₆-alkyl;
R¹⁰ is carboxy-C₁₋₆-alkyl, C₁₋₆-alkoxycarbonyl-C₁₋₆-alkyl, C₁₋₆-alkyl or hydrogen;
R¹¹ is hydrogen, halogen or C₁₋₆-alkyl;
R¹² is hydrogen, halogen or C₁₋₆-alkyl;
R¹¹ and R¹², together with the C-atom to which they are attached, may also be C₃₋₈-cycloalkyl;
U is hydrogen, C₁₋₆-alkyl, cyano, trifluoromethyl, optionally substituted C₃₋₁₂-cycloalkyl, aryl, or heterocyclyl;
X is a bond, oxygen or sulphur or is >CR¹¹R¹², >CHOR⁹, -O-CO-, >CO, >C=NOR¹⁰, -O-CR¹¹R¹²-, -O-CR¹¹R¹²-CO-NR⁹-, -CO-NR⁹- or -NR⁹-, where a bond starting from a nitrogen, oxygen or sulphur atom leads to a saturated C atom of the Z group or to R¹;
W is oxygen or sulphur;
Z is C₁₋₆-alkylene, C₂₋₆-alkenylene, hydroxy-C₁₋₆-alkylidene, -0-, -N-, -S-, -O-alk-, -NR⁹-alk, -S-alk-, -alk-O-, -alk-S- or -alk-NR⁹-, where alk denotes C₁₋₆-alkylene; and where
(a) if Z is -0- or -S-, X is -CR¹¹R¹²- and either R² contains an L1-T1-L2-T2-L3-T3-L4-T4-L5-U substituent or R⁴ is a substituent other than hydrogen as defined above;
(b) if Z is -O-alk- or -S-alk-, X is -CR¹¹R¹²-; and
(c) if X is a bond, Z is C₁₋₆-alkylene, C₂₋₆-alkenylene, -NR⁹-alk-, -alk-NR⁹-, -alk-O- or -alk-S-;
n is 1 or, when X is -O-CO-, is 0 or 1;
m is 0 or 1;
or a pharmaceutically acceptable salt thereof.

5. A renin inhibitor according to Claim 1 or 2 being a compound of the general formula 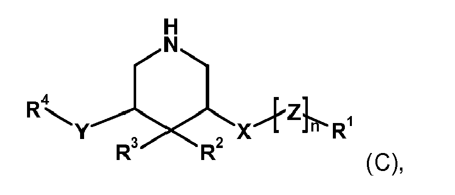 in which
(A) R¹ is aryl when R² is tetrazolyl or imidazolyl, each of which may be substituted by C₁₋₈alkoxy-C₁₋₈alkoxy-C₁₋₈alkyl, C₁₋₈alkoxy-C₁₋₈alkyl, aryloxy-C₁₋₈alkyl, heterocyclyloxy-C₁₋₈alkyl; or
(B) R¹ is aryl when X is -O-CHR⁵-CO-NR⁶-; or
(C) R¹ is aryl when Z is -Alk-NR⁶-, where Alk is C₁₋₈alkylene, and n is 1; or
(D) R¹ is aryl which is substituted by 1-4 acetamidinyl-C₁₋₈alkyl, acyl-C₁₋₈alkoxy-C₁₋₈alkyl, (N-acyl)-C₁₋₈alkoxy-C₁₋₈alkylamino, C₁₋₈alkoxy, C₁₋₈alkoxy-C₁₋₈alkoxy, C₁₋₈alkoxy-C₁₋₈alkoxy-C₁₋₈alkyl, C₁₋₈alkoxy-C₁₋₈alkyl, (N-C₁₋₈alkoxy)-C₁₋₈alkylaminocarbonyl-C₁₋₈alkoxy, (N-C₁₋₈alkoxy)-C₁₋₈alkylaminocarbonyl-C₁₋₈alkyl, C₁₋₈alkoxy-C₁₋₈alkylcarbamoyl, C₁₋₈alkoxy-C₁₋₈alkylcarbonyl, C₁₋₈alkoxy-C₁₋₈alkylcarbonylamino, 1-C₁₋₈alkoxy-C₁₋₈alkylimidazol-2-yl, 2-C₁₋₈alkoxy-C₁₋₈alkyl-4-oxoimidazol-1-yl, 1-C₁₋₈alkoxy-C₁₋₈alkyltetrazol-5-yl, 5-C₁₋₈alkoxy-C₁₋₈alkyltetrazol-1-yl, 6-alkoxy-aminocarbonyl-C₁₋₈alkoxy, C₁₋₈alkoxyaminocarbonyl-C₁₋₈alkyl, C₁₋₈alkoxycarbonyl, C₁₋₈alkoxycarbonyl-C₁₋₈alkoxy, C₁₋₈alkoxycarbonyl-C₁₋₈alkyl, C₁₋₈alkoxycarbonylamino-C₁₋₈alkoxy, C₁₋₈alkoxycarbonylamino-C₁₋₈alkyl, C₁₋₈alkyl, (N-C₁₋₈alkyl)-C₁₋₈alkoxy-C₁₋₈alkylcarbamoyl, (N-C₁₋₈alkyl)-C₁₋₈alkoxy-C₁₋₈alkylcarbonylamino, (N-C₁₋₈alkyl)-C₁₋₈alkoxycarbonylamino, (N-C₁₋₈alkyl)-C₀₋₈alkylcarbonylamino-C₁₋₈alkoxy, (N-C₁₋₈alkyl)-C₀₋₈alkylcarbonylamino-C₁₋₈alkyl, (N-C₁₋₈alkyl)-C₁₋₈alkylsulphonylamino-C₁₋₈alkoxy, (N-C₁₋₈alkyl)-C₁₋₈alkylsulphonylamino-C₁₋₈alkyl, C₁₋₈alkylamidinyl, C₁₋₈alkylaminocarbonyl-C₁₋₈alkoxy, di-C₁₋₈alkylaminocarbonyl-C₁₋₈alkoxy, C₁₋₈alkylaminocarbonyl-C₁₋₈alkoxy-C₁₋₈alkyl, C₁₋₈alkylaminocarbonyl-C₁₋₈alkyl, C₁₋₈alkylaminocarbonylamino-C₁₋₈alkoxy, C₁₋₈alkylaminocarbonylamino-C₁₋₈alkyl, di-C₁₋₈alkylaminocarbonyl-C₁₋₈alkyl, C₁₋₈alkylaminO-C₂₋₈alkoxy, di-C₁₋₈alkylamino-C₂-₈alkoxy, C₁₋₈alkylamino-C₁₋₈alkyl, di-C₁₋₈alkylamino-C₁₋₈alkyl, C₁₋₈alkylcarbamoyl, di-C₁₋₈alkylcarbamoyl, C₀₋₈alkylcarbonylamino-C₁₋₈alkoxy, C₀₋₈alkylcarbonylamino, C₀₋₈alkylcarbonylamino-C₁₋₈alkyl, C₁₋₈alkylcarbonyloxy-C₁₋₈alkoxy, C₁₋₈alkylcarbonyloxy-C₁₋₈alkyl, C₁₋₈alkylsulphonyl, C₁₋₈alkylsulphonyl-C₁₋₈alkoxy, C₁₋₈alkylsulphonyl-C₁₋₈alkyl, C₁₋₈alkylsulphonylamino-C₁₋₈alkoxy, C₁₋₈alkylsulphonylamino-C₁₋₈alkyl, carbamoyl, carbamoyl-C₁₋₈alkoxy, carbamoyl-C₁₋₈alkyl, carboxy-C₁₋₈alkoxy, carboxy-C₁₋₈alkoxy-C₁₋₈alkyl, carboxy-C₁₋₈alkyl, cyano, cyano-C₁₋₈alkoxy, cyano-C₁₋₈alkyl, C₃₋₈cycloalkyl-C₁₋₈alkoxy, C₃₋₈cycloalkyl-C₁₋₈alkyl, C₃₋₈cycloalkyl-carbonylamino-C₁₋₈alkoxy, C₃₋₈cycloalkylcarbonylamino-C₁₋₈alkyl, O,N-dimethyl-hydroxylamino-C₁₋₈alkyl, halogen, hydroxy-C₁₋₈alkoxy-C₁₋₈alkoxy, hydroxy-C₁₋₈alkoxy-C₁₋₈alkyl, hydroxy-C₁₋₈alkyl, (N-hydroxy)-C₁₋₈alkylaminocarbonyl-C₁₋₈alkoxy, (N-hydroxy)-C₁₋₈alkylaminocarbonyl-C₁₋₈alkyl, (N-hydroxy)aminocarbonyl-C₁₋₈alkoxy, (N-hydroxy)aminocarbonyl-C₁₋₈alkyl, 2-oxoxazolidinyl-C₁₋₈alkoxy, 2-oxoxazolidinyl-C₁₋₈alkyl, O-methyloximyl-C₁₋₈alkyl or trifluoromethyl; or
(E) R¹ is aryl which is substituted by 1-4 3-acetamidomethylpyrrolidinyl, 3-C₁₋₈alkoxy-C₁₋₈alkylpyrrolidinyl, 3,4-dihydroxypyrrolidinyl, 2,6-dimethylmorpholinyl, 3,5-dimethylmorpholinyl, dioxanyl, dioxolanyl, 4,4-dioxothiomorpholinyl, dithianyl, dithiolanyl, 2-hydroxymethylpyrrolidinyl, 4-hydroxypiperidinyl, 3-hydroxypyrrolidinyl, imidazolyl-alkoxy, imidazolylalkyl, 2-methylimidazolylalkoxy, 2-methylimidazolylalkyl, 3-methyl-[1,2,4]-oxadiazol-5-ylalkoxy, 5-methyl-[1,2,4]-oxadiazol-3-ylalkoxy, 3-methyl-[1,2,4]-oxadiazol-5-ylalkyl, 5-methyl-[1,2,4]-oxadiazol-3-ylalkyl, 4-methylpiperazinyl, 5-methyltetrazol-1-ylalkoxy, 5-methyltetrazol-1-ylalkyl, morpholinyl, [1,2,4]-oxadiazol-5-ylalkoxy, [1,2,4]-oxadiazol-5-ylalkyl, oxazol-4-ylalkoxy, oxazol-4-ylalkyl, 2-oxo-[1,3]-oxazinyl, 2-oxoxazolidinyl, 2-oxoimidazolidinyl, 2-oxopyrrolidinyl, 4-oxopiperidinyl, 2-oxopyrrolidinylalkoxy, 2-oxopyrrolidinylalkyl, 2-oxotetrahydropyrimidinyl 4-oxo-thiomorpholinyl, piperazinyl, piperidinyl, pyrrolidinyl, pyrrolyl, [1,2,4]-triazol-1-yl-alkoxy, [1,2,4]-triazol-4-ylalkoxy, [1,2,4]-triazol-1-ylalkyl, [1,2,4]-triazol-4-ylalkyl, tetrazol-1-ylalkoxy, tetrazol-2-ylalkoxy, tetrazol-5-ylalkoxy, tetrazol-1-ylalkyl, tetrazol-2-ylalkyl, tetrazol-5-ylalkyl, thiazol-4-ylalkoxy, thiazol-4-ylalkyl or thiomorpholinyl; or
(F) R¹ is heterocyclyl optionally substituted by oxo or oxide or as indicated under (D) or (E), especially azepanyl, benzo[1,3]dioxolyl, benzofuranyl, benzoimidazolyl, 4H-benzo[1,4]oxazinyl, benzoxazolyl, 4H-benzo[1,4]thiazinyl, 1H-quinolinyl, 2H-chromenyl, dihydrobenzo[e][1,4]diazepinyl, dihydrobenzofuranyl, 3,4-dihydro-2H-benzo[1,4]oxazinyl, dihydro-3H-benzo[1,4]oxazinyl, dihydrobenzo[d][1,3]oxazinyl, dihydro-2H-benzo[1,4]thiazinyl, dihydro-2H-1λ6-benzo[1,4]thiazinyl, dihydro-1H-quinazolinyl, 1a,7b-dihydro-1H-cyclopropa[c]chromenyl, dihydroimidazolyl, 1,3-dihydroindolyl, 2,3-dihydroindolyl, dihydro-1H-pyrido[2,3-b][1,4]oxazinyl, indazolyl, indolyl, 3H-isobenzofuranyl, [1,5]naphthyridyl, oxazolyl, phthalazinyl, piperidinyl, pyrazolyl, 1 H-pyrido[2,3-b][1,4]oxazinyl, pyridyl, 1 H-pyrrolizinyl, 1 H-pyrrolo[2,3-b]pyridyl, pyrrolyl, tetrahydrobenzo[e][1,4]diazepinyl, 3H-thieno[2,3-d]pyrimidinyl, tetrahydro-quinoxalinyl, 1,1a,2,7b-tetrahydrocyclopropa[c]chromenyl, tetrahydropyranyl or triazinyl;
R² is phenyl, naphthyl, acenaphthyl, cyclohexyl, pyridyl, pyrimidinyl, pyrazinyl, oxopyridinyl, diazinyl, triazolyl, thienyl, oxazolyl, oxadiazolyl, thiazolyl, pyrrolyl, furyl, tetrazolyl or imidazolyl, where the radicals are substituted by 1-3 C₂₋₈alkenyloxy-C₁₋₈alkyl, C₁₋₈alkoxy-C₁₋₈alkyl, C₁₋₈alkoxy-C₁₋₈alkylamino-C₁₋₈alkyl, C₁₋₈alkoxy-C₁₋₈alkylsulphanyl-C₁₋₈alkyl, C₁₋₈alkoxy-C₀₋₈alkyl-C₃₋₈cycloalkyl-C₀₋₈alkoxy-C₁₋₈alkyl, C₁₋₈alkyl, C₁₋₈alkylsulphanyl-C₁₋₈alkoxy-C₁₋₈alkyl, C₁₋₈alkylsulphanyl-C₁₋₈alkyl, C₁₋₈alkylsulphonyl-C₁₋₈alkoxy-C₁₋₈alkyl, C₃₋₈cycloalkyl-C₀₋₈alkoxy-C₁₋₈alkoxy-C₁₋₈alkyl, C₃₋₈cycloalkyl-C₀₋₈alkoxy-C₁₋₈alkyl, optionally halogen-substituted C₁₋₈alkoxy-C₁₋₈alkoxy-C₁₋₈alkyl, or (oxygen-heterocyclyl)-C₀₋₈alkoxy-C₁₋₈alkyl, and may, in addition to the aforementioned substituents, also be substituted by a maximum of 4 halogen;
R³ is hydrogen, hydroxy, C₁₋₈alkoxy or C₂₋₈alkenyloxy;
R⁴ is optionally halogen- and/or hydroxy-substituted C₁₋₈alkyl, optionally halogen-and/or hydroxy-substituted C₁₋₈alkoxy-C₁₋₈alkyl, optionally N-mono- or N,N-di-C₁₋₈-alkylated amino-C₁₋₈alkyl, optionally N-mono- or N,N-di-C₁₋₈-alkylated or optionally hydroxy-substituted amino-C₀₋₈alkylcarbonyl-C₁₋₈alkyl, hydroxy-C₀₋₈alkylcarbonyl-C₀₋₈alkyl, C₁₋₈alkoxy-C₀₋₈alkylcarbonyl-C₀₋₈alkyl, optionally N-C₁₋₈-alkylated C₁₋₈alkoxycarbonylamino-C₁₋₈alkyl, optionally N-C₁₋₈-alkylated C₁₋₈alkoxy-C₁₋₈alkylamino-C₁₋₈alkyl, optionally N-C₁₋₈-alkylated or optionally halogen-substituted C₁₋₈alkylcarbonylamino-C₁₋₈alkyl, cyano-C₁₋₈alkyl, optionally N-C₁₋₈-alkylated or optionally halogen-substituted C₃₋₈-cycloalkyl-C₀₋₈alkylcarbonylamino-C₁₋₈alkyl, optionally N-C₁₋₈-alkylated hydroxy-C₁₋₈alkylamino-C₁₋₈alkyl, heterocyclylcarbonyl-C₀₋₈alkylamino-C₁₋₈alkyl, C₁₋₈alkoxycarbonylamino-C₁₋₈alkyl, optionally N-C₁₋₈-alkylated or optionally halogen-substituted heterocyclyl-C₀₋₈alkylcarbonylamino-C₁₋₈alkyl, C₃₋₈cycloalkyl-C₀₋₈alkyl, C₃₋₈cycloalkyloxy-C₁₋₈alkyl, heterocyclyl-C₀₋₈-(optionally hydroxy-substituted)alkyl, optionally N-C₁₋₈-alkylated heterocyclyl-C₀₋₈alkylamino-C₀₋₈alkylcarbonyl-C₀₋₈alkyl, C₁₋₈alkylsulphonyl-C₁₋₈alkyl, C₂₋₈alkinyl, heterocyclyl-C₂₋₈alkinyl, optionally N-mono- or N,N-di-C₁₋₈-alkylated amino-C₂₋₈alkinyl, N-mono- or N,N-di-C₁₋₈-alkylated aminocarbonyl-C₂₋₈alkinyl, heterocyclylcarbonyl-C₀₋₈alkyl or heterocyclyloxy-C₁₋₈alkyl;
and where
(a) if Y is -O- and R² is not para-C₁₋₈alkyl-substituted phenyl, then R⁴ may additionally also be hydrogen, and
(b) if Y is oxo, then R⁴ is absent;
R⁵ is acyl, C₂₋₈alkenyl, C₁₋₈alkyl, aryl-C₁₋₈alkyl or hydrogen;
R⁶ is acyl, C₁₋₈alkoxy-C₁₋₈alkyl, C₁₋₈alkyl or aryl-C₁₋₈alkyl or hydrogen;
R⁷ is C₁₋₈alkoxycarbonyl-C₁₋₈alkyl, C₁₋₈alkyl, carboxy-C₁₋₈alkyl or hydrogen;
X is a bond, oxygen or sulphur, where the bond originating from an oxygen or sulphur atom leads to a saturated C atom of the group Z, or is a group >CH-R⁵, >CHOR⁶, - O-CO-, >CO, >C=NOR⁷, -O-CHR⁵- or -O-CHR⁵-CO-NR⁶-;
Y is -O-, oxo or a bond;
Z is C₁₋₈alkylene, C₂₋₈alkenylene, hydroxy-C₁₋₈alkylidene, -O-, -S-, -O-Alk-, -S-Alk-, -Alk-O-,
-Alk-S- or -Alk-NR⁶-, where Alk is C₁₋₈alkylene; and where
(a) if Z is -O-Alk- or -S-Alk-, then X is -CHR⁵-; and
(b) if X is a bond, then Z is C₂₋₈alkenylene, -Alk-O- or -Alk-S-;
n is 1, or, if X is -O-CO- or -O-CHR⁵-CO-NR⁶-, is 0 or 1;
or a pharmaceutically acceptable salt thereof.

6. A renin inhibitor according to Claim 1 or 2 being a compound of the general formula 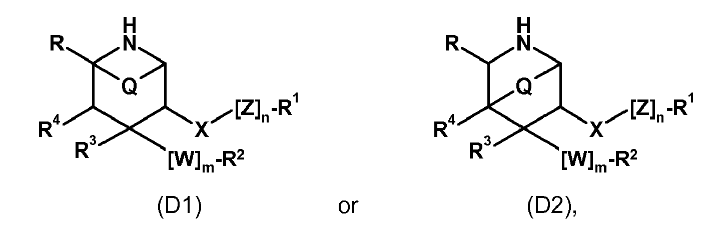 where
R is C₂₋₈-alkenyl, C₁₋₈-alkyl, C₂₋₈-alkynyl, C₀₋₈-alkyl-carbonyl-amino-C₁₋₈-alkyl, C₃₋₈-cycloalkyl-C₀₋₈-alkyl, C₁₋₈-alkyl-sulfonyl-C₁₋₈-alkyl, optionally N-mono- or N,N-di-C₁₋₈-alkylated carbamoyl-C₀₋₈-alkyl, optionally O-C₁₋₈-alkylated carboxyl-C₀₋₈-alkyl, optionally N and/or N' mono-, di- or tri-C₁₋₈-alkylated ureido-C₁₋₈-alkyl or hetero-cyclylcarbonyl-C₀₋₈-alkyl, each of said radicals may be substituted, preferably by 1-4 C₁₋₈-alkoxy, C₁₋₈-alkoxy-C₁₋₈-alkoxy, C₁₋₈-alkoxycarbonyl-(N-C₁₋₈-alkyl)-amino, C₁₋₈-alkyl, C₁₋₈-alkyl-carbonyl-(N-C₁₋₈-alkyl)-amino, C₁₋₈-alkyl-sulfanyl, C₁₋₈-alkyl-sulfinyl, aryl-C₀₋₈-alkoxy, which is optionally substituted by 1 or 2 aryl or C₁₋₈-alkoxy radicals, aryl, which is optionally substituted by 1 or 2 aryl or C₁₋₈-alkoxy radicals, aryl-amino, cyano, C₃₋₈-cycloalkoxy, halogen, heterocyclyl-C₀₋₈-alkyl, heterocyclyl-C₀₋₈-alkoxy, heterocyclyl-C₀₋₈-alkyl-amino, heterocyclyl-carbonyl, hydroxyl, optionally N-mono- or N,N-di-C₁₋₈-alkylated amino, optionally N-mono- or N,N-di-C₁₋₈-alkylated carbamoyl-C₀₋₈-alkyl, optionally N-mono- or N,N-di-C₁₋₈-alkylated carbamoyloxy, optionally N-mono- or N,N-di-C₁₋₈-alkylated sulfamoyl, optionally N-arylated or N-heterocyclyl-substituted carbamoyl, oxo, trifluoromethoxy or trifluoromethyl;
R¹ is aryl or heterocyclyl;
R² is acenaphthyl, cyclohexyl, diazinyl, furyl, imidazolyl, naphthyl, oxadiazolyl, oxazolyl, phenyl, pyrazinyl, pyridyl, pyrimidinyl, pyrrolyl, oxopyridinyl, tetrazolyl, thienyl, or triazolyl, each of said radicals may be substituted by 1-3 C₁₋₈-alkanoyloxy-C₁₋₈-alkyl, C₂₋₈-alkenyloxy, C₁₋₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, C₁₋₈-alkoxycarbonyl, C₁₋₈-alkoxycarbonyloxy-C₁₋₈-alkyl, C₁₋₈-alkyl, carboxy-C₁₋₈-alkyl, C₁₋₈-alkoxy-C₁₋₈-alkylsulfanyl, C₁₋₈-alkyl-sulfanyl, C₁₋₈-alkylsulfanyl-C₁₋₈-alkoxy, C₁₋₈-alkylsulfanyl-C₁₋₈-alkoxy-C₁₋₈-alkyl, C₁₋₈-alkylsulfanyl-C₁₋₈-alkyl, cyano, cyano-C₁₋₈-alkyl, C₃₋₈-cycloalkyl-C₀₋₆-alkoxy-C₁₋₈-alkoxy, C₁₋₆-alkoxy-C₀₋₆-alkyl-C₃₋₈-cycloalkyl-C₀₋₆-alkoxy-C₁₋₈-alkyl, C₃₋₈-cycloalkyl-C₀₋₆-alkoxy-C₁₋₈-alkyl, halo-C₁₋₈-alkyl, halogen, hydroxy-C₁₋₈-alkyl, hydroxyl, oxide, trifluoromethoxy or trifluoromethyl groups, or a C₁₋₈-alkylenedioxy group, and/or by an L1-T1-L2-T2-L3-T3-L4-T4-L5-U radical;
L1, L2, L3, L4 and L5 are each independently a bond, C₁₋₈-alkylene, C₂₋₈-alkenylene or C₂₋₈-alkynylene, C₃₋₈-cycloalkene or are absent;
T1, T2, T3 and T4 are each independently
(a) a bond, or are absent, or are one of the groups
(b) -CH(OH)-
(c) -CH(OR⁶)-
(d) -CH(NR⁵R⁶)-
(e) -CO-
(f) -CR⁷R⁸-
(g) -O- or -NR⁶-
(h) -S(O)₀₋₂-
(i) -SO₂NR⁶-
(j) -NR⁶SO₂-
(k) -CONR⁶-
(l) -NR⁶CO-
(m) -O-CO-
(n) -CO-O-
(o) -O-CO-O-
(p) -O-CO-NR⁶-
(q) -N(R⁶)-CO-N(R⁶)-
(r) -N(R⁶)-CO-O-
(s) pyrrolidinylene, piperidinylene or piperazinylene
(t) -C(R¹¹)(R¹²)-,
where the bonds starting from (b)-(t) lead to a saturated or aromatic carbon atom of the adjacent group if the bond starts from a heteroatom, and where not more than two (b)-(f) groups, three (g)-(h) groups and one (i)-(t) group are present;
R³ is hydrogen, hydroxyl, C₁₋₈-alkoxy or C₂₋₈-alkenyloxy;
R⁴ is hydrogen, C₂₋₈-alkenyl, C₁₋₈-alkoxy, C₁₋₈-alkoxy-C₁₋₈-alkyl, C₁₋₈-alkoxy-C₁₋₈-alkoxy, C₁₋₈-alkyl, optionally (N-C₁₋₈-alkyl)-C₁₋₈-alkoxycarbonyl-amino-C₁₋₈-alkoxy, optionally (N-C₁₋₈-alkyl)-C₁₋₈-alkylcarbonyl-amino-C₁₋₈-alkoxy, optionally (N-mono- or N,N-di-C₁-C₈-alkyl)-amino-C₁₋₈-alkoxy, benzyl, C₃₋₈-cycloalkyloxy, C₃₋₈-cycloalkyloxy-C₁₋₈-alkoxy, heterocyclyl-C₀₋₈-alkoxy, heterocyclyloxy-C₁₋₈-alkoxy, hydroxy, hydroxy-C₁₋₈-alkoxy-C₁₋₈-alkoxy, hydroxy-C₁₋₈-alkyl, oxo or a
R^{4a}-Z1-X1- group where R^{4a} is
(a) H-
(b) C₁₋₈-alkyl-
(c) C₂₋₈-alkenyl-
(d) hydroxy-C₁₋₈-alkyl-
(e) polyhydroxy-C₁₋₈-alkyl-
(f) C₁₋₈-alkyl-O-C₁₋₈-alkyl-
(g) aryl-
(h) heterocyclyl-
(i) arylalkyl-
(j) heterocyclylalkyl-
(k) aryloxyalkyl-
(l) heterocyclyloxyalkyl-
(m) (R⁵,R⁶)N-(CH₂)₁₋₃-
(n) (R⁵,R⁶)N-
(o) C₁₋₈-alkyl-S(O)₀₋₂-
(p) aryl-S(O)₀₋₂-
(q) heterocyclyl-S(O)₀₋₂-
(r) HO-SO₃- or salts thereof
(s) H₂N-C(NH)-NH-
(t) NC-
and the bonds starting from (n)-(t) lead to a carbon atom of the adjacent group and this carbon atom is saturated if the bond starts from a heteroatom;
Z1
(a) is a bond, is absent, or is one of the groups
(b) -C₁₋₈-alkylene-
(c) -C₂₋₈-alkenylene-
(d) -O-, -N(R¹¹)-, -S(O)₀₋₂-
(e) -CO-
(f) -O-CO-
(g) -O-CO-O-
(h) -O-CO-N(R¹¹)-
(i) -N(R¹¹)-CO-O-
(j) -CO-N(R¹¹)-
(k) -N(R¹¹)-CO-
(l) -N(R¹¹)-CO-N(R¹¹)-
(m) -CH(OR⁹)-
and the bonds starting from (d) and (f)-(m) lead to a carbon atom of the adjacent group and this carbon atom is saturated if the bond starts from a heteroatom;
X1
(a) is a bond, is absent, or is one of the groups
(b) -0-
(c) -N(R¹¹)-
(d) -S(O)₀₋₂-
(e) -(CH₂)₁₋₃-;
or R³ and R⁴ in formula (I) together are a bond;
R⁵ and R⁶ are each independently hydrogen, C₁₋₈-alkyl, C₂₋₈-alkenyl, aryl-C₁₋₈-alkyl or acyl, or, together with the nitrogen atom to which they are bonded, are a 5- or 6-membered heterocyclic ring which may contain an additional nitrogen, oxygen or sulfur atom or a -SO- or -SO₂- group, and the additional nitrogen atom may optionally be substituted by C₁₋₈-alkyl radicals;
R⁷ and R⁸, together with the carbon atom to which they are bonded, are a 3-7-membered ring which may contain one or two -0- or -S - atoms or -SO- or -SO₂-groups;
R⁹ is hydrogen, C₁₋₈-alkyl, C₁₋₈-alkoxy-C₁₋₈-alkyl, acyl or arylalkyl;
R¹⁰ is carboxyalkyl, alkoxycarbonylalkyl, alkyl or hydrogen;
R¹¹ is hydrogen or C₁₋₈-alkyl;
R¹² is hydrogen or C₁₋₈-alkyl;
Q is ethylene or is absent (formula I) or is ethylene or methylene (formula II);
U is hydrogen, C₁₋₈-alkyl, cyano, optionally substituted C₃₋₈-cycloalkyl, aryl or heterocyclyl;
W is oxygen or sulfur;
X is a bond, oxygen or sulfur, or is a >CH-R¹¹, >CHOR⁹, -O-CO-, >CO, >C=NOR¹⁰, - O-CHR¹¹- or -O-CHR¹¹-CO-NR⁹- group and the bond starting from an oxygen or sulfur atom leads to a saturated carbon atom of the Z group or to R¹;
Z is C₁₋₈-alkylene, C₂₋₈-alkenylene, hydroxy-C₁₋₈-alkylidene, -0-, -S-, -O-alk-, -S-alk-, - alk-O-, -alk-S- or -alk-NR⁹-, where alk is C₁₋₈-alkylene; and where
(a) if Z is -0- or -S-, X is >CH-R¹¹ and either R² contains an L1-T1-L2-T2-L3-T3-L4-T4-L5-U substituent or R⁴ is a substituent other than hydrogen as defined above;
(b) if Z is -O-alk- or -S-alk-, X is >CH-R¹¹; and
(c) if X is a bond, Z is C₂₋₈-alkenylene, -alk-O- or -alk-S-;
m is 0 or 1;
n is 0 or 1;
or a pharmaceutically acceptable salt thereof.

7. A renin inhibitor according to Claim 1 or 2 being a compound of the general formula E 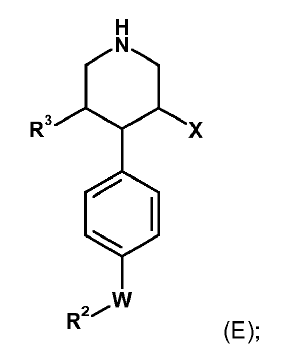 in which
(A) R¹ is heterocyclyl substituted by oxo or oxide or as indicated under (B) or (C), in particular azepanyl, benzo[1,3]dioxolyl, benzofuranyl, benzoimidazolyl, 4H-benzo[1,4]oxazinyl, benzooxazolyl, 4H-benzo[1,4]thiazinyl, quinolinyl, chromenyl, dihydro-benzo[e][1,4]diazepinyl, dihydrobenzofuranyl, 3,4-dihydro-2H-benzo[1,4]oxazinyl, dihydro-3H-benzo[1,4]oxazinyl, dihydrobenzo[d][1,3]oxazinyl, 3,4-dihydro-2H-benzo[1,4]thiazinyl, dihydro-2H-1λ6-benzo[1,4]thiazinyl, dihydro-1H-quinazolinyl, 1a,7b-dihydro-1H-cyclopropa[c]chromenyl, dihydroimidazolyl, 1,3-dihydroindolyl, 2,3-dihydroindolyl, dihydro-1H-pyrido[2,3-b][1,4]oxazinyl, indazolyl, indolyl, 3H-isobenzofuranyl, [1,5]naphthyridyl, oxazolyl, phthalazinyl, piperidinyl, pyrazolyl, 1 H-pyrido[2,3-b][1,4]oxazinyl, pyridyl, 1 H-pyrrolizinyl, 1 H-pyrrolo[2,3-b]pyridyl, pyrrolyl, tetrahydrobenzo[e][1,4]diazepinyl, 3H-thieno[2,3-d]pyrimidinyl, tetrahydro-quinoxalinyl, 1,1a,2,7b-tetrahydrocyclopropa[c]chromenyl, tetrahydropyranyl or triazinyl; or
(B) R¹ is aryl which is substituted by 1-4-acetamidinyl-₁₋₈alkyl, acyl-C₁₋₈alkoxy-C₁₋₈alkyl, (N-acyl)-C₁₋₈alkoxy-C₁₋₈alkylamino, C₁₋₈alkoxy, C₁₋₈alkoxy-C₁₋₈alkoxy, C₁₋₈alkoxy-C₁₋₈alkoxy-C₁₋₈alkyl, C₁₋₈alkoxy-C₁₋₈alkyl, (N-C₁₋₈alkoxy)-C₁₋₈alkylaminocarbonyl-C₁₋₈alkoxy, (N-C₁₋₈alkoxy)-C₁₋₈alkylaminocarbonyl-C₁₋₈alkyl, C₁₋₈alkoxy-C₁₋₈alkyl-carbamoyl, C₁₋₈alkoxy-C₁₋₈alkylcarbonyl, C₁₋₈alkoxy-C₁₋₈alkylcarbonylamino, 1-C₁₋₈alkoxy-C₁₋₈alkylimidazol-2-yl, 2-C₁₋₈alkoxy-C₁₋₈alkyl-4-oxoimidazol-1-yl, 1-C₁₋₈alkoxy-C₁₋₈-alkyltetrazol-5-yl, 5-C₁₋₈alkoxy-C₁₋₈-alkyltetrazol-1-yl, 6-alkoxy-aminocarbonyl-C₁₋₈alkoxy, C₁₋₈alkoxyaminocarbonyl-C₁₋₈alkyl, C₁₋₈alkoxycarbonyl, C₁₋₈alkoxycarbonyl-C₁₋₈alkoxy, C₁₋₈alkoxycarbonyl-C₁₋₈alkyl, C₁₋₈alkoxycarbonylamino-C₁₋₈alkoxy, C₁₋₈alkoxycarbonylamino-C₁₋₈alkyl, C₁₋₈alkyl, (N-C₁₋₈alkyl)-C₁₋₈alkoxy-C₁₋₈alkylcarbamoyl, (N-C₁₋₈alkyl)-C₁₋₈alkoxy-C₁₋₈alkylcarbonylamino, (N-C₁₋₈alkyl)-C₁₋₈alkoxycarbonylamino, (N-C₁₋₈alkyl)-C₀₋₈alkylcarbonylamino-C₁₋₈alkoxy, (N-C₁₋₈alkyl)-C₀₋₈alkylcarbonylamino-C₁₋₈alkyl, (N-C₁₋₈alkyl)-C₁₋₈alkylsulfonylamino-C₁₋₈alkoxy, (N-C₁₋₈alkyl)-C₁₋₈alkylsulfonylamino-C₁₋₈alkyl, C₁₋₈alkyl-amidinyl, C₁₋₈alkylaminocarbonyl-C₁₋₈alkoxy, di-C₁₋₈alkylaminocarbonyl-C₁₋₈alkoxy, C₁₋₈alkylaminocarbonyl-C₁₋₈alkoxy-C₁₋₈alkyl, C₁₋₈alkylaminocarbonyl-C₁₋₈alkyl, C₁₋₈alkylaminocarbonylamino-C₁₋₈alkoxy, C₁₋₈alkylaminocarbonylamino-C₁₋₈alkyl, di-C₁₋₈alkylaminocarbonyl-C₁₋₈alkyl, C₁₋₈alkylamino-C₂₋₈alkoxy, di-C₁₋₈alkylamino-C₂₋₈alkoxy, C₁₋₈alkylamino-C₁₋₈alkyl, di-C₁₋₈alkylamino-C₁₋₈alkyl, C₁₋₈alkylcarbamoyl, di-C₁₋₈alkylcarbamoyl, C₀₋₈alkylcarbonylamino-C₁₋₈alkoxy, C₀₋₈alkylcarbonylamino, C₀₋₈alkylcarbonylamino-C₁₋₈alkyl, C₁₋₈alkylcarbonyloxy-C₁₋₈alkoxy, C₁₋₈alkylcarbonyloxy-C₁₋₈alkyl, C₁₋₈alkylsulfonyl, C₁₋₈alkylsulfonyl-C₁₋₈alkoxy, C₁₋₈alkylsulfonyl-C₁₋₈alkyl, C₁₋₈alkylsulfonylamino-C₁₋₈alkoxy, C₁₋₈alkylsulfonylamino-C₁₋₈alkyl, carbamoyl, carbamoyl-C₁₋₈alkoxy, carbamoyl-C₁₋₈alkyl, carboxy-C₁₋₈alkoxy, carboxy-C₁₋₈alkoxy-C₁₋₈alkyl, carboxy-C₁₋₈alkyl, cyano, cyano-C₁₋₈alkoxy, cyano-C₁₋₈alkyl, C₃₋₈cycloalkyl-C₁₋₈alkoxy, C₃₋₈cycloalkyl-C₁₋₈alkyl, C₃₋₈cycloalkylcarbonyl-amino-C₁₋₈alkoxy, C₃₋₈cycloalkylcarbonylamino-C₁₋₈alkyl, O,N-dimethylhydroxylamino-C₁₋₈alkyl, halogen, hydroxy-C₁₋₈alkoxy-C₁₋₈alkoxy, hydroxy-C₁₋₈alkoxy-C₁₋₈alkyl, hydroxy-C₁₋₈alkyl, (N-hydroxy)-C₁₋₈alkylaminocarbonyl-C₁₋₈alkoxy, (N-hydroxy)-C₁₋₈alkylaminocarbonyl-C₁₋₈alkyl, (N-hydroxy)aminocarbonyl-C₁₋₈alkoxy, (N-hydroxy)aminocarbonyl-C₁₋₈alkyl, 2-oxooxazolidinyl-C₁₋₈alkoxy, 2-oxooxazolidinyl-C₁₋₈alkyl, O-methyloximyl-C₁₋₈alkyl, polyhalo-C₁₋₈alkoxy or polyhalo-C₁₋₈alkyl; or
(C) R¹ is aryl which is substituted by 3-acetamidomethylpyrrolidinyl, 3-C₁₋₈alkoxy-C₁₋₈alkyl-pyrrolidinyl, 3,4-dihydroxypyrrolidinyl, 2,6-dimethylmorpholinyl, 3,5-dimethylmorpholinyl, dioxanyl, dioxolanyl, 4,4-dioxothiomorpholinyl, dithianyl, dithiolanyl, 2-hydroxymethylpyrrolidinyl, 4-hydroxypiperidinyl, 3-hydroxypyrrolidinyl, imidazolyl-alkoxy, imidazolylalkyl, 2-methylimidazolylalkoxy, 2-methylimidazolylalkyl, 3-methyl-[1,2,4]-oxadiazol-5-ylalkoxy, 5-methyl-[1,2,4]-oxadiazol-3-ylalkoxy, 3-methyl-[1,2,4]-oxadiazol-5-ylalkyl, 5-methyl-[1,2,4]-oxadiazol-3-ylalkyl, 4-methylpiperazinyl, 5-methyltetrazol-1-ylalkoxy, 5-methyltetrazol-1-ylalkyl, morpholinyl, [1,2,4]-oxadiazol-5-ylalkoxy, [1,2,4]-oxadiazol-5-ylalkyl, oxazol-4-ylalkoxy, oxazol-4-ylalkyl, 2-oxo-[1,3]oxazinyl, 2-oxooxazolidinyl, 2-oxoimidazolidinyl, 2-oxopyrrolidinyl, 4-oxo-piperidinyl, 2-oxopyrrolidinylalkoxy, 2-oxopyrrolidinylalkyl, 2-oxotetrahydropyrimidinyl 4-oxothiomorpholinyl, piperazinyl, piperidinyl, pyrrolidinyl, pyrrolyl, [1,2,4]-triazol-1-yl-alkoxy, [1,2,4]-triazol-4-ylalkoxy, [1,2,4]-triazol-1-ylalkyl, [1,2,4]-triazol-4-ylalkyl, tetrazol-1-ylalkoxy, tetrazol-2-ylalkoxy, tetrazol-5-ylalkoxy, tetrazol-1-ylalkyl, tetrazol-2-ylalkyl, tetrazol-5-ylalkyl, thiazol-4-ylalkoxy, thiazol-4-ylalkyl or thiomorpholinyl; or
(D) R¹ is aryl when X is -O-CHR⁶-CO-NR⁴-R¹ or -O-CHR⁶-CO-NR⁴-Z, where Z is Alk-R¹ where Alk is C₁₋₈alkylene; or
(E) R¹ is aryl when X is -O-Z, where Z is Alk-NR⁴-R¹ or X is -Z, where Z is -Alk-NR⁴-R¹, where Alk is C₁₋₈alkylene;
R²
a) is absent when W is cyano; or
b) is C₁₋₈alkyl, C₂₋₈alkenyl, C₂₋₈alkynyl, C₁₋₈alkoxy-C₁₋₈alkyl, C₁₋₈alkoxy-C₃₋₈cycloalkyl-C₁₋₈-alkyl, C₁₋₈alkylsulfanyl-C₁₋₈alkyl, C₁₋₈alkylsulfonyl-C₁₋₈alkyl when W is -O- or -S-;
R³
a) is halogen- and/or hydroxy-substituted C₁₋₈alkoxy, halogen- and/or hydroxy-substituted C₁₋₈alkoxy-C₁₋₈alkoxy, branched C₁₋₈alkoxy-C₁₋₈alkoxy, optionally N-mono- or N,N-di-C₁₋₈-alkylated amino-C₁₋₈alkoxy, optionally N-C₁₋₈alkylated C₁₋₈alkoxy-C₁₋₈alkylamino-C₁₋₈alkoxy, optionally N-mono- or N,N-di-C₁₋₈alkylated amino-C₀₋₈-alkylcarbonyl-C₁₋₈alkoxy, hydroxy-C₀₋₈-alkylcarbonyl-C₀₋₈alkoxy, C₁₋₈alkoxy-C₀₋₈alkylcarbonyl-C₀₋₈alkoxy, C₁₋₈-alkylcarbonylamino-C₁₋₈alkoxy, cyano-C₁₋₈alkoxy, substituted C₃₋₈cycloalkyl-C₀₋₈alkoxy, heterocyclyl-C₀₋₈alkoxy, optionally N-C₁₋₈alkylated heterocyclyl-C₀₋₈alkylamino-C₀₋₈alkylcarbonyl-C₀₋₈alkoxy, C₁₋₈alkylsulfonyl-C₁₋₈alkoxy, C₂₋₈alkynyloxy, heterocyclyl-C₂₋₈alkynyloxy, optionally N-mono- or N,N-di-C₁₋₈alkylated amino-C₂₋₈alkynyloxy, N-mono- or N,N-di-C₁₋₈alkylated aminocarbonyl-C₂₋₈alkynyloxy, heterocyclylcarbonyl-C₀₋₈alkoxy, optionally N-mono- or N,N-di-C₁₋₈alkylated amino-C₁₋₈alkyl, optionally N-C₁₋₈alkylated C₁₋₈alkoxy-C₁₋₈alkylamino-C₁₋₈alkyl, optionally N-mono- or N,N-di-C₁₋₈alkylated and optionally hydroxy-substituted amino-C₀₋₈alkyl-carbonyl-C₀₋₈alkyl, optionally N-C₁₋₈alkylated heterocyclyl-C₀₋₈alkylamino-C₀₋₈alkylcarbonyl-C₀₋₈alkyl, optionally halogen- or hydroxy-substituted C₁₋₈alkoxy-C₁₋₈alkyl, optionally halogen- or hydroxy-substituted hydroxy-C₁₋₈alkyl, optionally N-C₁₋₈alkylated hydroxy-C₁₋₈alkylamino-C₁₋₈alkyl, heterocyclylcarbonyl-C₀₋₈alkyl, heterocyclylcarbonyl-C₀₋₈alkylamino-C₁₋₈alkyl, heterocyclyl-C₁₋₈alkyl, C₁₋₈alkoxycarbonylamino-C₁₋₈alkyl, optionally halogen-substituted heterocyclyl-C₀₋₈alkylcarbonylamino-C₁₋₈alkyl, optionally halogen-substituted C₃₋₈cycloalkyl-C₀₋₈alkylcarbonylamino-C₁₋₈alkyl or optionally halogen-substituted C₁₋₈alkylcarbonylamino-C₁₋₈alkyl; or additionally
b) is hydroxy, unsubstituted C₁₋₈alkoxy, unsubstituted, unbranched C₁₋₈alkoxy-C₁₋₈-alkoxy or unsubstituted C₃₋₈cycloalkyl-C₀₋₈alkoxy if -W-R² is not C₁₋₈alkoxy;
R⁴ is acyl, C₁₋₈alkoxy-C₁₋₈alkyl, C₁₋₈alkyl, aryl-C₁₋₈alkyl, C₃₋₈cycloalkyl-C₀₋₈alkyl, or hydrogen;
R⁵ is C₁₋₈alkoxycarbonyl-C₁₋₈alkyl, C₁₋₈alkyl, carboxy-C₁₋₈alkyl or hydrogen;
R⁶ is acyl, C₂₋₈alkenyl, C₁₋₈alkyl, aryl-C₁₋₈alkyl or hydrogen;
X is Z, -O-Z or -S-Z, where the bond originating from an oxygen or sulfur atom leads to a saturated C atom of the group Z, or is a group -CHR⁶-Z, -CHOR⁴-Z, -O-CO-Z, -O-CO-R¹ -CO-Z, -C=NOR⁵-Z, -O-CHR⁶-Z, -O-CHR⁶-CO-NR⁴-Z, -O-CHR⁶-CO-NR⁴-R¹, or -O-CHR⁶-R¹;
W is -O-, -S- or cyano;
Z is C₁₋₈-Alk-R¹, C₂₋₈alkenylene-R¹, hydroxy-substituted -Alk-R¹, -O-R¹, -S-R¹, -O-Alk-R¹,
-S-Alk-R¹, -Alk-O-R¹, -Alk-S-R¹ or -Alk-NR⁴-R¹, where Alk is C₁₋₈alkylene; and where
(a) X is -CH-R⁶-Z if Z is -O-R¹ or -S-R¹
(b) X is -CH-R⁶-Z if Z is -O-Alk-R¹ or -S-Alk-R¹; and
(c) Z is C₂₋₈alkenylene-R¹, -Alk-O-R¹, -Alk-S-R¹ or -Alk-NR⁴-R¹ if X is Z;
or a pharmaceutically acceptable salt thereof.

8. A renin inhibitor according to Claim 1 or 2 being a compound of the general formula 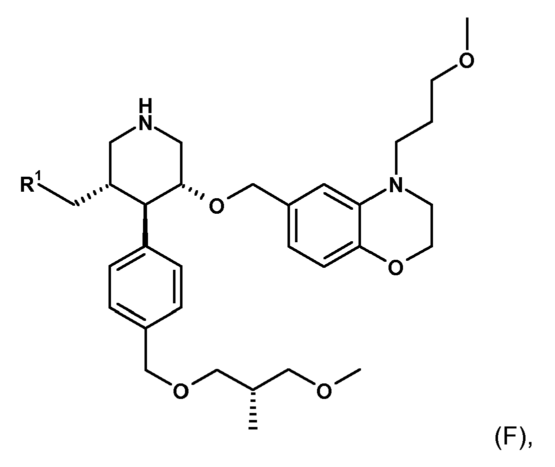 in which
R¹ is straight-chain C₁₋₈-alkanoyloxy, straight-chain C₁₋₈-alkoxy, straight-chain C₁₋₈-alkoxy-straight-chain-C₁₋₈-alkoxy, straight-chain C₁₋₈-alkoxycarbonylamino, straight-chain C₀₋₈-alkylcarbonylamino, optionally N-mono- or N,N-di-C₁₋₈-alkylated amino or hydroxy or straight-chain omega-hydroxy-C₁₋₈-alkyl;
or a pharmaceutically acceptable salt thereof.

9. A renin inhibitor according to Claim 1 or 2 being a compound of the general formula 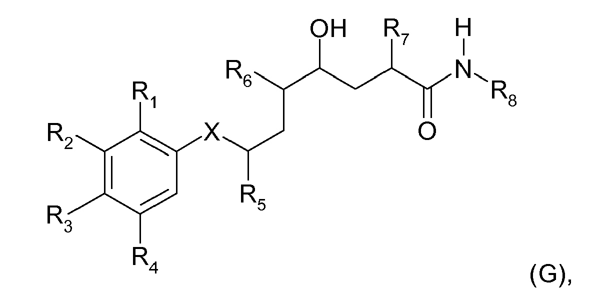 wherein
R₁ is hydrogen, hydroxy, lower alkoxy, cycloalkoxy, lower alkoxy-lower alkoxy or free or esterified or amidareal carboxy-lower alkoxy,
R₂ is hydrogen, lower alkyl, cycloalkyl, lower alkoxy-lower alkyl, lower alkoxy-lower alkoxy-lower alkyl, cycloalkoxy-lower alkyl, hydroxy, optionally lower alkanoylated, halogenated or sulfonylated hydroxy-lower alkoxy; amino-lower alkyl that is unsubstituted or substituted by lower alkyl, by lower alkanoyl and/or by lower alkoxycarbonyl; optionally hydrogenated heteroaryl-lower alkyl; amino-lower alkoxy that is substituted by lower alkyl, by lower alkanoyl and/or by lower alkoxycarbonyl; oxo-lower alkoxy, lower alkoxy, cycloalkoxy, lower alkenyloxy, cycloalkoxy-lower alkoxy, lower alkoxy-lower alkoxy, lower alkoxy-lower alkenyl, lower alkenyloxy-lower alkoxy, lower alkoxy-lower alkenyloxy, lower alkenyloxy-lower alkyl, lower alkanoyl-lower alkoxy, optionally S-oxidised lower alkylthio-lower alkoxy, lower alkylthio-(hydroxy)-lower alkoxy, aryl-lower alkoxy, optionally hydrogenated heteroaryl-lower alkoxy, cyano-lower alkoxy, free or esterified or amidated carboxy-lower alkoxy or free or esterified or amidated carboxy-lower alkyl,
R₃ is optionally halogenated lower alkyl, lower alkoxy-lower alkyl, cycloalkoxy-lower alkyl, hydroxy-lower alkyl, optionally S-oxidised lower alkylthio-lower alkyl, optionally hydrogenareal heteroarylthio-lower alkyl, optionally hydrogenated heteroaryl-lower alkyl; amino-lower alkyl that is unsubstituted or N-mono- or N,N-di-lower alkylated, N-lower alkanoylated or N-lower alkanesulfonylated or N,N-disubstituted by lower alkylene, by unsubstituted or N'-lower alkylated or N'-lower alkanoylated aza-lower alkylene, by oxa-lower alkylene or by optionally S-oxidised thia-lower alkylene; cyano-lower alkyl, free or esterified or amidated carboxy-lower alkyl, cycloalkyl, aryl, hydroxy, lower alkoxy, cycloalkoxy, lower alkoxy-lower alkoxy, cycloalkoxy-lower alkoxy, hydroxy-lower alkoxy, aryl-lower alkoxy, optionally halogenated lower alkoxy, optionally S-oxidised lower alkylthio-lower alkoxy, optionally hydrogenated heteroaryl-lower alkoxy, optionally hydrogenated heteroarylthio-lower alkoxy; amino-lower alkoxy that is unsubstituted or N-mono- or N,N-di-lower alkylated, N-lower alkanoylated or N-lower alkanesulfonylated or substituted by lower alkylene, by unsubstituted or N'-lower alkylated or N'-lower alkanoylated aza-lower alkylene, by oxa-lower alkylene or by optionally S-oxidised thia-lower alkylene; cyano-lower alkoxy or free or esterified or amidated carboxy lower alkoxy, or together with R4 is lower alkylenedioxy or a fused-on benzo or cyclohexeno ring,
R₄ together with R₃ is lower alkylenedioxy or a fused-on benzo or cyclohexeno ring, or is hydrogen, lower alkyl, hydroxy, lower alkoxy or cycloalkoxy,
X is methylene or hydroxymethylene,
R₅ is lower alkyl or cycloalkyl,
R₆ is unsubstituted or N-mono- or N,N-di-lower alkylated or N-lower alkanoylated amino,
R₇ is lower alkyl, lower alkenyl, cycloalkyl or aryl-lower alkyl, and
R₈ is lower alkyl, cycloalkyl, free or aliphatically esterideal or etherideal hydroxy-lower alkyl; amino-lower alkyl that is unsubstituted or N-lower alkanoylated or N-mono- or N,N-di-lower alkylated or N,N-disubstituted by lower alkylene, by hydroxy-, lower alkoxy- or lower alkanoyloxy-lower alkylene, by unsubstituted or N'-lower alkanoylated or N'-lower alkylated aza-lower alkylene, by oxa-lower alkylene or by optionally S-oxidised thia-lower alkylene; free or esterified or amidated carboxy-lower alkyl, free or esterified or amidated dicarboxy-lower alkyl, free or esterideal or amidated carboxy-(hydroxy)-lower alkyl, free or esterified or amidated carboxycycloalkyl-lower alkyl, cyano-lower alkyl, lower alkanesulfonyl-lower alkyl, unsubstituted or N-mono- or N,N-di-lower alkylated thiocarbamoyl-lower alkyl, unsubstituted or N-mono- or N,N-di-lower alkylated sulfamoyl-lower alkyl, or a heteroaryl radical bonded via a carbon atom and optionally hydrogenated and/or oxo-substituted, or lower alkyl substituted by a heteroaryl radical bonded via a carbon atom and optionally hydrogenated and/or oxo-substituted,
or a pharmaceutically acceptable salt thereof,

10. A renin inhibitor according to Claim 1 or 2 being a compound of the general formula 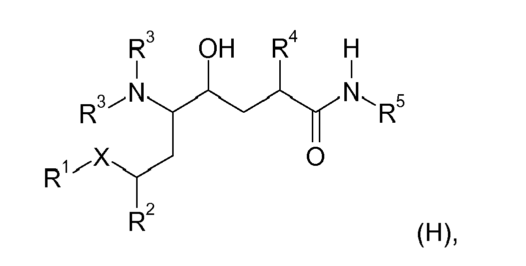 where
X is -CH₂- or >CH-OH;
(A) R¹ is an optionally substituted heterocyclyl radical or an optionally substituted polycyclic, unsaturated hydrocarbon radical where X is hydroxymethylene; or
(B) R¹ is a heterocyclyl radical or a polycyclic, unsaturated hydrocarbon radical which is substituted by one to four radicals selected from C₁-C₆-alkyl, C₃₋₈-cycloalkyl, C₃₋₈-cycloalkoxy, C₃₋₈-cycloalkoxy-C₁₋₆-alkyl, C₃₋₈-cycloalkoxy-C₁₋₆-alkoxy, C₁-C₆-alkylamino, di-C₁-C₆-alkylamino, amino-C₁₋₆-alkyl, amino-C₂₋₇-alkoxy, polyhalo-C₁₋₆-alkyl, polyhalo-C₂₋₇-alkoxy, nitro, amino, oxo, oxide, C₂-C₆-alkenyl, C₁-C₆-alkoxy, C₁-C₆-alkanoyloxy, hydroxy, halogen, cyano, carbamoyl, carboxyl, C₁-C₆-alkylenedioxy, phenyl, phenoxy, phenylthio, phenyl-C₁-C₆-alkyl or phenyl-C₁-C₆-alkoxy, pyridylcarbonylamino-C₁₋₆-alkyl, C₂₋₇-alkenyloxy, C₁₋₆-alkoxy-C₁₋₆-alkoxy, C₁₋₆-alkoxy-C₁₋₆-alkoxy-C₁₋₆-alkyl, methoxybenzyloxy, hydroxybenzyloxy, methylenedioxybenzyloxy, dioxolanyl-C₁₋₆-alkoxy, C₃₋₈-cycloalkyl-C₁₋₆-alkyl, C₃₋₈-cycloalkyl-C₁₋₆-alkoxy, hydroxy-C₂₋₇-alkoxy, carbamoyloxy-C₂₋₇-alkoxy, pyridylcarbamoyloxy-C₂₋₇-alkoxy, benzoyloxy-C₂₋₇-alkoxy, C₁₋₆-alkoxycarbonyl, C₁₋₆-alkylcarbonylamino, C₁₋₆-alkylcarbonylamino-C₁₋₆-alkyl, C₁₋₆-alkylcarbonylamino-C₂₋₇-alkoxy, (N-C₁₋₆-alkyl)-C₁₋₆-alkylcarbonylamino-C₁₋₆-alkyl, (N-C₁₋₆-alkyl)-C₁₋₆-alkylcarbonylamino-C₂₋₇-alkoxy, C₃₋₈-cycloalkylcarbonylamino-C₁₋₆-alkyl, C₃₋₈-cycloalkylcarbonylamino-C₂₋₇-alkoxy, C₁₋₆-alkoxy-C₁₋₆-alkyl, hydroxy-C₁₋₆-alkyl, hydroxy-C₂₋₇-alkoxy-C₁₋₆-alkyl, hydroxy-C₂₋₇-alkoxy-C₁₋₆-alkoxy, C₁₋₆-alkoxycarbonylamino-C₁₋₆-alkyl, C₁₋₆-alkoxycarbonylamino-C₂₋₇-alkoxy, C₁₋₆-alkylaminocarbonylamino-C₁₋₆-alkyl, C₁₋₆-alkylaminocarbonyl-amino-C₂₋₇-alkoxy, C₁₋₆-alkylaminocarbonyl-C₁₋₆-alkyl, C₁₋₆-alkylaminocarbonyl-C₁₋₆-alkoxy, C₁₋₆-alkylaminocarbonyl-C₁₋₆-alkoxy-C₁₋₆-alkyl, di-C₁₋₆-alkylaminocarbonyl-C₁₋₆-alkyl, di-C₁₋₆-alkylaminocarbonyl-C₁₋₆-alkoxy, C₁₋₆-alkylcarbonyloxy-C₁₋₆-alkyl, C₁₋₆-alkylcarbonyloxy-C₂₋₆-alkoxy, cyano-C₁₋₆-alkyl, cyano-C₁₋₆-alkoxy, 2-oxooxazolidinyl-C₁₋₆-alkyl, 2-oxooxazolidinyl-C₁₋₆-alkoxy, C₁₋₆-alkoxycarbonyl-C₁₋₆-alkyl, C₁₋₆-alkoxycarbonyl-C₁₋₆-alkoxy, C₁₋₆-alkylsulphonylamino-C₁₋₆-alkyl, C₁₋₆-alkylsulphonylamino-C₁₋₆-alkoxy, (N-C₁₋₆-alkyl)-C₁₋₆-alkylsulphonylamino-C₁₋₆-alkyl, (N-C₁₋₆-alkyl)-C₁₋₆-alkylsulphonylamino-C₂₋₇-alkoxy, C₁₋₆-alkylamino-C₁₋₆-alkyl, C₁₋₆-alkylamino-C₂₋₇-alkoxy, di-C₁₋₆-alkylamino-C₁₋₆-alkyl, di-C₁₋₆-alkylamino-C₂₋₇-alkoxy, C₁₋₆-alkylsulphonyl-C₁₋₆-alkyl, C₁₋₆-alkylsulphonyl-C₁₋₆-alkoxy, carboxy-C₁₋₆-alkyl, carboxy-C₁₋₆-alkoxy, carboxy-C₁₋₆-alkoxy-C₁₋₆-alkyl, C₁₋₆-alkoxy-C₁₋₆-alkylcarbonyl, acyl-C₁₋₆-alkoxy-C₁₋₆-alkyl, (N-C₁₋₆-alkyl)-C₁₋₆-alkoxy-carbonylamino, (N-hydroxy)-C₁₋₆-alkylaminocarbonyl-C₁₋₆-alkyl, (N-hydroxy)-C₁₋₆-alkylaminocarbonyl-C₁₋₆-alkoxy, (N-hydroxy)aminocarbonyl-C₁₋₆-alkyl, (N-hydroxy)aminocarbonyl-C₁₋₆-alkoxy, C₁₋₆-alkoxyaminocarbonyl-C₁₋₆-alkyl, 6-alkoxyaminocarbonyl-C₁₋₆-alkoxy, (N-C₁₋₆-alkoxy)-C₁₋₆-alkylaminocarbonyl-C₁₋₆-alkyl, (N-C₁₋₆-alkoxy)-C₁₋₆-alkylaminocarbonyl-C₁₋₆-alkoxy, (N-acyl)-C₁₋₆-alkoxy-C₁₋₆-alkylamino, C₁₋₆-alkoxy-C₁₋₆-alkylcarbamoyl, (N-C₁₋₆-alkyl)-C₁₋₆-alkoxy-C₁₋₆-alkylcarbamoyl, C₁₋₆-alkoxy-C₁₋₆-alkylcarbonyl, C₁₋₆-alkoxy-C₁₋₆-alkylcarbonylamino, (N-C₁₋₆-alkyl)-C₁₋₆-alkoxy-C₁₋₆-alkylcarbonylamino, 1-C₁₋₆-alkoxy-C₁₋₆-alkylimidazol-2-yl, 1-C₁₋₆-alkoxy-C₁₋₆-alkyltetrazol-5-yl, 5-C₁₋₆-alkoxy-C₁₋₆-alkyltetrazol-1-yl, 2-C₁₋₆-alkoxy-C₁₋₆-alkyl-4-oxoimidazol-1-yl, carbamoyl-C₁₋₆-alkyl, carbamoyl-C₁₋₆-alkoxy, C₁₋₆-alkylcarbamoyl, di-C₁₋₆-alkylcarbamoyl, C₁₋₆-alkylsulphonyl, C₁₋₆-alkylamidinyl, acetamidinyl-C₁₋₆-alkyl, O-methyl-oximyl-C₁₋₆-alkyl, O,N-dimethylhydroxylamino-C₁₋₆-alkyl, C₃₋₆-cycloalkyl-C₁₋₆-alkanoyl, aryl-C₁₋₆-alkanoyl or heterocyclyl-C₁₋₆-alkanoyl, each of which is optionally substituted by halogen, C₁-C₆-alkyl, C₁₋₆-alkoxy, hydroxy, C₁-C₆-alkylamino, di-C₁-C₆-alkylamino, C₁₋₆-alkoxycarbonyl, hydroxy-C₁₋₆-alkyl or trifluoromethyl, and also pyridyl, pyridyloxy, pyridylthio, pyridylamino, pyridyl-C₁₋₆-alkyl, pyridyl-C₁₋₆-alkoxy, pyrimidinyl, pyrimidinyloxy, pyrimidinylthio, pyrimidinylamino, pyrimidinyl-C₁₋₆-alkyl, pyrimidinyl-C₁₋₆-alkoxy, thienyl, thienyl-C₁₋₆-alkyl, thienyl-C₁₋₆-alkoxy, furyl, furyl-C₁₋₆-alkyl or furyl-C₁₋₆-alkoxy, piperidinoalkyl, piperidinoalkoxy, piperidinoalkoxyalkyl, morpholinoalkyl, morpholinoalkoxy, morpholinoalkoxyalkyl, piperazinoalkyl, piperazinoalkoxy, piperazinoalkoxyalkyl, [1,2,4]triazol-1-ylalkyl, [1,2,4]triazol-1-ylalkoxy, [1,2,4]triazol-4-ylalkyl, [1,2,4]triazol-4-ylalkoxy, [1,2,4]oxadiazol-5-ylalkyl, [1,2,4]oxadiazol-5-yl-alkoxy, 3-methyl[1,2,4]oxadiazol-5-ylalkyl, 3-methyl[1,2,4]oxadiazol-5-ylalkoxy, 5-methyl[1,2,4]oxadiazol-3-ylalkyl, 5-methyl[1,2,4]oxadiazol-3-ylalkoxy, tetrazol-1-ylalkyl, tetrazol-1-ylalkoxy, tetrazol-2-ylalkyl, tetrazol-2-ylalkoxy, tetrazol-5-ylalkyl, tetrazol-5-ylalkoxy, 5-methyltetrazol-1-ylalkyl, 5-methyltetrazol-1-ylalkoxy, thiazol-4-ylalkyl, thiazol-4-ylalkoxy, oxazol-4-ylalkyl, oxazol-4-ylalkoxy, 2-oxopyrrolidinylalkyl, 2-oxopyrrolidinylalkoxy, imidazolylalkyl, imidazolylalkoxy, 2-methylimidazolylalkyl, 2-methylimidazolylalkoxy or N-methylpiperazinoalkyl, N-methylpiperazinoalkoxy, N-methylpiperazinoalkoxyalkyl, dioxolanyl, dioxanyl, dithiolanyl, dithianyl, pyrrolidinyl, piperidinyl, piperazinyl, pyrrolyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 3-acetamidomethylpyrrolidinyl, 3-C₁₋₆-alkoxy-C₁₋₆-alkylpyrrolidinyl, 4-hydroxypiperidinyl, 4-oxopiperidinyl, 3,5-dimethylmorpholinyl, 4,4-dioxothiomorpholinyl, 4-oxo-thiomorpholinyl, 2,6-dimethylmorpholinyl, 2-oxoimidazolidinyl, 2-oxooxazolidinyl, 2-oxopyrrolidinyl, 2-oxo[1,3]oxazinyl, 2-oxotetrahydropyrimidinyl, each of which is optionally substituted by halogen, C₁₋₆-alkyl, C₁₋₆-alkoxy or dihydroxy-C₁₋₆-alkylaminocarbonyl, and the -O-CH₂CH(OH)CH₂NRₓ radical where NRₓ is a mono- or di-C₁₋₆-alkylamino, piperidino, morpholino, piperazino or N-methylpiperazino radical,
where, in the case that R¹ is naphthyl or cyclohexenophenyl, at least the ring system not bonded to X is substituted as specified; or
(C) R¹ is pyrazinyl, triazolyl, imidazolyl, benzthiazolyl, pyranyl, tetrahydropyranyl, azetidinyl, morpholinyl, quinazolinyl, quinoxalinyl, isoquinolyl, benzo[b]thienyl, isobenzofuranyl, benzimidazolyl, 2-oxobenzimidazolyl, oxazolyl, thiazolyl, pyrrolyl, pyrazolyl, triazinyl, dihydrobenzofuranyl, 2-oxodihydrobenzo[d][1,3]oxazinyl, 4-oxodihydroimidazolyl, 5-oxo-4H-[1,2,4]triazinyl, 3-oxo-4H-benzo[1,4]thiazinyl, tetrahydroquinoxalinyl, 1,1,3-trioxodihydro-2H-1λ⁶-benzo[1,4]thiazinyl, 1-oxopyridyl, dihydro-3H-benzo[1,4]oxazinyl, 2-oxotetrahydrobenzo[e][1,4]diazepinyl, 2-oxo-dihydrobenzo[e][1,4]diazepinyl, 1H-pyrrolizinyl, phthalazinyl, 1-oxo-3H-isobenzofuranyl, 4-oxo-3H-thieno[2,3-d]pyrimidinyl, 3-oxo-4H-benzo[1,4]oxazinyl, [1,5]naphthyridyl, dihydro-2H-benzo[1,4]thiazinyl, 1,1-dioxodihydro-2H-benzo[1,4]thiazinyl, 2-oxo-1H-pyrido[2,3-b][1,4]oxazinyl, dihydro-1H-pyrido[2,3-b][1,4]oxazinyl, 1 H-pyrrolo[2,3-b]pyridyl, benzo[1,3]dioxolyl, benzooxazolyl, 2-oxobenzooxazolyl, 2-oxo-1,3-dihydroindolyl, 2,3-dihydroindolyl, indazolyl, benzofuranyl, dioxolanyl, dioxanyl, dithiolanyl, dithianyl, pyrrolidinyl, piperidinyl, piperazinyl, 4-methylpiperazinyl, morpholinyl, thiomorpholinyl, 2-hydroxymethylpyrrolidinyl, 3-hydroxypyrrolidinyl, 3,4-dihydroxypyrrolidinyl, 4-hydroxypiperidinyl, 4-oxopiperidinyl, 3,5-dimethylmorpholinyl, 4,4-dioxothiomorpholinyl, 4-oxothiomorpholinyl, 2,6-dimethylmorpholinyl, tetrahydropyranyl, 2-oxoimidazolidinyl, 2-oxooxazolidinyl, 2-oxopiperidinyl, 2-oxopyrrolidinyl, 2-oxo[1,3]oxazinyl, 2-oxoazepanyl, or 2-oxotetrahydropyrimidinyl;
R² is C₁-C₆-alkyl or C₃-C₆-cycloalkyl;
R³ are each independently H, C₁-C₆-alkyl, C₁₋₆-alkoxycarbonyl or C₁-C₆-alkanoyl;
R⁴ is C₁-C₆-alkyl, C₃-C₆-cycloalkyl, C₂-C₆-alkenyl or unsubstituted or substituted aryl-C₁-C₆-alkyl;
R⁵ is C₁-C₆-alkyl, C₁-C₆-hydroxyalkyl, C₁-C₆-alkoxy-C₁-C₆-alkyl, C₁-C₆-alkanoyloxy-C₁-C₆-alkyl, C₁-C₆-aminoalkyl, C₁-C₆-alkylamino-C₁-C₆-alkyl, C₁-C₆-dialkylamino-C₁-C₆-alkyl, C₁-C₆-alkanoylamido-C₁-C₆-alkyl, HO(O)C-C₁-C₆-alkyl, C₁-C₆-alkyl-O-(O)C-C₁-C₆-alkyl, H₂N-C(O)-C₁-C₆-alkyl, C₁-C₆-alkyl-HN-C(O)-C₁-C₆-alkyl, (C₁-C₆-alkyl)₂N-C(O)-C₁-C₆-alkyl, C₂-C₈-alkenyl, C₂-C₈-alkynyl, cyano-C₁-C₆-alkyl, halo-C₁-C₆-alkyl, optionally substituted aryl-C₀-C₆-alkyl, optionally substituted C₃-C₈-cycloalkyl-C₀₋C₆-alkyl or optionally substituted heterocyclyl-C₀₋C₆-alkyl, ,
or a pharmaceutically acceptable salt thereof.

11. A renin inhibitor according to Claim 1 or 2 being a compound of the general formula 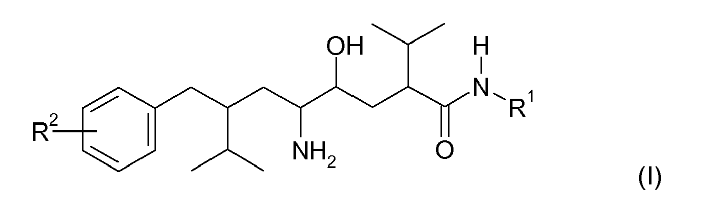 in which
R¹ is a) saturated heterocyclyl which is optionally substituted one or more times by C₁₋₈- alkanoyl, C₂₋₆alkenyl, C₂₋₆alkynyl, C₁₋₆alkoxy, C₁₋₆alkoxy-C₁₋₆alkoxy, C₁₋₆alkoxy-C₁₋₆alkyl, C₁₋₆alkoxycarbonylamino, C₁₋₆alkyl, C₀₋₆alkylcarbonylamino, C₁₋₆alkyl-carbonyloxy, C₁₋₆alkylenedioxy, optionally N-mono or N,N-di-C₁₋₆-alkylated amino, aryl, optionally N-mono or N,N-di-C₁₋₆-alkylated carbamoyl, optionally esterified carboxy, cyano, C₃₋₈cydoalkoxy, C₃₋₈cycloalkyl-C₀₋₆alkyl, halogen, halo-C₁₋₆alkoxy, halo-C₁₋₆alkyl, heteroaryl, unsaturated, partially saturated or saturated heterocyclyl, hydroxy, nitro, oxide or oxo which is bonded via a C atom; or
b) azepanyl-, azetidinyl-, aziridinyl-, dioxanyl-, dioxepanyl-, dioxolanyl-, dithianyl-, dithiolanyl-, furanyl-, oxathianyl-, oxepanyl-, tetrahydropyranyl-, tetrahydrofuranyl-, tetrahydrothiophenyl-, tetrahydrothiopyranyl-, thiepanyl- or bicyclic saturated heterocyclyl-C₁₋₄alkyl each of which is optionally substituted one or more times by C₁₋₈alkanoyl, C₂₋₆alkenyl, C₂₋₆alkynyl, C₁₋₆alkoxy, C₁₋₆alkoxy-C₁₋₆alkoxy, C₁₋₆alkoxy-C₁₋₆alkyl, C₁₋₆alkoxycarbonylamino, C₁₋₆alkyl, C₀₋₆alkylcarbonylamino, C₁₋₆alkyl-carbonyloxy, C₁₋₆alkylenedioxy, optionally N-mono or N,N-di-C₁₋₆-alkylated amino, aryl, optionally N-mono or N,N-di-C₁₋₆-alkylated carbamoyl, optionally esterified carboxy, cyano, C₃₋₈cycloalkoxy, C₃₋₈cycloalkyl-C₀₋₆alkyl, halogen, halo-C₁₋₆alkoxy, halo-C₁₋₆alkyl, heteroaryl, unsaturated, partially saturated or saturated heterocyclyl, hydroxy, nitro, oxide or oxo; or
c) C₂₋₈alkynyl which is optionally substituted one or more times by C₁₋₈alkanoyl, C₂₋₆alkenyl, C₂₋₆alkynyl, C₁₋₆alkoxy, C₁₋₆alkoxy-C₁₋₆alkoxy, C₁₋₆alkoxy-C₁₋₆alkyl, C₁₋₆alkoxycarbonylamino, C₁₋₆alkyl, C₀₋₆alkylcarbonylamino, C₁₋₆alkylcarbonyloxy, C₁₋₆alkylenedioxy, optionally N-mono or N,N-di-C₁₋₆-alkylated amino, aryl, optionally N-mono or N,N-di-C₁₋₆-alkylated carbamoyl, optionally esterified carboxy, cyano, C₃₋₈cycloalkoxy, C₃₋₈cycloalkyl-C₀₋₆alkyl, halogen, halo-C₁₋₆-alkoxy, halo-C₁₋₆alkyl, heteroaryl, unsaturated, partially saturated or saturated heterocyclyl, hydroxy, nitro or oxo; or
R² is, independently of one another, 1-4 radicals selected from: C₁₋₈alkanoyl, C₁₋₈alkanoyl-C₂₋₄alkoxy bearing the alkanoyl group in a position higher than .C₁₋₈alkanoylamino-C₁₋₄alkoxy, N-C₁₋₄alkanoylamino-C₁₋₄alkyl, C₁₋₈alkanoyloxy-C₁₋₄alkyl, N'-C₂₋₈alkanoylpiperazino-C₁₋₄alkoxy, N'-C₂₋₈alkanoylpiperazino-C₁₋₄alkyl, C₁₋₈alkanesulphonyl-C₁₋₄alkoxy, C₁₋₈alkanesulphonyl-C₁₋₄alkyl, C₁₋₈alkanesulphonyl-amino-C₁₋₄alkoxy, C₁₋₄alkanesulphonylamino-C₁₋₄alkyl, C₁₋₈alkanesulphonyl-C₁₋₄(hydroxy)alkoxy, C₂₋₈alkenyloxy, C₂₋₈alkenyloxy-C₁₋₄alkoxy, C₂₋₈alkenyloxy-C₁₋₄alkyl, C₁₋₈alkoxy, C₁₋₆alkoxy-C₁₋₆alkanoyl, C₁₋₄alkoxy-C₂₋₄alkenyl, C₁₋₄alkoxy-C₂₋₄alkenyloxy, C₁₋₄alkoxy-C₁₋₄alkoxy, C₁₋₄alkoxy-C₁₋₄alkoxy-C₁₋₄alkyl, C₁₋₄alkoxy-C₁₋₄alkyl, C₁₋₄alkoxycarbonyl-C₁₋₄alkoxy, C₁₋₄alkoxycarbonyl-C₁₋₄alkyl, C₁₋₈alkoxycarbonylamino-C₁₋₄alkoxy, C₁₋₈alkoxycarbonylamino-C₁₋₄alkyl, C₁₋₈alkyl, C₁₋₄alkylamino, N,N-di-C₁₋₄alkylamino, C₁₋₄alkylamino-C₁₋₄alkoxy, N,N-di-C₁₋₄alkylamino-C₁₋₄alkoxy, C₁₋₄alkylamino-C₁₋₄alkyl, N,N-di-C₁₋₄alkylamino-C₁₋₄alkyl, N-mono- or N,N-di-C₁₋₄alkylcarbamoyl-C₁₋₄alkoxy, N-mono- or N,N-di-C₁₋₄alkylcarbamoyl-C₁₋₄alkyl, N'-C₁₋₄alkylpiperazino-C₁₋₄alkoxy, N'-C₁₋₄alkylpiperazino-C₁₋₄alkyl, C₁₋₄alkylthio-C₁₋₄alkoxy, C₁₋₄alkylthio-C₁₋₄(hydroxy)alkoxy, C₁₋₄alkylthio-C₁₋₄alkyl, amino-C₂₋₄alkoxy, amino-C₁₋₄alkyl, carbamoyl-C₁₋₄alkoxy, carbamoyl-C₁₋₈alkyl, carboxy-C₁₋₄alkoxy, carboxy-C₁₋₄alkyl, cyano-C₁₋₄alkoxy, cyano-C₁₋₄alkyl, C₃₋₈-cycloalkoxy, C₃₋₈cycloalkoxy-C₁₋₄alkoxy, C₃₋₈cycloalkoxy-C₁₋₄alkyl, C₃₋₈cycloalkyl, S,S-dioxothiomorpholino-C₁₋₄alkoxy, S.S-dioxothiomorpholino-C₁₋₄alkyl, halogen, halo-C₁₋₄alkoxy, halo-C₁₋₄alkyl, halo-C₂₋₈(hydroxy)alkoxy, hydroxy, hydroxy-C₂₋₈alkoxy, hydroxy-C₂₋₈alkyl, imidazolylthio-C₁₋₄alkoxy, imidazolylthio-C₁₋₄alkyl, optionally N-oxidized morpholino-C₁₋₄alkoxy, morpholino-C₁₋₄alkyl, S-oxothio-morpholino-C₁₋₄alkoxy, S-oxothiomorpholino-C₁₋₄alkyl, piperazino-C₁₋₄alkoxy, piperazino-C₁₋₄alkyl, piperidino-C₁₋₄alkoxy, piperidino-C₁₋₄alkyl, optionally partially hydrogenated pyridyl- or N-oxidopyridyl-C₁₋₄alkoxy, optionally partially hydrogenated pyridyl- or N-oxidopyridyl-C₁₋₄alkyl, optionally N-oxidized pyridylthio-C₁₋₄alkoxy, optionally N-oxidized pyridylthio-C₁₋₄alkyl, pyrimidinylthio-C₁₋₄alkoxy, pyrimidinylthio-C₁₋₄alkyl, pyrrolidino-C₁₋₄alkoxy, pyrrolidino-C₁₋₄alkyl, thiazolinylthio-C₁₋₄alkoxy, thiazolinylthio-C₁₋₄alkyl, thiazolyl-C₁₋₄alkoxy, thiazolylthio-C₁₋₄alkoxy, thiazolylthio-C₁₋₄alkyl, thiomorpholino-C₁₋₄alkoxy, thiomorpholino-C₁₋₄alkyl, trifluoro-C₁₋₈alkanesulphonyl-C₁₋₄alkoxy, trifluoro-C₁₋₈alkanesulphonylamino-C₁₋₄alkyl, phenyl or naphthyl which is unsubstituted or mono-, di- or trisubstituted by C₁₋₄alkoxy, C₁₋₄alkyl, C₁₋₄alkylamino, di-C₁₋₄alkylamino, halogen, hydroxy and/or trifluoromethyl, and phenyl- or naphthyl-C₁₋₄alkoxy which is unsubstituted or mono-, di- or trisubstituted by C₁₋₄alkoxy, C₁₋₄alkyl, C₁₋₄alkylamino, di-C₁₋₄alkylamino, halogen, hydroxy and/or trifluoromethyl,
or a pharmaceutically acceptable salt thereof.

12. A renin inhibitor according to Claim 1 or 2 being a compound of the general formula 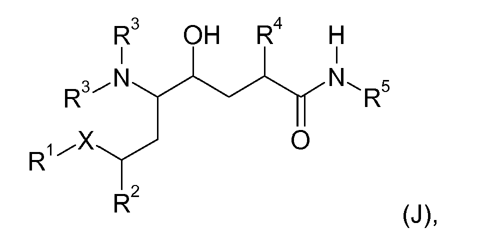 in which
X is -CH₂- ;
R¹ is a mono- to tetrasubstituted, mono- or bicyclic, unsaturated heterocyclic radical having 1 to 4 nitrogen atoms, where the substituents of the said radicals are selected independently of one another from the group consisting of acetamidinyl-C₁₋₆alkyl, 3-acetamidomethylpyrrolidinyl, acyl-C₁₋₆alkoxy-C₁₋₆alkyl, (N-acyl)-C₁₋₆alkoxy-C₁₋₆alkylamino, C₁₋₆alkanoyl, C₁₋₆alkanoyloxy, C₂₋₆alkenyl, C₂₋₆alkenyloxy, C₂₋₆alkenyloxy-C₁₋₆alkyl, C₁₋₆alkoxy, C₁₋₆alkoxy-C₁₋₆alkoxy, C₁₋₆alkoxy-C₁₋₆alkoxy-C₁₋₆alkyl, C₁₋₆alkoxy-C₁₋₆alkyl, (N-C₁₋₆alkoxy)-C₁₋₆alkylaminocarbonyl-C₁₋₆alkoxy, (N-C₁₋₆alkoxy)-C₁₋₆alkylaminocarbonyl-C₁₋₆alkyl, C₁₋₆alkoxy-C₁₋₆alkylcarbamoyl, C₁₋₆alkoxy-C₁₋₆alkylcarbonyl, C₁₋₆alkoxy-C₁₋₆alkylcarbonylamino, 1-C₁₋₆alkoxy-C₁₋₆alkylimidazol-2-yl, 2-C₁₋₆alkoxy-C₁₋₆alkyl-4-oxoimidazol-1-yl, 3-C₁₋₆alkoxy-C₁₋₆alkylpyrrolidinyl, 1-C₁₋₆alkoxy-C₁₋₆alkyltetrazol-5-yl, 5-C₁₋₆alkoxy-C₁₋₆alkyltetrazol-1-yl, C₁₋₆alkoxyaminocarbonyl-C₁₋₆alkoxy, C₁₋₆alkoxyaminocarbonyl-C₁₋₆alkyl, C₁₋₆alkoxycarbonyl, C₁₋₆alkoxycarbonyl-C₁₋₆alkoxy, C₁₋₆alkoxycarbonyl-C₁₋₆alkyl, C₁₋₆alkoxycarbonylamino, C₁₋₆alkoxycarbonylamino-C₁₋₆alkoxy, C₁₋₆alkoxycarbonylamino-C₁₋₆alkyl, C₁₋₆alkyl, (N-C₁₋₆alkyl)-C₁₋₆alkoxy-C₁₋₆alkylcarbamoyl, (N-C₁₋₆alkyl)-C₁₋₆alkoxy-C₁₋₆alkylcarbonylamino, (N-C₁₋₆alkyl)-C₁₋₆alkoxycarbonylamino, (N-C₁₋₆alkyl)-C₀₋₆alkylcarbonylamino-C₁₋₆alkoxy, (N-C₁₋₆alkyl)-C₀₋₆alkylcarbonylamino-C₁₋₆alkyl, (N-C₁₋₆alkyl)-C₁₋₆alkylsulphonylamino-C₁₋₆alkoxy, (N-C₁₋₆alkyl)-C₁₋₆alkylsulphonylamino-C₁₋₆alkyl, C₁₋₆alkylamidinyl, C₁₋₆alkylamino, di-C₁₋₆alkylamino, C₁₋₆alkylamino-C₂₋₆alkoxy, di-C₁₋₆alkylamino-C₂₋₆alkoxy, C₁₋₆alkylamino-C₁₋₆alkyl, C₁₋₆alkylaminocarbonyl, C₁₋₆alkylaminocarbonyl-C₁₋₆alkoxy, di-C₁₋₆alkylaminocarbonyl-C₁₋₆alkoxy, C₁₋₆alkylaminocarbonyl-C₁₋₆alkoxy-C₁₋₆alkyl, C₁₋₆alkylaminocarbonyl-C₁₋₆alkyl, C₁₋₆alkylaminocarbonylamino-C₁₋₆alkoxy, C₁₋₆alkylamino-carbonylamino-C₁₋₆alkyl, di-C₁₋₆alkylaminocarbonyl-C₁₋₆alkyl, di-C₁₋₆alkylamino-C₁₋₆alkyl, C₁₋₆alkylcarbamoyl, di-C₁₋₆alkylcarbamoyl, C₀₋₆alkylcarbonylamino, C₀₋₆alkylcarbonylamino-C₁₋₆alkoxy, C₀₋₆alkylcarbonylamino-C₁₋₆alkyl, C₁₋₆alkyl-carbonyloxy-C₁₋₆alkoxy, C₁₋₆alkylcarbonyloxy-C₁₋₆alkyl, C₁₋₆alkylenedioxy, C₁₋₆alkylsulphonyl, C₁₋₆alkylsulphonyl-C₁₋₆alkoxy, C₁₋₆alkylsulphonyl-C₁₋₆alkyl, C₁₋₆alkylsulphonylamino-C₁₋₆alkoxy, C₁₋₆alkylsulphonylamino-C₁₋₆alkyl, amino, amino-C₂₋₇alkoxy, amino-C₁₋₆alkyl, aryl-C₁₋₆alkanoyl, benzoyloxy-C₂₋₆alkoxy, carbamoyl, carbamoyl-C₁₋₆alkoxy, carbamoyl-C₁₋₆alkyl, carboxy, carboxy-C₁₋₆alkoxy, carboxy-C₁₋₆alkoxy-C₁₋₆alkyl, carboxy-C₁₋₆alkyl, cyano, cyano-C₁₋₆alkoxy, cyano-C₁₋₆alkyl, C₃₋₈cycloalkyl-C₁₋₆alkanoyl, C₃₋₈cycloalkyl-C₀₋₆alkoxy, C₃₋₈cycloalkyl-C₀₋₆alkyl, C₃₋₈cycloalkylcarbonylamino, C₃₋₈cycloalkylcarbonylamino-C₁₋₆alkoxy, C₃₋₈cyclo-alkylcarbonylamino-C₁₋₆alkyl, 3,4-dihydroxypyrrolidinyl, O,N-dimethylhydroxylamino-C₁₋₆alkyl, 2,6-dimethylmorpholinyl, 3,5-dimethylmorpholinyl, dioxanyl, dioxolanyl, dioxolanyl-C₁₋₆alkoxy, 4,4-dioxothiomorpholinyl, dithianyl, dithiolanyl, optionally C₁₋₆alkoxy-, C₁₋₆alkyl-, dihydroxy-C₁₋₆alkylaminocarbonyl- or halogen-substituted furyl, furyl-C₁₋₆alkoxy, furyl-C₁₋₆alkyl, pyridyl, pyridyl-C₁₋₆alkoxy, pyridyl-C₁₋₆alkyl, pyridylamino, pyridyloxy, pyridylthio, pyrimidinyl, pyrimidinyl-C₁₋₆alkoxy, pyrimidinyl-C₁₋₆alkyl, pyrimidinylamino, pyrimidinyloxy, pyrimidinylthio, thienyl, thienyl-C₁₋₆alkoxy or thienyl-C₁₋₆alkyl, halogen, heterocyclyl-C₁₋₆alkanoyl, hydroxy, hydroxy-C₂₋₆alkoxy, hydroxy-C₂₋₆alkoxy-C₁₋₆alkoxy, hydroxy-C₂₋₆alkoxy-C₁₋₆alkyl, hydroxy-C₁₋₆alkyl, (N-hydroxy)-C₁₋₆alkylaminocarbonyl-C₁₋₆alkoxy, (N-hydroxy)-C₁₋₆alkylaminocarbonyl-C₁₋₆alkyl, (N-hydroxy)aminocarbonyl-C₁₋₆alkoxy, (N-hydroxy)aminocarbonyl-C₁₋₆alkyl, hydroxybenzyloxy, 2-hydroxymethylpyrrolidinyl, 4-hydroxypiperidinyl, 3-hydroxypyrrolidinyl, imidazolyl-C₁₋₆alkoxy, imidazolyl-C₁₋₆alkyl, methoxybenzyloxy, methylenedioxybenzyloxy, 2-methylimidazolyl-C₁₋₆alkoxy, 2-methylimidazolyl-C₁₋₆alkyl, 3-methyl-[1,2,4]-oxadiazol-5-yl-C₁₋₆alkoxy, 5-methyl-[1,2,4]-oxadiazol-3-yl-C₁₋₆alkoxy, 3-methyl-[1,2,4]-oxadiazol-5-yl-C₁₋₆alkyl, 5-methyl-[1,2,4]-oxadiazol-3-yl-C₁₋₆alkyl, O-methyl-oximyl-C₁₋₆alkyl, 4-methylpiperazinyl, N-methylpiperazino-C₁₋₆alkoxy, N-methylpiperazino-C₁₋₆alkoxy-C₁₋₆alkyl, N-methylpiperazino-C₁₋₆alkyl, 5-methyltetrazol-1-yl-C₁₋₆alkoxy, 5-methyltetrazol-1-yl-C₁₋₆alkyl, morpholinyl, morpholino-C₁₋₆alkoxy, morpholino-C₁₋₆alkoxy-C₁₋₆alkyl, morpholino-C₁₋₆alkyl, nitro, [1,2,4]-oxadiazol-5-yl-C₁₋₆alkoxy, [1,2,4]-oxadiazol-5-yl-C₁₋₆alkyl, oxazol-4-yl-C₁₋₆alkoxy, oxazol-4-yl-C₁₋₆alkyl, oxide, oxo, 2-oxoimidazolidinyl, 2-oxo-[1,3]oxazinyl, 2-oxooxazolidinyl, 2-oxooxazolidinyl-C₁₋₆alkoxy, 2-oxooxazolidinyl-C₁₋₆alkyl, 4-oxopiperidinyl, 2-oxopyrrolidinyl, 2-oxopyrrolidinyl-C₁₋₆alkoxy, 2-oxopyrrolidinyl-C₁₋₆alkyl, 2-oxotetrahydropyrimidinyl, 4-oxothiomorpholinyl, optionally C₁₋₆alkoxy-, C₁₋₆alkoxycarbonyl-, C₁₋₆alkyl-, C₁₋₆alkylamino-, di-C₁₋₆alkylamino-, halogen-, hydroxy-, hydroxy-C₁₋₆alkyl-, or trifluoromethyl-substituted phenoxy, phenyl, phenyl-C₁₋₆alkoxy, phenyl-C₁₋₆alkyl or phenylthio, piperazinyl, piperazino-C₁₋₆alkoxy, piperazino-C₁₋₆alkoxy-C₁₋₆alkyl, piperazino-C₁₋₆alkyl, piperidinyl, piperidino-C₁₋₆alkoxy, piperidino-C₁₋₆alkoxy-C₁₋₆alkyl, polyhalogen-C₁₋₆alkoxy, polyhalogen-C₁₋₆alkyl, pyridylcarbamoyloxy-C₁₋₆alkoxy, pyridylcarbonylamino-C₁₋₆alkyl, pyrrolidinyl, pyrrolyl, tetrazol-1-yl-C₁₋₆alkoxy, tetrazol-2-yl-C₁₋₆alkoxy, tetrazol-5-yl-C₁₋₆alkoxy, tetrazol-1-yl-C₁₋₆alkyl, tetrazol-2-yl-C₁₋₆alkyl, tetrazol-5-yl-C₁₋₆alkyl, thiazol-4-yl-C₁₋₆alkoxy, thiazol-4-yl-C₁₋₆alkyl, thiomorpholinyl, [1,2,4]-thazol-1-yl-C₁₋₆alkoxy, [1,2,4]-triazol-4-yl-C₁₋₆alkoxy, [1,2,4]-triazol-1-yl-C₁₋₆alkyl, [1,2,4]-triazol-4-yl-C₁₋₆alkyl and the radical -O-CH₂CH(OH)CH₂NRx, where NRx is a mono- or di-C₁₋₆alkylamino, N-methylpiperazino, morpholino, piperazino or piperidino radical, and where in the case where R¹ is a bicyclic heterocyclic ring system, at least the ring not directly bonded to X is substituted as indicated;
R² is C₁₋₆alkyl or C₃₋₆cycloalkyl;
R³ is independently of one another H, C₁₋₆alkyl, C₁₋₆alkoxycarbonyl or C₁₋₆alkanoyl;
R⁴ is C₂₋₆alkenyl, C₁₋₆alkyl, unsubstituted or substituted aryl-C₁₋₆alkyl or C₃₋₈cycloalkyl;
R⁵ is -Lₘ-R⁶;
L is C₁₋₈alkylene which is optionally substituted by 1-4 halogen, or a linker: 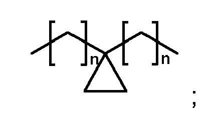
n = 0, 1 or 2;
m = 0 or 1;
R⁶ is a radical composed of 2 cyclic systems selected from bicyclo[x.y.z]alkyl, spiro[o.p]alkyl, mono- or bioxabicyclo[x.y.z]alkyl or mono- or bioxaspiro[o.p]alkyl, all of which may be substituted by 1-3 substituents selected from C₁₋₆alkyl, C₁₋₆alkoxy, cyano, halogen, C₁₋₆alkoxy-C₁₋₆alkyl, hydroxy-C₁₋₆alkyl or dialkylamino, or if m = 0: is also saturated C₃₋₈heterocyclyl which comprises 1-2 oxygen atoms, substituted by 1-3 substituents selected from C₁₋₆alkyl, C₁₋₆alkoxy, cyano, halogen, C₁₋₆alkoxy-C₁₋₆alkyl, hydroxy-C₁₋₆alkyl or dialkylamino, or
if m = 1: is also saturated C₃₋₈heterocyclyl which comprises 1-2 oxygen atoms, optionally substituted by 1-3 substituents selected from C₁₋₆alkyl, C₁₋₆alkoxy, cyano, halogen, C₁₋₆-alkoxy-C₁₋₆alkyl, hydroxy-C₁₋₆alkyl or dialkylamino;
o = 2, 3, 4, 5 or 6
p = 2, 3, 4, 5 or 6
x = 1, 2, 3, 4 or 5;
y = 1, 2, 3, 4 or 5;
z = 0, 1, 2, 3, 4 or 5; where x ≥ y ≥ z;
or a pharmaceutically acceptable salt thereof.

13. Use of a renin inhibitor for producing a human medicine for the prevention, for the retardation of progression or for the treatment of psoriasis.

14. Method for the prevention, for the retardation of progression or for the treatment of psoriasis, in which a therapeutically effective amount of a renin inhibitor is used.
